(19) Europäisches Patentamt
European Patent Office
Office européen des brevets

(11) EP 2 522 666 A1

(12) EUROPEAN PATENT APPLICATION
published in accordance with Art. 153(4) EPC

(43) Date of publication:
**14.11.2012 Bulletin 2012/46**

(21) Application number: **11731808.9**

(22) Date of filing: **06.01.2011**

(51) Int Cl.:
***C07D 417/06*** *(2006.01)* ***A01N 43/78*** *(2006.01)*
***A61P 7/02*** *(2006.01)* ***A61P 7/04*** *(2006.01)*
***C07D 417/14*** *(2006.01)*

(86) International application number:
**PCT/JP2011/050074**

(87) International publication number:
**WO 2011/083810 (14.07.2011 Gazette 2011/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2010 JP 2010001486**
**06.01.2010 JP 2010001485**
**06.01.2010 JP 2010001484**

(71) Applicants:
- **Gifu University**
**Gifu-shi**
**Gifu 501-1193 (JP)**
- **Meiji Seika Pharma Co., Ltd.**
**Tokyo 104-8002 (JP)**

(72) Inventors:
- **KAGABU, Shinzo**
**Gifu-shi**
**Gifu 501-1193 (JP)**
- **KUMAZAWA, Satoru**
**Tokyo 103-8552 (JP)**
- **WATANABE, Tsumoru**
**Tokyo 103-8552 (JP)**
- **MIYAKE, Taiji**
**Tokyo 103-8552 (JP)**
- **NOMURA, Masahiro**
**Yokohama-shi**
**Kanagawa 222-8569 (JP)**
- **NAKAMURA, Satoshi**
**Yokohama-shi**
**Kanagawa 222-8569 (JP)**
- **MITOMI, Masaaki**
**Yokohama-shi**
**Kanagawa 222-8569 (JP)**

(74) Representative: **Polypatent**
**An den Gärten 7**
**51491 Overath (DE)**

# (54) IMINO DERIVATIVES, PROCESS FOR PREPARATION THEREOF, AND INSECTICIDES CONTAINING SAME

(57) To provide a novel imino derivative capable of being an insecticide compound which is excellent in characteristics such as sustained effects and a broad spectrum.

An imino derivative represented by Formula (A) shown below:

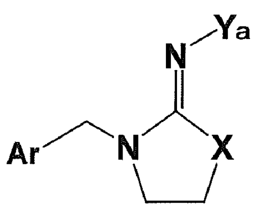

····· Formula (A)

wherein "Ar" represents a heterocyclic group which may have a substituent on the ring, "X" represents a sulfur atom or $CH_2$, NR; "R" represents a hydrogen atom or an alkyl group;
"Ya" is selected from "$C(=S)NR_1R_2$", "$C(=S)SR_3$", "$C(=O)SR_4$", "$C(=S)OR_4$", "$SO_2Z_\alpha$", and "OZp"; and, each of "$R_1$" to "$R_4$", "$Z_\alpha$", and "Zp" represents a hydrogen atom or a certain substituent;

is provided.



Printed by Jouve, 75001 PARIS (FR)

## Description

[Technical Field]

The present invention relates to a novel imino derivative, a method for producing the same and a use thereof. More specifically, the invention relates to a novel imino derivative, a method for producing the derivative, and an insecticide containing the derivative as an active ingredient.

[Background Art]

An insecticide employed in the field of agriculture has conventionally been required to have a diversity of the characteristics. The characteristics required for an insecticide may for example be sustained effects and broad spectrum, safety when handling, easiness in using in combination with other drugs or formulation auxiliaries. Being less expensive is also required as a matter of course.
In conjunction with the invention, Patent Literature 1 and 2 describe insecticide compounds having imino structures.
In addition, insecticide compounds having sulfonylimino structures which have already been reported include the compounds described in Patent Literature 1 and Patent Literature 3.
On the other hand, there is no report of the synthesis of an insecticide compound having an oxime ether structure, and this compound belongs to an insecticide compound group having an entirely novel backbone. A reported insecticide compound having a structure analogous, at least, to that of this compound is a compound having an imino structure (see Patent Literature 1).

[Citation List]

[Patent Literature]

[PTL 1] Japanese Unexamined Patent Application Publication No. 5-78323
[PTL 2] Japanese Translation of PCT No. 11-509530
[PTL 3] Japanese Unexamined Patent Application Publication No. 2002-322175

[Summary of Invention]

[Technical Problem]

An insecticide employed in the field of the agriculture suffers from a problem which is the appearance of a pest which has acquired a resistance against a particular drug when such a drug has been employed for a prolonged period.
In order to prevent the appearance of such a drug-resistance pest, and also to combat against such a drug-resistance pest which has already appeared, a novel insecticide having various characteristics described above are still needed to be developed in these days.
While synthesis of insecticide compounds having the imino structures such as 2-pyrazylcarbonyliminodihydrothiazole disclosed in the above-indicated Patent Literature 1 and a substituted N-methylenethiourea disclosed in Patent Literature 2 are known, no hopeful insecticide compounds relating to the inventive compounds have been identified.
On the other hand, while heterocyclic compounds disclosed in the above-indicated Patent Literature 1 and Patent Literature 3 are known, a compound having a sulfonylimino structure which is an insecticide compound having further excellent characteristics is demanded.
Accordingly, a major objective of the invention is to provide a novel imino derivative which can be an insecticide compound having excellent characteristics, such as sustained effects and a broad spectrum.

[Solution to Problem]

(The reference of the application involved)

The present application contains a priority claim based on JP2010-001484, JP2010-001485, and JP2010-001486 filed in January 6, 2010, the all disclosures of these Japanese applications refered is the part of the disclosure of the present application.

To solve the above-indicated problems, the invention provides an imino derivative represented by Formula (A) shown below:

[C. 1]

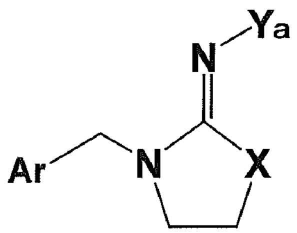

..... Formula (A)

wherein "Ar" represents a heterocyclic group which may have a substituent on the ring; "X" represents a sulfur atom or $CH_2$, NR; "R" represents a hydrogen atom or an alkyl group;

"Ya" represents "C(=S)$NR_1R_2$", "C(=S)$SR_3$", "C(=O)$SR_4$", "C(=S)$OR_4$","$SO_2Z_\alpha$,", or "$OZ_\beta$";

when "Ya" is "C(=S)$NR_1R_2$", each of "$R_1$" and "$R_2$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a substituted or unsubstituted C6 to C10 arylcarbonyl group, a (CH to C3)alkylsulfonyl (C1 to C3)alkyl group, a (CH to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridylmethyl group, a substituted or unsubstituted pyridyl group, a substituted or unsubstituted imidazolylmethyl group, or a substituted or unsubstituted heterocyclic ring or aromatic ring; provided that "$R_1$" and "$R_2$" do not represent hydrogen atoms simultaneously; "$NR_1R_2$" may form a ring;

when "Ya" is "C(=S)$SR_3$", "$R_3$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a (CH to C3)alkoxycarbonyl(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring;

when "Ya" is "C(=O)$SR_4$" or "C(=S)$OR_4$", "$R_4$" represents a C1 to C5 alkyl group, or a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3) alkyl group, a (C1 to C3)alkoxy(C1 to C3)alkyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted imidazolylmethyl group, a substituted or unsubstituted pyridyl group, a substituted or unsubstituted heterocyclic ring, aromatic ring;

when "Ya" is "$SO_2Z_\alpha$", "$Z_\alpha$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C6 to C10)aryl(C2 to C5)alkenyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted thienyl group, or a substituted or unsubstituted heterocyclic ring or aromatic ring; or it represents "$NR_5R_6$"; in "$NR_5R_6$", each of "$R_5$" and "$R_6$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C4 alkoxy group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C1 to C4 alkoxycarbonyl group, a C3 to C7 substituted or unsubstituted cycloalkyl group, a 3-membered ring to 7-membered ring substituted or unsubstituted heterocycloalkyl group, a substituted or unsubstituted pyridyl group, a substituted or unsubstituted heterocyclic ring or aromatic ring group; "$NR_5R_6$" may form a ring;

when "Ya" is "$OZ_\beta$", "$Z_\beta$" represents "$R_7$", "$COR_8$" "$CONR_9R_{10}$" or "$SO_2R_1$" when "$Z_\beta$" represents $R_7$, "$R_7$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring;

when "$Z_\beta$" represents $COR_8$, "$R_8$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a (C1 to C3)alkylsulfonyl(C1 to C3)alkyl group, a (C1 to C3) alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, a substituted or unsubstituted heterocyclic ring or aromatic ring;

when "$Z_\beta$" represents $CONR_9R_{10}$, each of "$R_9$" and "$R_{10}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (CH to C3)alkoxy(C1 to C3)alkyl group, a C1 to C3 alkoxycarbonyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted pyridyl group, a substituted or unsubstituted imidazolidylmethyl group, a substituted or unsubstituted heterocyclic ring or aromatic ring; "$NR_9R_{10}$" may form a ring;

when "$Z_\beta$" represents $SO_2R_{11}$, "$R_{11}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted imidazolidylmethyl group, a substituted or unsubstituted thienyl group, or a substituted or unsubstituted heterocyclic ring or aromatic ring; and, provided that compounds wherein "Ar" is 2-chloro-5-pyridyl, "X" is a sulfur atom or an oxygen atom, "Ya" is $SO_2$-methyl, or wherein "Ar" is 2-chloro-5-pyridyl, "Ya" is $SO_2$-substituted phenyl are excluded.

The invention also provides an imino derivative represented by Formula (1) shown below:

[C. 2]

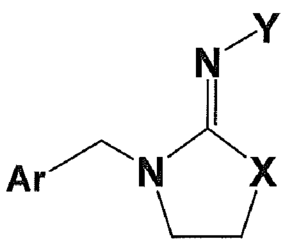

..... Formula (1)

wherien "Ar" represents the same meanings as "Ar" in Formula (A) shown above; "X" represents a sulfur atom or $CH_2$, NR; "R" represents a hydrogen atom or an alkyl group; "Y" represents "$C(=S)NR_1R_2$", "$C(=S)SR_3$", "$C(=O)SR_4$", or "C

(=S)$OR_4$" (wherein R, $R_1$, $R_2$, $R_3$, and $R_4$ represents the same meanings as R, $R_1$, $R_2$, $R_3$, and $R_4$ in Formula (A) shown above.

The invention also provides a sulfonylimino derivative represented by Formula (2) shown below:

[C. 3]

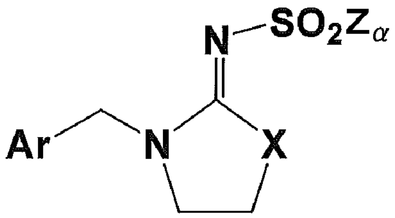

Formula (2)

wherien "Ar" represents the same meanings as "Ar" in Formula (A) shown above; "X" represents a sulfur atom or $CH_2$, NR; "R" represents a hydrogen atom or an alkyl group; "$Z_\alpha$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C6 to C10)aryl(C2 to C5)alkenyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted thienyl group, or a substituted or unsubstituted hetero ring or aromatic ring; or it represents "$NR_5R_6$" (wherein, $R_5$, $R_6$ have the same meanings as $R_5$, $R_6$ in Formula (A) shown above); and, "$NR_5R_6$" may form a ring.

The invention also provides an oxime ether derivative represented by Formula (3) shown below:

[C. 4]

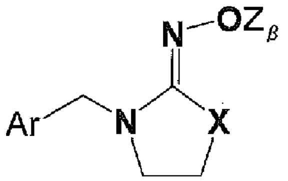

Formula (3)

wherien "Ar" represents the same meanings as "Ar" in Formula (A) shown above; "X" represents a sulfur atom or $CH_2$, NR; "R" represents a hydrogen atom or an alkyl group; "$Z_\beta$" represents "$R_7$", "$COR_8$", "$CONR_9R_{10}$", or "$SO_2R_{11}$" (wherein $R_7$, $R_8$, $R_9$, $R_{10}$, and $R_{11}$ represents the same meanings as $R_7$, $R_8$, $R_9$, and $R_{10}$, and $R_{11}$ in Formula (A) shown above.)

The invention also provides a method for producing the imino derivative described above.

A compound represented by Formula (7) shown below which is Formula (1) wherein "Y" is "C(=S)$NR_1R_2$" can be produced by reacting a compound represented by Formula (4) shown below with a compound represented by Formula (5) shown below or a compound represented by Formula (6) shown below:

[C. 5]

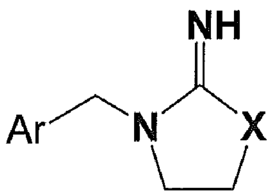

Formula (4)

[C. 6] **$R_1NCS$** Formula (5)

[C. 7] $R_1R_2NCSA$ Formula (6)

[C. 8]

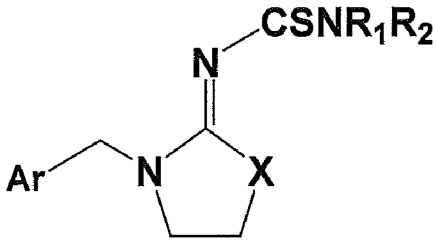

····· Formula (7)

wherein "Ar" represents a heterocyclic group which may have a substituent on the ring; "X" represents a sulfur atom or $CH_2$, NR; "R" represents a hydrogen atom or an alkyl group;
each of "$R_1$" and "$R_2$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a substituted or unsubstituted C6 to C10 arylcarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridylmethyl group, a substituted or unsubstituted pyridyl group, a substituted or unsubstituted imidazolylmethyl group, or a substituted or unsubstituted heterocyclic ring or aromatic ring; "$NR_1R_2$" may form a ring; and, "A" represents a halogen atom such as chlorine, bromine, or iodine.

A compound represented by Formula (7) shown below which is Formula (A) wherein "Ya" is "C(=S)$NR_1R_2$" and "$R_2$" is a hydrogen atom can be produced by reacting a compound represented by Formula (9) shown below, which is produced by reacting a compound represented by Formula (4) shown below with a compound represented by Formula (8) shown below, with a compound represented by Formula (10) shown below:

[C. 9]

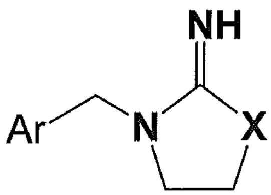

····· Formula (4)

[C. 10]

Formula (8)

**BtCSBt** ·····式(7)

[C. 11]

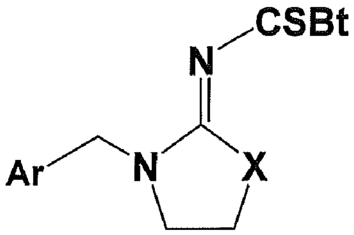

..... Formula (9)

[C. 121 $R_1NH_2$ Formula (10)

[C. 13]

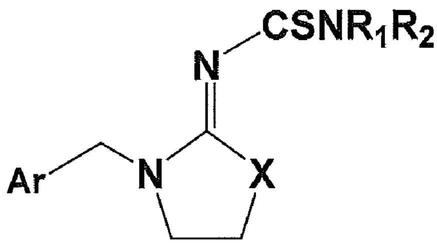

..... Formula (7)

wherein "Ar" represents a heterocyclic group which may have a substituent on the ring; "X" represents a sulfur atom or $CH_2$, NR; "R" represents a hydrogen atom or an alkyl group;
"Bt" represents 1-(1,2,3-benzotriazol);
"$R_1$" represents a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a substituted or unsubstituted C6 to C10 arylcarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridylmethyl group, a substituted or unsubstituted pyridyl group, a substituted or unsubstituted imidazolylmethyl group, or a substituted or unsubstituted heterocyclic ring or aromatic ring; and, "$R_2$" represents a hydrogen atom.

A compound represented by Formula (14) shown below which is Formula (A) wherein "Ya" is "C(=S)$SR_3$" can be produced by reacting a compound represented by Formula (4) shown below with a compound represented by Formula (11) shown below, a compound represented by Formula (12) shown below, or a compound represented by Formula (13) shown below:

[C. 14]

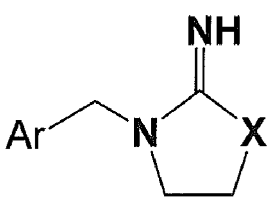

..... Formula (4)

[C. 15] $(R_3S)_2CS$ Formula (11)

[C. 16] $ClCS_2R_3$ Formula (12)

[C. 17]

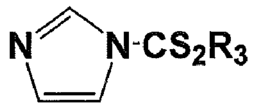

..... Formula (13)

[C. 18]

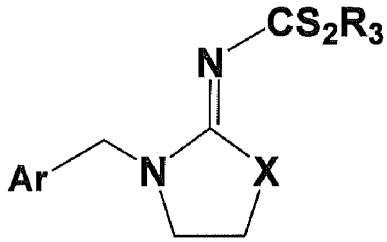

..... Formula (14)

wherein "Ar" represents a heterocyclic group which may have a substituent on the ring; "X" represents a sulfur atom or $CH_2$, NR; "R" represents a hydrogen atom or an alkyl group; and,

"$R_3$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a (C1 to C3) alkoxycarbonyl(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

Furthermore, a compound represented by Formula (16) shown below which is Formula (A) wherein "Ya" is "$C(=O)SR_4$" or "$C(=S)OR_4$" can be produced by reacting a compound represented by Formula (4) shown below with a compound represented by Formula (15) shown below:

[C. 19]

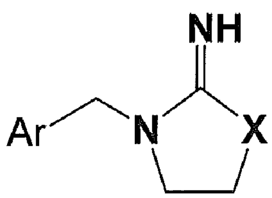

..... Formula (4)

[C. 201 **$BC(=P)QR_4$** Formula (15)

[C. 21]

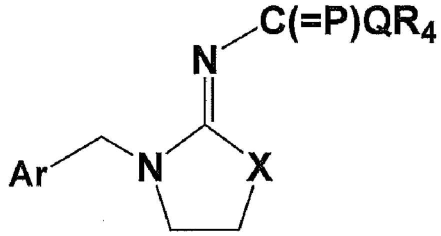

..... Formula (16)

wherein "Ar" represents a heterocyclic group which may have a substituent on the ring; "X" represents a sulfur atom or $CH_2$, NR; "R" represents a hydrogen atom or an alkyl group;
"B" represents a halogen atom such as chlorine, bromine, or iodine; each of "P", "Q" represents a sulfur atom or an oxygen atom; provided that "P" and "Q" are different from each other; and,
"$R_4$" represents a C1 to C5 alkyl group, or a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted imidazolylmethyl group, a substituted or unsubstituted pyridyl group, a substituted or unsubstituted heterocyclic ring, aromatic ring.

The invention also provides a method for producing the sulfonylimino derivative represented by Formula (2) shown above.
A compound represented by Formula (2) shown below according to the invention can be produced by reacting a compound represented by Formula (17) shown below with a compound represented by Formula (4) shown below:

[C. 22]

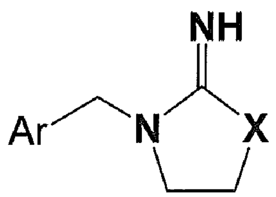

..... Formula (4)

[C. 23] $Z_\alpha SO_2A$ Formula (17)

[C. 24]

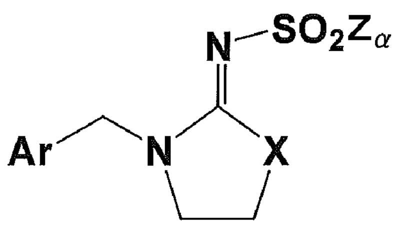

..... Formula (2)

wherein "Ar" represents a heterocyclic group which may have a substituent on the ring; "X" represents a sulfur atom or $CH_2$, NR; "R" represents a hydrogen atom or an alkyl group;
"$Z_\alpha$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10

aryl group, a substituted or unsubstituted (C6 to C10)aryl(C1 to C3)alkyl group, a (C6 to C10)aryl(C2 to C5)alkenyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy (C2 to C5)alkyl group, a substituted or unsubstituted pyridylmethyl group, a substituted or unsubstituted pyridyl group, a substituted or unsubstituted thienyl group, or a substituted or unsubstituted heterocyclic ring or aromatic ring; or it represents "$NR_5R_6$"; in "$NR_5R_6$", each of "$R_5$" and "$R_6$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C4 alkoxy group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C1 to C4 alkoxycarbonyl group, a C3 to C7 substituted or unsubstituted cycloalkyl group, a 3-membered ring to 7-membered ring substituted or unsubstituted heterocycloalkyl group, a substituted or unsubstituted pyridyl group, a substituted or unsubstituted heterocyclic ring or aromatic ring group; "$NR_5R_6$" may form a ring; and,
"A" represents a halogen atom such as chlorine, bromine, or iodine or "$OSO_2Z_\alpha$".

The invention also provides a method for producing the oxime ether derivative represented by Formula (3) shown above.
A compound represented by Formula (19) shown below which is Formula (3) wherein "$Z_\beta$" represents "$R_7$" can be produced by reacting a compound represented by Formula (4) shown below with a compound represented by Formula (18) shown below:

[C. 25]

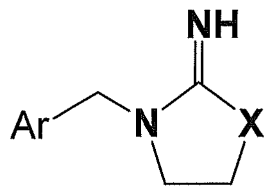

····· Formula (4)

[C. 26] **$NH_2OR_7$** Formula (18)

[C. 27]

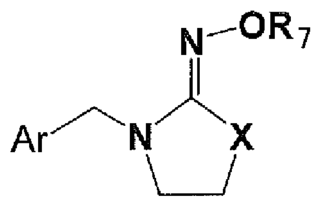

····· Formula (19)

wherein "Ar" represents a heterocyclic group which may have a substituent on the ring; "X" represents a sulfur atom or $CH_2$, NR; "R" represents a hydrogen atom or an alkyl group; and,
"$R_7$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

A compound represented by Formula (23) shown below which is Formula (3) wherein "$Z_\beta$" represents "$COR_8$" can be produced by reacting a compound represented by Formula (20) shown below with a compound represented by Formula (21) shown below or a compound represented by Formula (22) shown below.

[C. 28]

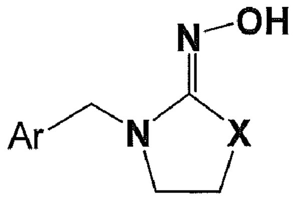

..... Formula (20)

[C. 29] **$R_8COA$** Formula (21)

[C. 30] **$R_8CO_2H$** Formula (22)

[C. 31]

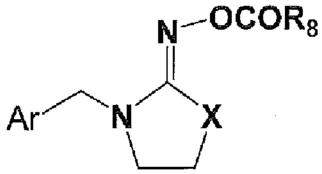

..... Formula (23)

wherein "Ar" represents a heterocyclic group which may have a substituent on the ring; "X" represents a sulfur atom or $CH_2$, NR; "R" represents a hydrogen atom or an alkyl group;
"A" represents a halogen atom such as chlorine, bromine, and iodine; and, "$R_8$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl (C1 to C3)alkyl group, a (C1 to C4)alkoxy(C1 to C5)alkyl group, a C1 to C3 alkoxycarbonyl group, a (C1 to C3) alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3) alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, a substituted or unsubstituted heterocyclic ring or aromatic ring.

Furthermore, a compound represented by Formula (26) shown below which is Formula (3) wherein "$Z_\beta$" represents "$CONR_9R_{10}$" can be produced by reacting a compound represented by Formula (20) shown below with a compound represented by Formula (24) shown below or a compound represented by Formula (25) shown below:

[C. 32]

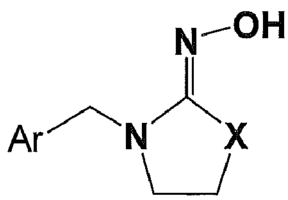

..... Formula (20)

[C. 33] **$R_9NCO$** Formula (24)

[C. 34] **$R_9R_{10}NCOB$** Formula (25)

[C. 35]

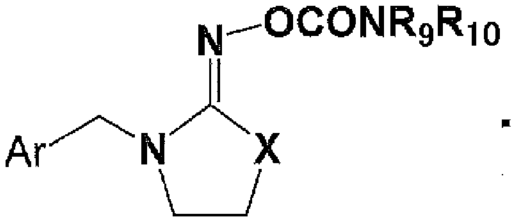

..... Formula (26)

wherein "Ar" represents a heterocyclic group which may have a substituent on the ring; "X" represents a sulfur atom or $CH_2$, NR; "R" represents a hydrogen atom or an alkyl group;

"B" represents a halogen atom such as chlorine, bromine, and iodine; each of "$R_9$" and "$R_{10}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a C1 to C3 alkoxycarbonyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted pyridyl group, a substituted or unsubstituted imidazolidylmethyl group, a substituted or unsubstituted heterocyclic ring or aromatic ring; provided that in Formula (24) "$R_9$" is other than a hydrogen atom; and, "$NR_9R_{10}$" may form a ring.

Finally, a compound represented by Formula (28) shown below which is Formula (3) wherein "$Z_\beta$" represents "$SO_2R_{11}$" can be produced by reacting a compound represented by Formula (20) shown below with a compound represented by Formula (27) shown below:

[C. 36]

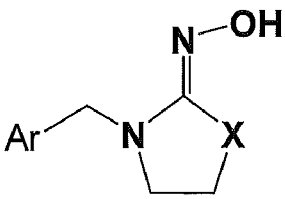

..... Formula (20)

[C. 37] **$R_{11}SO_2D$** Formula (27)

[C. 38]

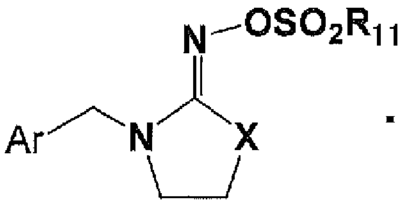

..... Formula (28)

wherein "Ar" represents a heterocyclic group which may have a substituent on the ring;
"X" represents a sulfur atom or $CH_2$, NR; "R" represents a hydrogen atom or an alkyl group;
"D" represents a halogen atom such as chlorine, bromine, and iodine; and, "$R_{11}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted imidazolidylmethyl group, a substituted or unsubstituted thienyl group, or a substituted or unsubstituted heterocyclic ring or aromatic ring.

The invention also provides an insecticide containing as an active ingredient an imino derivative represented by Formula (A) shown above.
The invention also provides an insecticide containing as an active ingredient the imino derivative represented by Formula (1) shown above.
The invention also provides an insecticide containing as an active ingredient the sulfonylimino derivative represented by Formula (2) shown above.
The invention also provides an insecticide containing as an active ingredient the oxime ether derivative represented by Formula (3) shown above.

[Advantageous Effects of Invention]

According to the invention, a novel imino derivative capable of being an insecticide compound having excellent characteristics such as sustained effects and broad spectrum is provided.

[Description of Embodiments]

Preferred embodiments for carrying out the invention are described below. The embodiments described below are only examples of representative embodiments of the invention and do not serve to allow the scope of the invention to be interpreted narrowly.
"An aryl group" in the present specification represents an aromatic ringed hydrocarbon gruop and specifically represents a phenyl group or a naphthyl group.

1. Imino derivatives

A novel derivative according to the invention is represented by Formula (A) shown below. This imino derivative is further described below.
[C. 39]

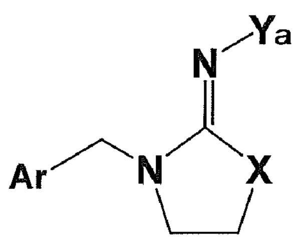

Formula (A)

In Formula (A), "Ar" represents a heterocyclic ring which may have a substituent on the ring. Specifically, the 5-membered or 6-membered heterocyclic ring may for example be pyridine, thiazole, tetrahydrofuran, furan, and thiophene. Those especially preferred are 3-pyridyl group, 5-thiazolyl group, and 3-tetrahydrofuryl group.
The substituent on the heterocyclic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl)amino group, and a nitro group. Preferably, the chlorine atom is

used as a substituent on the heterocyclic ring.

In Formula (A), "X" represents a sulfur atom or $CH_2$, NR, and "R" represents a hydrogen atom or an alkyl group. The alkyl group may be a C1 to C4 alkyl group, which may be any of normal, secondary, and tertiary groups, and may for example be a methyl group, an ethyl group, an n-propyl group, an isopropyl group, an n-butyl group, an isobutyl group, a sec-butyl group, and a tert-butyl group.

In Formula (A), when "Ya" is "C(=S)$NR_1R_2$", each of "$R_1$" and "$R_2$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a substituted or unsubstituted C6 to C10 arylcarbonyl group, a (C1 to C3) alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridylmethyl group, a substituted or unsubstituted pyridyl group, a substituted or unsubstituted imidazolylmethyl group, or a substituted or unsubstituted heterocyclic ring or aromatic ring. "$R_1$" and "$R_2$" do not represent hydrogen atoms simultaneously. "$NR_1R_2$" may form a ring.

Each of "$R_1$" and "$R_2$" groups preferably represents a hydrogen atom or a C1 to C5 alkyl group, a C1 to C5 halogenated alkyl group, a substituted or unsubstituted (C6 to C10)aryl(C1 to C3)alkyl group, a C1 to C3 alkoxycarbonyl group, a C1 to C3 alkylcarbonyl group, a substituted or unsubstituted C6 to C10 arylcarbonyl group, a (C1 to C3)alkylthio (C1 to C3)alkyl group, a C3 to C7 substituted or unsubstituted cycloalkyl group.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The alkyl group of the C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The substituted or unsubstituted (C6 to C10)aryl(C1 to C3)alkyl group may for example be a benzyl group or a phenyl ethyl group.

The C1 to C3 alkoxycarbonyl group is the carbonyl group having a C1 to C3 alkyloxy group, and may for example be a methoxycarbonyl group an ethoxycarbonyl group, or a propyloxycarbonyl group.

The C1 to C3 alkylcarbonyl group is the carbonyl group having a C1 to C3 alkyl group, and may for example be an acetyl group, a propionyl group, or a butyryl group.

The substituted or unsubstituted C6 to C10 arylcarbonyl group is the carbonyl group having a C6 to C10 aryl group, and may for example be a benzoyl group or a naphthoyl group.

The (C1 to C3)alkylthio(C1 to C3)alkyl group may for example be a methylthioethyl group, a methylthiopropyl group, or ethylthiomethyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the substituted phenoxy(C1 to C3)alkyl-substituted benzene ring is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

When "$NR_1R_2$" forms a ring, it is preferred that "$R_1$" and "$R_2$" are in a C2 to C6 cyclic structure, or a 3-membered ring to 7-membered ring cyclic structure containing 1 to 2 hetero atoms such as oxygen, sulfur, and nitrogen atoms in the ring of the 3-membered ring to 7-membered ring as "$NR_1R_2$" is preferred.

In Formula (A), when "Ya" is "C(=S)$SR_3$", "$R_3$" represents a C1 to C5 alkyl group, a C1 to C5 halogenated alkyl

group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C3) alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

"$R_3$" group is preferably a C1 to C5 alkyl group, a substituted or unsubstituted (C6 to C10)aryl(C1 to C3)alkyl group.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The substituted or unsubstituted C6 to C10 arylcarbonyl group is the carbonyl group having a C6 to C10 aryl group, and may for example be a benzoyl group or a naphthoyl group.

The alkyl group of the C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the substituted phenoxy(C1 to C3)alkyl-substituted phenylbenzene ring is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

In Formula (A), when "Ya" is "$C(=O)SR_4$" or "$C(=S)OR_4$", "$R_4$" represents a C1 to C5 alkyl group, or a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10) aryl(C1 to C3)alkyl group, a (C1 to C3)alkoxy(C1 to C3)alkyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted imidazolylmethyl group, a substituted or unsubstituted pyridyl group, a substituted or unsubstituted heterocyclic ring, aromatic ring.

"$R_4$" group is preferably a C1 to C5 alkyl group or a substituted or unsubstituted C6 to C10 aryl group, a substituted or unsubstituted pyridyl group.

The C1 to C5 alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The substituted or unsubstituted C6 to C10 arylcarbonyl group is the carbonyl group having a C6 to C10 aryl group, and may for example be a benzoyl group or a naphthoyl group.

The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a

C1 to C3 alkyl group. The substituent attached to the pyridine ring in the substituted pyridylmethyl group is not limited particularly, and may for example be a halogen atom (which may be any of chlorine, bromine, fluorine, and iodine) or a C1 to C3 alkyl group. The substituent attached to the benzene ring in the substituted benzenesulfonyl group is not limited particularly, and may for example be a halogen atom (which may be any of chlorine, bromine, fluorine, and iodine) or a nitro group, a di(C1 to C4 alkyl)amino group, an acetylamino group, a C1 to C3 alkyl group.

Specifically, the heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, 3-tetrahydrofuryl group. The aromatic ring may for example be a phenyl group or a naphtyl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl)amino group, a nitro group, and an acetylamino group.

In Formula (A), when "Ya" is "$SO_2Z_\alpha$","$Z_\alpha$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C6 to C10)aryl(C2 to C5)alkenyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted thienyl group, or a substituted or unsubstituted heterocyclic ring or aromatic ring.

"$Z_\alpha$" group is preferably a hydrogen atom or a C1 to C5 alkyl group, a C1 to C5 halogenated alkyl group, a C2 to C5 alkenyl group, a substituted or unsubstituted C6 to C10 aryl group, a substituted or unsubstituted (C6 to C10)aryl (C1 to C3)alkyl group, a (C6 to C10)aryl(C2 to C5)alkenyl group, a (C1 to C4)alkoxy(C1 to C3)alkyl group, a C3 to C7 substituted or unsubstituted cycloalkyl group, a substituted or unsubstituted pyridyl group, a substituted or unsubstituted thienyl group.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The alkyl group of a C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the substituted phenoxy(C1 to C3)alkyl-substituted phenylbenzene ring is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

"$Z_\alpha$" also represents "$NR_5R_6$". Each of "$R_5$" and "$R_6$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C4 alkoxy group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C1 to C4 alkoxycarbonyl group, a C3 to C7 substituted or unsubstituted cycloalkyl group, a 3-membered ring to 7-membered ring substituted or unsubstituted heterocycloalkyl group, a substituted or unsubstituted pyridyl group, a substituted or unsubstituted heterocyclic ring or aromatic ring group. "$NR_5R_6$" may form a ring.

Each of "$R_5$" and "$R_6$" groups is preferably a hydrogen atom or a C1 to C5 alkyl group, a C1 to C4 alkoxycarbonyl group.

The C1 to C5 alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C1 to C4 alkoxycarbonyl group is the carbonyl group having a C1 to C4 alkyloxy group, and may for example be a methoxycarbonyl group an ethoxycarbonyl group, a propyloxycarbonyl group, or a butyloxycarbonyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent attached to the pyridine ring in the substituted pyridylmethyl group is not limited particularly, and may for example be a halogen atom (which may be any of chlorine, bromine, fluorine, and iodine) or a C1 to C3 alkyl group.

When "$NR_5R_6$" forms a ring, it is preferred that "$R_5$" and "$R_6$" are in a C2 to C6 cyclic structure. Alternatively, a 3-membered ring to 7-membered ring cyclic structure containing 1 to 2 hetero atoms such as oxygen, sulfur, and nitrogen atoms in the ring of the 3-membered ring to 7-membered ring as "$NR_5R_6$" is preferred.

When "Ya"" is "$OZ_\beta$" in Formula (A), when "$Z_\beta$" represents $R_7$, "$R_7$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl (C1 to C3)alkyl group, a (C1 to C4)alkoxy(C1 to C5)alkyl group, a C1 to C3 alkoxycarbonyl group, a (C1 to C3)alkylsulfonyl (C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

"$R_7$" group is preferably a hydrogen atom or a C1 to C5 alkyl group, a substituted or unsubstituted (C6 to C10) aryl(C1 to C3)alkyl group.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The substituted or unsubstituted C6 to C10 aryl group may for example be a phenyl group or a naphthyl group.

The substituted or unsubstituted (C6 to C10)aryl(C1 to C3)alkyl group may for example be a benzyl group or a phenyl ethyl group.

The alkyl group of a C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly, and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the phenyl group in the substituted phenoxy(C1 to C3)alkyl group is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

When "$Z_\beta$" represents $COR_8$, "$R_8$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C5) alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, a substituted or unsubstituted heterocyclic ring or aromatic ring.

"$R_8$" group is preferably a C1 to C5 alkyl group, a C1 to C5 halogenated alkyl group, a substituted or unsubstituted C6 to C10 aryl group, a (C1 to C4)alkoxy(C1 to C5)alkyl group, a substituted or unsubstituted pyridyl group, a substituted or unsubstituted heterocyclic ring or aromatic ring.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The substituted or unsubstituted C6 to C10 aryl group may for example be a phenyl group or a naphthyl group.

The (C1 to C4)alkoxy(C1 to C5)alkyl group,group may for example be a methoxymethyl group an methoxyethyl group, or an ethoxymethyl group.

The alkyl group of a C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly, and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the phenyl group in the substituted phenoxy(C1 to C3)alkyl group is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

When "$Z_\beta$" represents $CONR_9R_{10}$, each of "$R_9$" and "$R_{10}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a C1 to C3 alkoxycarbonyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted pyridyl group, a substituted or unsubstituted imidazolidylmethyl group, a substituted or unsubstituted heterocyclic ring or aromatic ring. "$NR_9R_{10}$" may form a ring.

Each of "$R_9$" and "$R_{10}$" groups is preferably a hydrogen atom or a C1 to C5 alkyl group, a substituted or unsubstituted C6 to C10 aryl group.

The C1 to C5 alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The substituted or unsubstituted C6 to C10 aryl group may for example be a phenyl group or a naphthyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen

atom (which may be any of chlorine, bromine, fluorine, and iodine) or a C1 to C3 alkyl group.
The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent attached to the pyridine ring in the substituted pyridylmethyl group is not limited particularly, and may for example be a halogen atom (which may be any of chlorine, bromine, fluorine, and iodine) or a C1 to C3 alkyl group. The substituent attached to the benzene ring in the substituted benzenesulfonyl group is not limited particularly, and may for example be a halogen atom (which may be any of chlorine, bromine, fluorine, and iodine) or a nitro group, a di(C1 to C4 alkyl)amino group, an acetylamino group, a C1 to C3 alkyl group.

When "$NR_9R_{10}$" forms a ring, it is preferred that "$R_9$" and "$R_{10}$" are in a C2 to C6 cyclic structure, or a 3-membered ring to 7-membered ring cyclic structure containing 1 to 2 hetero atoms such as oxygen, sulfur, and nitrogen atoms in the ring of the 3-membered ring to 7-membered ring as "$NR_9R_{10}$" is preferred.

When "$Z_\beta$" represents $SO_2R_{11}$, "$R_{11}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted imidazolidylmethyl group, a substituted or unsubstituted thienyl group, or a substituted or unsubstituted heterocyclic ring or aromatic ring.

"$R_{11}$" group is preferably a hydrogen atom or a C1 to C5 alkyl group, a substituted or unsubstituted C6 to C10 aryl group.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.
The substituted or unsubstituted C6 to C10 aryl group may for example be a phenyl group or a naphthyl group.
The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.
The C3 to C7 substituted or unsubstituted cycloalkyl group may for example be a cyclopropyl group, a cyclobutyl group, a cyclopentyl group, and a cyclohexyl group. The substituent is not limited particularly, and may for example be a halogen atom (which may be any of chlorine, bromine, fluorine, and iodine) or a C1 to C3 alkyl group. The substituent attached to the benzene ring in the substituted phenyl group is not limited particularly, and may for example be a halogen atom (which may be any of chlorine, bromine, fluorine, and iodine) or a nitro group, a di(C1 to C4 alkyl)amino group, an acetylamino group, a C1 to C3 alkyl group.

A preferred embodiment of the invention is a compound wherein "Ar" represents a 3-pyridyl group, 5-thiazolyl group, or 3-tetrahydrofuryl group, "X" represents a nitrogen atom or a sulfur atom, "Ya" represents "$C(=S)NR_1R_2$" (wherein each of "$R_1$" and "$R_2$" represents a hydrogen atom or a C1 to C5 alkyl group, a C1 to C5 halogenated alkyl group, a substituted or unsubstituted (C6 to C10)aryl(C1 to C3)alkyl group, a C1 to C3 alkoxycarbonyl group, a C1 to C3 alkylcarbonyl group, a substituted or unsubstituted C6 to C10 arylcarbonyl group, a (C1 to C3)alkylthio(C1 to C3)alkyl group, a C3 to C7 substituted or unsubstituted cycloalkyl group; "$R_1$" and "$R_2$" do not represent hydrogen atoms simultaneously.), or "$C(=S)SR_3$" (wherein "$R_3$" represents a C1 to C5 alkyl group, a substituted or unsubstituted (C6 to C10)aryl(Cl to C3) alkyl group), or "$C(=O)SR_4$", "$C(=S)OR_4$" (wherein "$R_4$" represents a C1 to C5 alkyl group or a substituted or unsubstituted C6 to C10 aryl group, a substituted or unsubstituted pyridyl group), or $SO_2Z_\alpha$ (wherein "$Z_\alpha$" represents a hydrogen atom or a C1 to C5 alkyl group, a C1 to C5 halogenated alkyl group, a C2 to C5 alkenyl group, a substituted or unsubstituted C6 to C10 aryl group, a substituted or unsubstituted (C6 to C10)aryl(C1 to C3)alkyl group, a (C6 to C10)aryl(C2 to C5) alkenyl group, a (C1 to C4)alkoxy(C1 to C3)alkyl group, a C3 to C7 substituted or unsubstituted cycloalkyl group, a substituted or unsubstituted pyridyl group, a substituted or unsubstituted thienyl group , $NR_5R_6$ (wherein each of "$R_5$" and "$R_6$" represents a hydrogen atom or a C1 to C5 alkyl group, a C1 to C4 alkoxycarbonyl group)), or "$OZ\beta$" (wherein "$Z_\beta$" represents a hydrogen atom or a C1 to C5 alkyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring, or "$COR_8$" (wherein "$R_8$" represents a C1 to C5 alkyl group, a C1 to C5 halogenated alkyl group, a substituted or unsubstituted C6 to C10 aryl group, a (C1 to C4)alkoxy(C1 to C5)alkyl group, a substituted or unsubstituted pyridyl group, a substituted or unsubstituted heterocyclic ring or aromatic ring), or "$CONR_9R_{10}$" (wherein

each of "$R_9$" and "$R_{10}$" represents a hydrogen atom or a C1 to C5 alkyl group, a substituted or unsubstituted C6 to C10 aryl group), or "$SO_2R_{11}$ (wherein "$R_{11}$" represents a hydrogen atom or a C1 to C5 alkyl group, a substituted or unsubstituted C6 to C10 aryl group)) or an insecticide having the compound.

An especially preferred embodiment is a compound wherein "Ar" represents a 3-pyridyl group, 5-thiazolyl group, or 3-tetrahydrofuryl group, "X" represents a sulfur atom, "Ya" represents "$C(=S)NR_1R_2$" (wherein each of "$R_1$" and "$R_2$" represents a hydrogen atom or a C1 to C3 alkoxycarbonyl group; "$R_1$" and "$R_2$" do not represent hydrogen atoms simultaneously.), or "$C(=O)SR_4$", "$C(=S)OR_4$" (wherein "$R_4$" represents a C1 to C5 alkyl group or a substituted or unsubstituted phenyl group, a substituted or unsubstituted pyridyl group ), or "$OZ_\beta$" (wherein "$Z_\beta$" represents "$COR_8$" (wherein "$R_8$" represents a C1 to C5 alkyl group, a C1 to C5 halogenated alkyl group, a substituted or unsubstituted C6 to C10 aryl group, a (C1 to C4)alkoxy(C1 to C5)alkyl group, a substituted or unsubstituted pyridyl group, a substituted or unsubstituted heterocyclic ring or aromatic ring), or "$CONR_9R_{10}$" (wherein each of "$R_9$" and "$R_{10}$" represents a hydrogen atom or a C1 to C5 alkyl group, a substituted or unsubstituted C6 to C10 aryl group), or "$SO_2R_{11}$" (wherein "$R_{11}$" represents a hydrogen atom or a C1 to C5 alkyl group, a substituted or unsubstituted C6 to C10 aryl group)) or an insecticide having the compound.

"Ar" represents a 2-chloro-5-pyridyl group or a 2-chloro-5-thiazolyl group, "X" represents a sulfur atom, "Ya" represents "$C(=S)NR_1R_2$", "$C(=S)SR_3$", "$C(=O)SR_4$", "$C(=S)OR_4$", or "$SO_2Z_\alpha$". When "Ya" is "$C(=S)NR_1R_2$", each of "$R_1$" and "$R_2$" represents a hydrogen atom or a C1 to C5 alkyl group, a C1 to C5 halogenated alkyl group, a C6 to C10 aryl group, a (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a C6 to C10 arylcarbonyl group, a (C1 to C3)alkylthio(C1 to C3)alkyl group, a C3 to C7 cycloalkyl group. When "Ya" is "$C(=S)SR_3$", "$R_3$" represents a Cm to C5 alkyl group, or a C6 to C10 aryl group, a (C6 to Cl0)aryl(Cl to C3)alkyl group, a (C1 to C3)alkoxy(C1 to C3)alkyl group, a (C1 to C3)alkoxycarbonyl(C1 to C3) alkyl group. When "Ya" is "$C(=O)SR_4$" or "$C(=S)OR_4$", "$R_4$" represents a C1 to C5 alkyl group, or a C6 to C10 aryl group, a pyridyl group. When "Ya" is "$SO_2Z_\alpha$", "$Z_\alpha$" represents a C2 to C5 alkyl group or a C1 to C5 halogenated alkyl group, a C2 to C5 alkenyl group, a C6 to C10 aryl group, a (C6 to C10)aryl(C1 to C3)alkyl group, a (C6 to C10)aryl(C2 to C5) alkenyl group, a (C1 to C4)alkoxy(C1 to C3)alkyl group, a C3 to C7 cycloalkyl group, a pyridyl group, a chloropyridyl group, a thienyl group, a chlorothienyl group; or it represents "$NR_5R_6$" in "$NR_5R_6$", each of "$R_5$" and "$R_6$" represents a hydrogen atom or a C1 to C5 alkyl group, a C1 to C4 alkoxycarbonyl group. When "Ya" is "$OZ_\beta$", "$Z_\beta$" represents "$R_7$", "$COR_8$", "$CONR_9R_{10}$" or "$SO_2R_{11}$". When "$Z_\beta$" is $R_7$, "$R_7$" represents a hydrogen atom or a C1 to C5 alkyl group, a C6 to C10 aryl group, a (C6 to C10)aryl(C1 to C3)alkyl group. When "$Z_\beta$" is $COR_2$, "$R_2$" represents a C1 to C5 alkyl group or a C6 to C10 aryl group, a (C1 to C4)alkoxy(C1 to C5)alkyl group, a pyridyl group. When "$Z_\beta$" is $CONR_3R_4$, each of "$R_3$" and "$R_4$" represents a hydrogen atom or a C1 to C5 alkyl group, a C6 to C10 aryl group. When "$Z_\beta$" is $SO_2R_5$, "$R_5$" represents a C1 to C5 alkyl group.

The second embodiment of the novel derivative according to the invention is represented by Formula (1) shown below. This imino derivative is further described below in detail.

[C. 40]

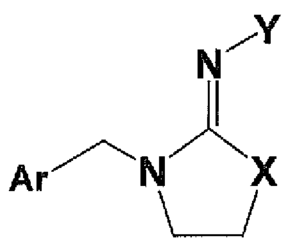

····· Formula (1)

In Formula (1), "Ar" represents a heterocyclic ring which may have a substituent on the ring, preferably a 5-membered or 6-membered heterocyclic ring. Specifically, the 5-membered or 6-membered heterocyclic ring may for example be pyridine, thiazole, tetrahydrofuran, furan, and thiophene. Those especially preferred are 3-pyridyl group, 5-thiazolyl group, and 3-tetrahydrofuryl group.

The substituent on the heterocyclic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl)amino group, and a nitro group. Preferably, the chlorine atom is used as a substituent on the heterocyclic ring.

In Formula (1), "X" represents a sulfur atom or $CH_2$, NR, and "R" represents a hydrogen atom or an alkyl group. The alkyl group may be a C1 to C4 alkyl group, which may be any of normal, secondary, and tertiary groups, and may

for example be a methyl group, an ethyl group, an n-propyl group, an isopropyl group, an n-butyl group, an isobutyl group, a sec-butyl group, and a tert-butyl group.

In Formula (1), when "Y" is "C(=S)N$R_1R_2$", each of "$R_1$" and "$R_2$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a substituted or unsubstituted C6 to C10 arylcarbonyl group, a (C1 to C3) alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridylmethyl group, a substituted or unsubstituted pyridyl group, a substituted or unsubstituted imidazolylmethyl group, or a substituted or unsubstituted heterocyclic ring or aromatic ring. "N$R_1R_2$" may form a ring.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The alkyl group of the C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the substituted phenoxy(C1 to C3)alkyl-substituted benzene ring is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 I to C4 alkyl) amino group, a nitro group, and an acetylamino group.

In Formula (1), when "Y" is "C(=S)S$R_3$", "$R_3$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C3) alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The alkyl group of the C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the substituted phenoxy(C1 to C3)alkyl-substituted phenylbenzene ring is not limited particularly, and may for example be a halogen atom (which may be any of chlorine, bromine, fluorine, and iodine)

or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.
The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

In Formula (1), when "Y" is "C(=O)S$R_4$" or "C(=S)O$R_4$", "$R_4$" represents a C1 to C5 alkyl group, or a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10) aryl(C1 to C3)alkyl group, a (C1 to C3)alkoxy(C1 to C3)alkyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted imidazolylmethyl group, a substituted or unsubstituted pyridyl group, a substituted or unsubstituted heterocyclic ring, aromatic ring.

The C1 to C5 alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.
The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.
The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.
The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent attached to the pyridine ring in the substituted pyridylmethyl group is not limited particularly, and may for example be a halogen atom (which may be any of chlorine, bromine, fluorine, and iodine) or a C1 to C3 alkyl group. The substituent attached to the benzene ring in the substituted benzenesulfonyl group is not limited particularly, and may for example be a halogen atom (which may be any of chlorine, bromine, fluorine, and iodine) or a nitro group, a di(C1 to C4 alkyl)amino group, an acetylamino group, a C1 to C3 alkyl group.

The third embodiment of the novel derivative according to the invention is a sulfonylimino derivative represented by Formula (2) shown below. This sulfonylimino derivative is further described below in detail.

[C. 41]

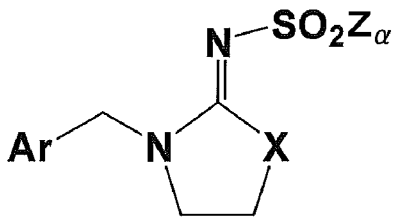

····· Formula (2)

In Formula (2), "Ar" represents a heterocyclic ring which may have a substituent on the ring, preferably a 5-membered or 6-membered heterocyclic ring. Specifically, the 5-membered or 6-membered heterocyclic ring may for example be pyridine, thiazole, tetrahydrofuran, furan, and thiophene. Those especially preferred are 3-pyridyl group, 5-thiazolyl group, and 3-tetrahydrofuryl group.

The substituent on the heterocyclic group is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl)amino group, and a nitro group. Preferably, the chlorine atom is used as a substituent on the heterocyclic ring.

In Formula (2), "X" represents a sulfur atom or $CH_2$, NR, and "R" represents a hydrogen atom or an alkyl group.

The alkyl group may be a C1 to C4 alkyl group, which may be any of normal, secondary, and tertiary groups, and may for example be a methyl group, an ethyl group, an n-propyl group, an isopropyl group, an n-butyl group, an isobutyl group, a sec-butyl group, and a tert-butyl group.

In Formula (2), "$Z_\alpha$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C6 to C10)aryl(C2 to C5) alkenyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5) alkyl group, a substituted or unsubstituted pyridylmethyl group, a substituted or unsubstituted pyridyl group, a substituted or unsubstituted thienyl group, or a substituted or unsubstituted hetero ring or aromatic ring.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The alkyl group of a C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the substituted phenoxy(C1 to C3)alkyl-substituted phenylbenzene ring is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

In Formula (2), "$Z_\alpha$" also represents "$NR_5R_6$". Each of "$R_5$" and "$R_6$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C4 alkoxy group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C1 to C4 alkoxycarbonyl group, a C3 to C7 substituted or unsubstituted cycloalkyl group, a 3-membered ring to 7-membered ring substituted or unsubstituted heterocycloalkyl group, a substituted or unsubstituted pyridyl group, a substituted or unsubstituted heterocyclic ring or aromatic ring group. "$NR_5R_6$" may form a ring.

The C1 to C5 alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent attached to the pyridine ring in the substituted pyridylmethyl group is not limited particularly, and may for example be a halogen atom (which may be any of chlorine, bromine, fluorine, and iodine) or a C1 to C3 alkyl group.

When "$NR_5R_6$" forms a ring, it is preferred that "$R_5$" and "$R_6$" are in a C2 to C6 cyclic structure. Alternatively, a 3-membered ring to 7-membered ring cyclic structure containing 1 to 2 hetero atoms such as oxygen, sulfur, and

nitrogen atoms in the ring of the 3-membered ring to 7-membered ring as "$NR_5R_6$" is preferred.

The fourth embodiment of the novel derivative according to the invention is an oxime ether derivative represented by Formula (3) shown below. This oxime ether derivative is further described below in detail.

[C. 42]

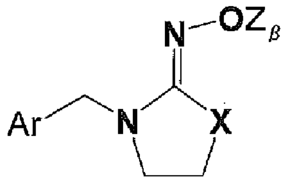

····· Formula (3)

In Formula (3), "Ar" represents a heterocyclic ring which may have a substituent on the ring, preferably a 5-membered or 6-membered heterocyclic ring. Specifically, the 5-membered or 6-membered heterocyclic ring may for example be pyridine, thiophene, tetrahydrofuran, furan, and thiazole. Those especially preferred are 3-pyridyl group, 5-thiazolyl group, and 3-tetrahydrofuryl group.

The substituent on the heterocyclic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl)amino group, and a nitro group. Preferably, the chlorine atom is used as a substituent on the heterocyclic ring.

In Formula (3), "X" represents a sulfur atom or $CH_2$, NR. "R" represents a hydrogen atom or an alkyl group. The alkyl group may be a C1 to C4 alkyl group, which may be any of normal, secondary, and tertiary groups, and may for example be a methyl group, an ethyl group, an n-propyl group, an isopropyl group, an n-butyl group, an isobutyl group, a sec-butyl group, and a tert-butyl group.

In Formula (3), when "$Z_\beta$" represents $R_7$, "$R_7$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The alkyl group of a C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

A C3 to C7 substituted or unsubstituted cycloalkyl group may for example be a cyclopropyl group, a cyclobutyl group, a cyclopentyl group, and a cyclohexyl group. The substituent is not limited particularly, and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the phenyl group in the substituted phenoxy(C1 to C3)alkyl group is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl)amino group, a nitro group, and an acetylamino group.

In Formula (3), when "$Z_\beta$" represents $COR_8$, "$R_8$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4) alkoxy(C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy (C1 to C3)alkyl group, a substituted or unsubstituted pyridyl group, a substituted or unsubstituted pyridylmethyl group, a substituted or unsubstituted imidazolylmethyl group, a substituted or unsubstituted heterocyclic ring or aromatic ring.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.

The alkyl group of a C1 to C5 halogenated alkyl group may be any of normal, secondary, and tertiary groups, and may for example be an ethyl group, an n-propyl group, an isopropyl group, an n-butyl group, an isobutyl group, a sec-butyl group, and a tert-butyl group.

A C3 to C7 substituted or unsubstituted cycloalkyl group may for example be a cyclopropyl group, a cyclobutyl group, a cyclopentyl group, and a cyclohexyl group. The substituent is not limited particularly, and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent on the phenyl group in the substituted phenoxy(C1 to C3)alkyl group is not limited particularly, and may for example be a halogen atom (which may be any of chlorine, bromine, fluorine, and iodine) or a C1 to C3 alkyl group. The substituent attached to the pyridine ring in the substituted pyridylmethyl group or the imidazole ring in the substituted imidazolylmethyl group is not limited particularly, and may for example be a halogen atom (which may be any of chlorine, bromine, fluorine, and iodine) or a C1 to C3 alkyl group.

The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

In Formula (3), when "$Z_\beta$" represents $CONR_9R_{10}$, each of "$R_9$" and "$R_{10}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a C1 to C3 alkoxycarbonyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted pyridyl group, a substituted or unsubstituted imidazolidylmethyl group, a substituted or unsubstituted heterocyclic ring or aromatic ring. "$NR_9R_{10}$" may form a ring.

The C1 to C5 alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.

The C3 to C7 substituted or unsubstituted cycloalkyl group may for example be a cyclopropyl group, a cyclobutyl group, a cyclopentyl group, and a cyclohexyl group. The substituent is not limited particularly and may for example be a halogen atom (which may be any of chlorine, bromine, fluorine, and iodine) or a C1 to C3 alkyl group.

The 3-membered ring to 7-membered ring substituted or unsubstituted heterocycloalkyl group is preferably a heterocycloalkyl group containing 1 to 2 hetero atoms such as oxygen, sulfur, and nitrogen atoms. The substituent is not limited particularly, and may for example be a halogen atom (which may be any of chlorine, bromine, fluorine, and iodine) or a C1 to C3 alkyl group. The substituent attached to the pyridine ring in the substituted pyridylmethyl group is not limited particularly, and may for example be a halogen atom (which may be any of chlorine, bromine, fluorine, and iodine) or a C1 to C3 alkyl group. The substituent attached to the benzene ring in the substituted benzenesulfonyl group is not limited particularly, and may for example be a halogen atom (which may be any of chlorine, bromine, fluorine, and iodine) or a

nitro group, a di(C1 to C4 alkyl)amino group, an acetylamino group, a C1 to C3 alkyl group.
The heterocyclic ring may for example be a quinoline ring, an indole ring, a pyridine ring, a pyrazine ring, a pyridazine ring, a pyrimidine ring, a thiophene ring, a thiazole ring, a tetrahydrofuran ring, and a furan ring. Those especially preferred are 3-pyridyl group, 5-thiazolyl group, and 3-tetrahydrofuryl group. The substituent on the heterocyclic ring or the aromatic ring is not limited particularly, and may for example be a halogen atom (which may be any of fluorine, chlorine, bromine, and iodine), a C1 to C4 alkyl group, a C1 to C4 halogenated alkyl group, a C1 to C4 alkoxy group, a di(C1 to C4 alkyl) amino group, a nitro group, and an acetylamino group.

When "$NR_9R_{10}$" forms a ring, it is preferred that "$R_9$" and "$R_{10}$" are in a C2 to C6 cyclic structure, or a 3-membered ring to 7-membered ring cyclic structure containing 1 to 2 hetero atoms such as oxygen, sulfur, and nitrogen atoms in the ring of the 3-membered ring to 7-membered ring as "$NR_9R_{10}$" is preferred.

In Formula (3), when "$Z_\beta$" represents $SO_2R_{11}$, "$R_{11}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted imidazolidylmethyl group, a substituted or unsubstituted thienyl group, or a substituted or unsubstituted heterocyclic ring or aromatic ring.

The C1 to C5 alkyl group may be any of normal, secondary, and tertiary groups, and may for example be a methyl group, an ethyl group, an n-propyl group, an isopropyl group, an n-butyl group, an isobutyl group, a sec-butyl group, and a tert-butyl group.
The C1 to C5 halogenated alkyl group may be any of normal, secondary, and tertiary groups, and those which are preferred may for example be a methyl group, an ethyl group, an n-propyl group, an isopropyl group, an n-butyl group, an isobutyl group, a sec-butyl group, and a tert-butyl group.
A C3 to C7 substituted or unsubstituted cycloalkyl group may for example be a cyclopropyl group, a cyclobutyl group, a cyclopentyl group, and a cyclohexyl group. The substituent is not limited particularly, and may for example be a halogen atom (which may be any of chlorine, bromine, fluorine, and iodine) or a C1 to C3 alkyl group. The substituent attached to the benzene ring in the substituted phenyl group is not limited particularly, and may for example be a halogen atom (which may be any of chlorine, bromine, fluorine, and iodine) or a nitro group, a di(C1 to C4 alkyl)amino group, an acetylamino group, a C1 to C3 alkyl group.

A novel imino derivative according to the invention has an excellent insecticidal activity, acaricidal activity and the like against a broad range of agricultural pests and household pests. Accordingly, it can be used as an insecticide against agricultural pests and household pests, an acaricide, a domestic pest controlling agent against a termite and the like, or a veterinarian drug, which contains such a compound as an active ingredient.

2. Methods for producing imino derivatives

Methods for producing imino derivatives according to the invention are described below.

(2-1) Method for producing compound represented by Formula (7) wherein "Ya" in Formula (A) is "$C(=S)NR_1R_2$"

A compound represented by Formula (7) shown below can be produced by reacting a compound represented by Formula (4) shown below with a compound represented by Formula (5) shown below or a compound represented by Formula (6) shown below in the presence of a base (see Reaction Schemes (1) and (2)):

Reaction Scheme (1)

[C. 43]

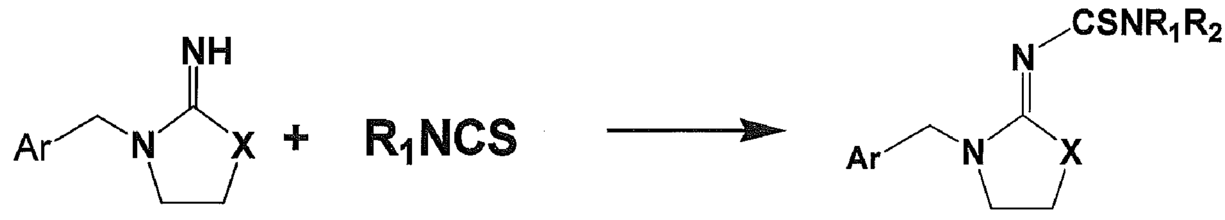

Formula (4) Formula (5) Formula (7)

Reaction Scheme (2)

[C. 44]

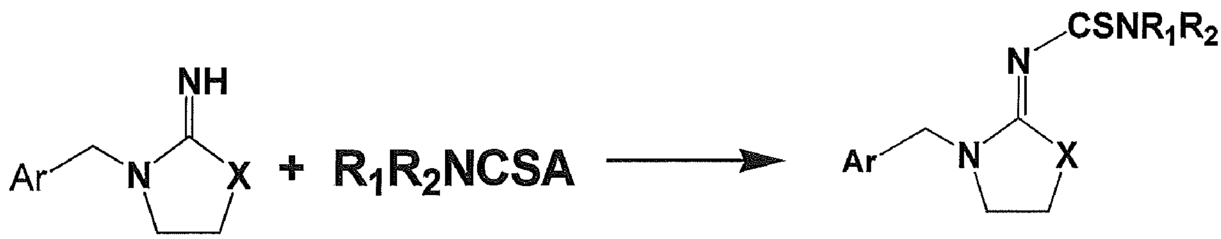

Formula (4) Formula (6) Formula (7)

wherein, "Ar" "X" "$R_1$", "$R_2$" represents the same meanings as "Ar" "X" "$R_1$", "$R_2$" in Formula (A) shown above. "A" represents a halogen atom such as chlorine, bromine, or iodine; and, "$R_2$" represents a hydrogen atom.

In this production step, 1 mole of a compound represented by Formula (4) is reacted with 1.0 to 3.0 moles of an isothiocyanate represented by Formula (5) or a thiocarbamic acid halide represented by Formula (6), thereby producing 1 mole of a compound represented by Formula (7).

In this production step, the amount of the compound represented by Formula (5) or the compound represented by Formula (6) to be added is preferably 1.0 to 2.0 moles per mole of the compound represented by Formula (4).

While the base employed in this production step is not limited particularly, those employed preferably include alkaline metal hydrides such as sodium hydride (NaH), acetates such as sodium acetate and potassium acetate, or carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, alcohols such as ethanol and propanol, and aliphatic hydrocarbons such as hexane, heptane, and octane, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction time may be adjusted appropriately in view of temperature, pressure or other conditions, 30 minutes to 24 hours are employed preferably.

(2-2) Method for producing compound represented by Formula (7) wherein "Ya" in Formula (A) is "C(=S)$NR_1R_2$" and "$R_2$" is a hydrogen atom

A compound represented by Formula (7) shown below ($R_2$=H) can be produced by reacting a compound represented by Formula (9) shown below, which is produced by reacting a compound represented by Formula (4) shown below with a compound represented by Formula (8) shown below, with a compound represented by Formula (10) shown below (see Reaction Schemes (3) and (4)):

Reaction Scheme (3)

[C. 45]

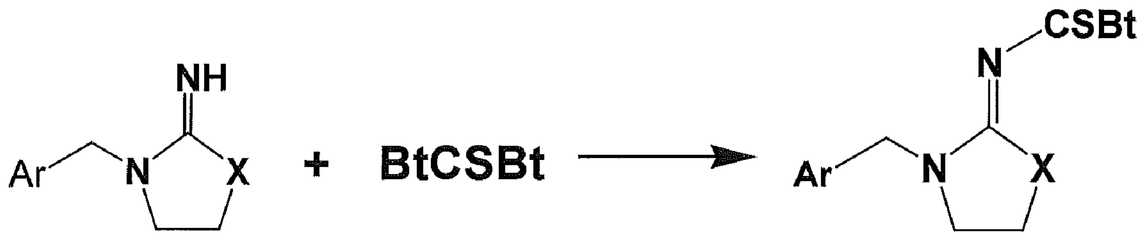

Formula (4) Formula (8) Formula (9)

Reaction Scheme (4)

[C. 46]

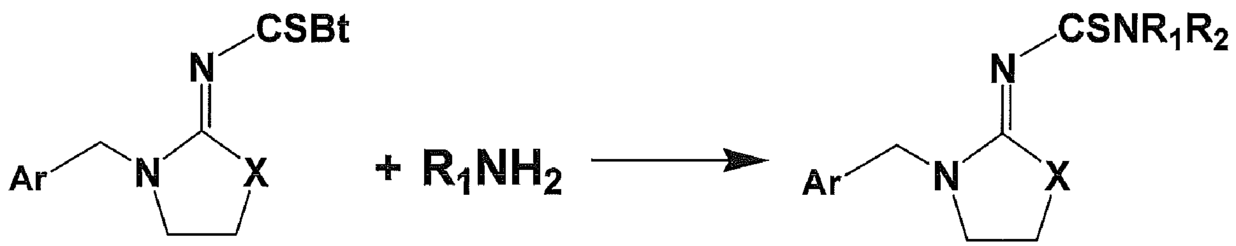

Formula (9) Formula (10) Formula (7)

wherein, "Ar", "X", "$R_1$" represents the same meanings as "Ar", "X", "$R_1$" in Formula (A) shown above. "Bt" represents 1-(1,2,3-benzotriazol); and, "$R_2$" represents a hydrogen atom.

In this production step, the amount of the compound represented by Formula (8) to be added is preferably 1.0 to 1.2 moles per mole of the compound represented by Formula (4). The amount of the compound represented by Formula (10) to be added is preferably 1.0 to 1.2 moles per mole of the compound represented by Formula (9).

While the base employed in this production step is not limited particularly, those employed preferably include alkaline metal hydrides such as sodium hydride (NaH), carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, alcohols such as ethanol and propanol, ketones such as acetone and methyl ethyl ketone, aliphatic hydrocarbons such as hexane, heptane, and octane, halogenated hydrocarbons such as dichloromethane, chloroform, chlorobenzene, and

dichlorobenzene, and water, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction can be conducted under reduced pressure, at atmospheric temperature, or under pressure, it is conducted preferably at atmospheric temperature. While the reaction time may be adjusted appropriately in view of temperature, pressure, or other conditions, 30 minutes to 24 hours are employed preferably.

(2-3) Method for producing a compound represented by Formula (14) wherein "Ya" in Formula (A) is "C(=S)$SR_3$"

A compound represented by Formula (14) shown below can be produced by reacting a compound represented by Formula (4) shown below with a compound represented by Formula (11) shown below, a compound represented by Formula (12) shown below, or a compound represented by Formula (13) shown below in the presence of a base (see Reaction Schemes (5) to (7)):

Reaction Scheme (5)

[C. 47]

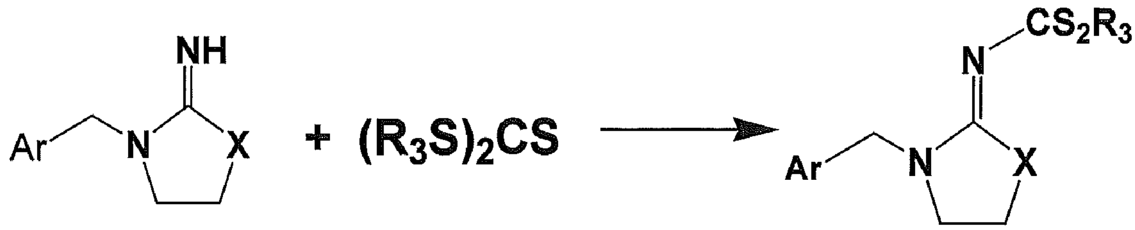

Formula (4) Formula (11) Formula (14)

Reaction Scheme (6)

[C. 48]

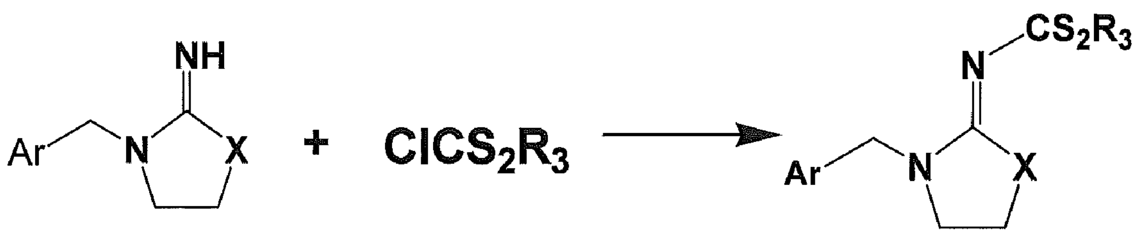

Formula (4) Formula (12) Formula (14)

Reaction Scheme (7)

[C. 49]

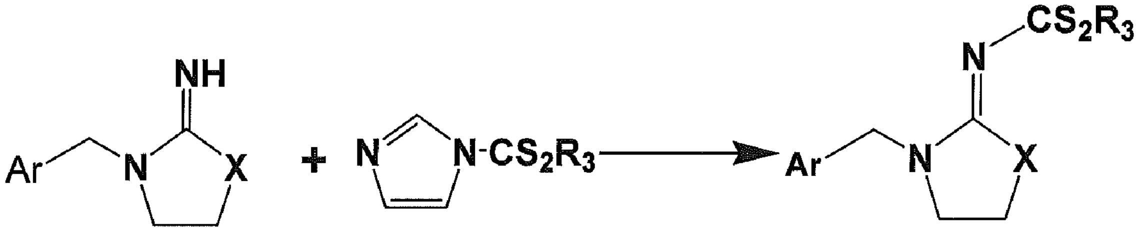

Formula (4) Formula (13) Formula (14)

wherein, "Ar", "X", "$R_3$" represents the same meanings as "Ar", "X", "$R_3$" in Formula (A) shown above.

In this production step, 1 mole of a compound represented by Formula (4) is reacted with 1 to 2 moles of a compound represented by Formula (11) to Formula (13), thereby producing 1 mole of a compound represented by Formula (14).

In this production step, the amount of the compound represented by Formula (11) to (13) to be added is preferably 1.0 to 1.2 moles per mole of the compound represented by Formula (4).

While the base employed in this production step is not limited particularly, those employed preferably include alkaline metal hydrides such as sodium hydride (NaH), carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, alcohols such as ethanol and propanol, ketones such as acetone and methyl ethyl ketone, aliphatic hydrocarbons such as hexane, heptane, and octane, halogenated hydrocarbons such as dichloromethane, chloroform, chlorobenzene, and dichlorobenzene, and water, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction can be conducted under reduced pressure, at atmospheric temperature, or under pressure, it is conducted preferably at atmospheric temperature. While the reaction time may be adjusted appropriately in view of temperature, pressure, or other conditions, 30 minutes to 24 hours are employed preferably.

(2-4) Method for producing a compound represented by Formula (16) wherein "Ya" in Formula (A) is "C(=O)$SR_4$" or "C (=S)$OR_4$"

A compound represented by Formula (16) shown below can be produced by reacting a compound represented by Formula (4) shown below with a compound represented by Formula (15) shown below in the presence of a base (see Reaction Scheme (8)):

Reaction Scheme (8)

[C. 50]

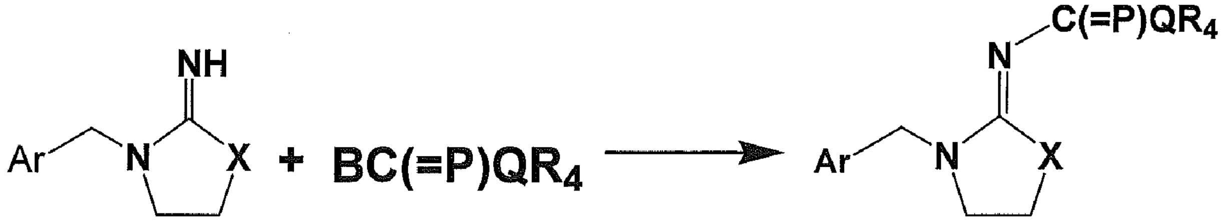

Formula (4) Formula (15) Formula (16)

wherein, "Ar", "X", "$R_3$" are defined as described above; "B" represents a halogen atom such as chlorine, bromine, or iodine; each of "P", "Q" represents a sulfur atom or an oxygen atom; and, provided that "P" and "Q" are different from each other.

In this production step, the amount of the compound represented by Formula (15) to be added is preferably 1.0 to 1.2 moles per mole of the compound represented by Formula (4).

While the base employed in this production step is not limited particularly, those employed preferably include alkaline metal hydrides such as sodium hydride (NaH), carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, alcohols such as ethanol and propanol, ketones such as acetone and methyl ethyl ketone, aliphatic hydrocarbons such as hexane, heptane, and octane, halogenated hydrocarbons such as dichloromethane, chloroform, chlorobenzene, and dichlorobenzene, and water, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction can be conducted under reduced pressure, at atmospheric temperature, or under pressure, it is conducted preferably at atmospheric temperature. While the reaction time may be adjusted appropriately in view of temperature, pressure, or other conditions, 30 minutes to 24 hours are employed preferably.

By employing the production methods according to the invention described above, the imino derivatives represented by Formula (A) according to the invention can be produced using relatively versatile substances as starting materials.

The compounds described in Examples shown below are encompassed more specifically within the definitions described below. Such compounds are especially preferred when they are employed as insecticides.

Thus, an imino derivative represented by Formula (1) shown below:

[C. 51]

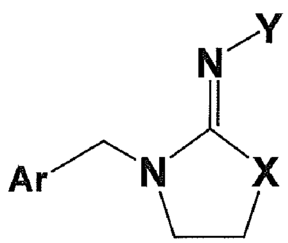

..... Formula (A)

wherein "Ar" represents a 2-chloro-5-pyridyl group or a 2-chloro-5-thiazolyl group; "X" represents a sulfur atom; "Y" represents "$C(=S)NR_1R_2$", "$C(=S)SR_3$", "$C(=O)SR_4$", or "$C(=S)OR_4$"; when "Y" is "$C(=S)NR_1R_2$", each of "$R_1$" and "$R_2$" represents a hydrogen atom or a C1 to C5 alkyl group, a C1 to C5 halogenated alkyl group, a C6 to C10 aryl group, a (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a C6 to C10 arylcarbonyl group, a (C1 to C3)alkylthio(C1 to C3)alkyl group, a C3 to C7 cycloalkyl group; when "Y" is "$C(=S)SR_3$", "$R_3$" represents a C1 to C5 alkyl group, or a C6 to C10 aryl group, a (C6 to C10)aryl(C1 to C3)alkyl group, a (C1 to C3)alkoxy(C1 to C3)alkyl group, a (C1 to C3)alkoxycarbonyl (C1 to C3)alkyl group; and,
when "Y" is "$C(=O)SR_4$" or "$C(=S)OR_4$", "$R_4$" represents a C1 to C5 alkyl group, or a C6 to C10 aryl group, a pyridyl group.

Among these compounds, a compound represented by Formula (7) wherein "Ya" is "$C(=S)NR_1R_2$" can be produced by reacting a compound represented by Formula (4) shown below with a compound represented by Formula (5) shown below or a compound represented by Formula (6) shown below:

[C. 52]

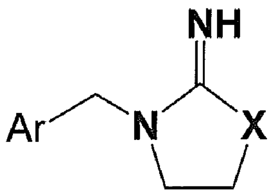

····· Formula (4)

[C. 53] **$R_1$NCS** Formula (5)

[C. 54] **$R_1R_2$NCSA** Formula (6)

[C. 55]

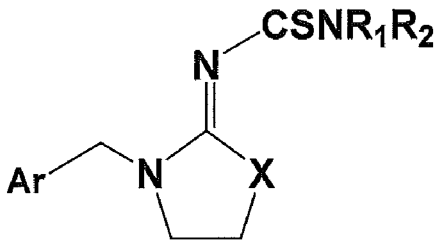

····· Formula (7)

wherein "Ar" represents a 2-chloro-5-pyridyl group or a 2-chloro-5-thiazolyl group; "X" represents a sulfur atom; each of "$R_1$" and "$R_2$" represents a hydrogen atom or a C1 to C5 alkyl group, a C1 to C5 halogenated alkyl group, a C6 to C10 aryl group, a (C6 to C10)aryl(C1 to C3)alkyl group, a (C1 to C4)alkoxy(Cl to C5)alkyl group, a C1 to C3 alkoxycarbonyl group, a C1 to C3 alkylcarbonyl group, a C6 to C10 arylcarbonyl group, a (C1 to C3)alkylthio(C1 to C3)alkyl group, a C3 to C7 cycloalkyl group; and,

"A" represents a halogen atom such as chlorine, bromine, or iodine.

A compound represented by Formula (7) wherein "Ya" is "C(=S)$NR_1R_2$" and "$R_2$" is a hydrogen atom can be produced by reacting a compound represented by Formula (9) shown below, which is produced by reacting a compound represented by Formula (4) shown below with a compound represented by Formula (8) shown below, with a compound represented by Formula (10) shown below:

[C. 56]

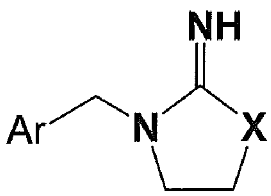

····· Formula (4)

[C. 57] **BtCSBt** Formula (8)

[C. 58]

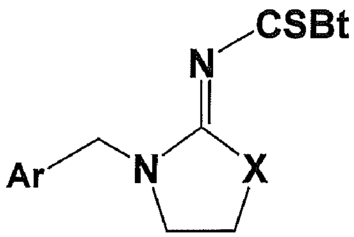

..... Formula (9)

[C. 59] $R_1NH_2$ Formula (10)

[C. 60]

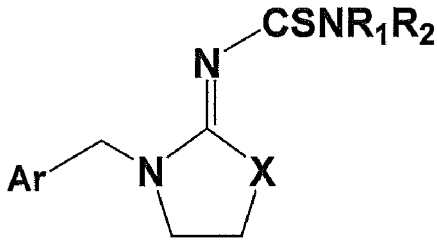

..... Formula (7)

wherein "Ar" represents a 2-chloro-5-pyridyl group or a 2-chloro-5-thiazolyl group; "X" represents a sulfur atom; "Bt" represents 1-(1,2,3-benzotriazol); and,

"$R_1$" represents a C1 to C5 alkyl group, a C1 to C5 halogenated alkyl group, a C6 to C10 aryl group, an aryl(C1 to C3)alkyl group, a (C1 to C4)alkoxy(C1 to C5)alkyl group, a C1 to C3 alkoxycarbonyl group, a (C1 to C3)alkylthio (C1 to C3)alkyl group, a C3 to C7 cycloalkyl group. "$R_2$" represents a hydrogen atom.

A compound represented by Formula (14) wherein "Ya" is "$C(=S)SR_3$" can be produced by reacting a compound represented by Formula (4) shown below with a compound represented by Formula (11) shown below, a compound represented by Formula (12) shown below, or a compound represented by Formula (13) shown below:

[C. 61]

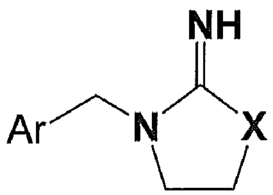

..... Formula (4)

[C. 62] $(R_3S)_2CS$ Formula (11)

[C. 63] $ClCS_2R_3$ Formula (12)

[C. 64]

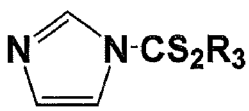

..... Formula (13)

[C. 65]

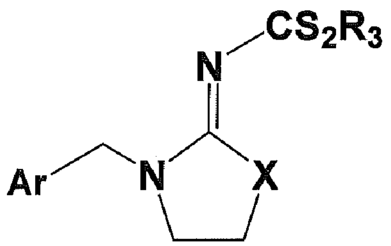

····· Formula (14)

wherein "Ar" represents a 2-chloro-5-pyridyl group or a 2-chloro-5-thiazolyl group; "X" represents a sulfur atom; and, "$R_3$" represents a C1 to C5 alkyl group, or a C6 to C10 aryl group, a (C6 to C10)aryl(C1 to C3)alkyl group, a (C1 to C3)alkoxy(C1 to C3)alkyl group, a (C1 to C3)alkoxycarbonyl(C1 to C3)alkyl group.

A compound represented by Formula (16) wherein "Ya" is "$C(=O)SR_4$" or "$C(=S)OR_4$" can be produced by reacting a compound represented by Formula (4) shown below with a compound represented by Formula (15) shown below:

[C. 66]

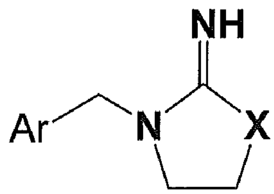

····· Formula (4)

[C.67] **$BC(=P)QR_4$** Formula (15)

[C. 68]

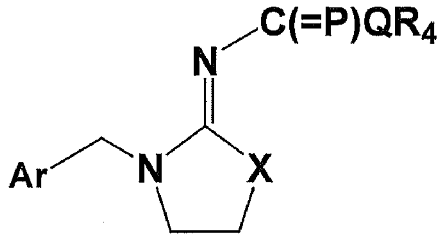

····· Formula (16)

wherein "Ar" represents a 2-chloro-5-pyridyl group or a 2-chloro-5-thiazolyl group; "X" represents a sulfur atom; "B" represents a halogen atom such as chlorine, bromine, or iodine; each of "P", "Q" represents a sulfur atom or an oxygen atom; provided that "P" and "Q" are different from each other; and,
"$R_4$" represents a C1 to C5 alkyl group, or a C6 to C10 aryl group.

(2-5) Method for producing a sulfonylimino derivative represented by Formula (2) wherein "Ya" in Formula (A) is "$SO_2Z_\alpha$"

Methods for producing sulfonylimino derivatives according to the invention are described below.
A compound represented by Formula (2) shown below can be produced by reacting a compound represented by Formula (4) shown below with a compound represented by Formula (17) shown below in the presence of a base (see Reaction Scheme (9)):
Reaction Scheme (9)

[C. 69]

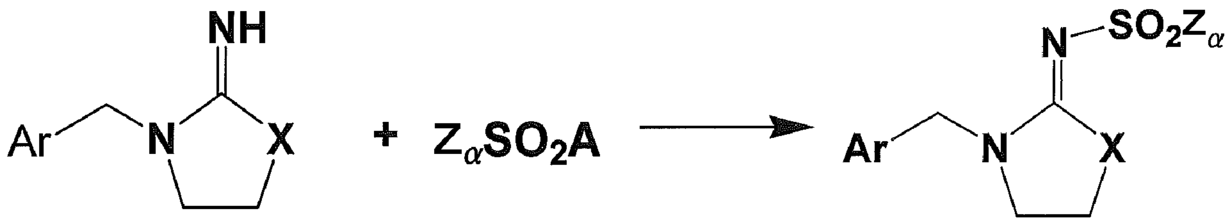

Formula (4) Formula (17) Formula (2)

wherein, "Ar", "X", "$Z_\alpha$" are defined as described above; and, "A" represents a halogen atom such as chlorine, bromine, or iodine or "$OSO_2Z_\alpha$".

In this production step, 1 mole of a compound represented by Formula (4) is reacted with 1.0 to 3.0 moles of a derivative represented by Formula (17), thereby producing 1 mole of a compound represented by Formula (2).

In this production step, the amount of the compound represented by Formula (17) to be added is preferably 1.0 to 2.0 moles per mole of the compound represented by Formula (4).

While the base employed in this production step is not limited particularly, those employed preferably include alkaline metal hydrides such as sodium hydride (NaH), acetates such as sodium acetate and potassium acetate, or carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, aliphatic hydrocarbons such as hexane, heptane, and octane, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction time may be adjusted appropriately in view of temperature, pressure, or other conditions, 30 minutes to 24 hours are employed preferably.

By employing the production methods according to the invention described above, the sulfonylimino derivatives represented by Formula (2) according to the invention can be produced using relatively versatile substances as starting materials.

The compounds described in Examples shown below are encompassed more specifically within the definitions described below. Such compounds are especially preferred when they are employed as insecticides.

Thus, a sulfonylimino derivative represented by Formula (2) shown below:

[C. 70]

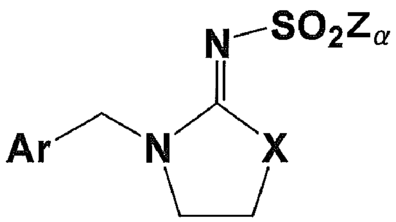

····· Formula (2)

wherein "Ar" represents a 2-chloro-5-pyridyl group or a 2-chloro-5-thiazolyl group;
"X" represents a sulfur atom;
"$Z_\alpha$" represents C2 to C5 alkyl group or a C1 to C5 halogenated alkyl group, a C2 to C5 alkenyl group, a C6 to C10

aryl group, a (C6 to C10)aryl(C1 to C3)alkyl group, a (C6 to C10)aryl(C2 to C5)alkenyl group, a (C1 to C4)alkoxy (C1 to C3)alkyl group, a C3 to C7 cycloalkyl group, a pyridyl group, a chloropyridyl group, a thienyl group, a chlorothienyl group; or it represents "$NR_5R_6$"; and, in "$NR_5R_6$", each of "$R_5$" and "$R_6$" represents a hydrogen atom or a C1 to C5 alkyl group, a C1 to C4 alkoxycarbonyl group.

These compounds can be produced by reacting compounds represented by Formula (4) shown below with compounds represented by Formula (17) shown below:

[C. 71]

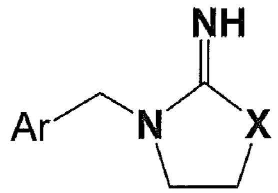

..... Formula (4)

[C. 72] $\mathbf{Z_{\alpha}SO_2A}$ Formula (17)

[C. 73]

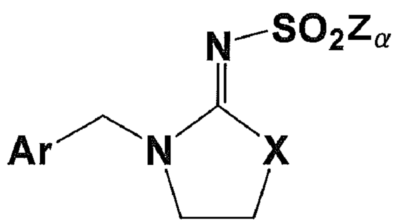

..... Formula (2)

wherein "Ar" represents a 2-chloro-5-pyridyl group or a 2-chloro-5-thiazolyl group;
"X" represents a sulfur atom;
"$Z_{\alpha}$" represents C2 to C5 alkyl group or a C1 to C5 halogenated alkyl group, a C2 to C5 alkenyl group, a C6 to C10 aryl group, a (C6 to C10)aryl(C1 to C3)alkyl group, a (C6 to C10)aryl(C2 to C5)alkenyl group, a (C1 to C4)alkoxy (C1 to C3)alkyl group, a C3 to C7 cycloalkyl group, a pyridyl group, a chloropyridyl group, a thienyl group, a chlorothienyl group; or it represents "$NR_5R_6$" in "$NR_5R_6$", each of "$R_5$" and "$R_6$" represents a hydrogen atom or a C1 to C5 alkyl group, a C1 to C4 alkoxycarbonyl group; and,
"A" represents a halogen atom such as chlorine, bromine, or iodine or "$OSO_2Z_{\alpha}$".

(2-6) Method for producing a compound represented by Formula (19) wherein "Ya" in Formula (A) represents "$OZ_{\beta}$" and "$Z_{\beta}$" represents "$R_7$"

A compound represented by Formula (19) shown below can be produced by reacting a compound represented by Formula (4) shown below with a compound represented by Formula (18) shown below in the presence of a base (see Reaction Scheme (10)):
Reaction Scheme (10)

[C. 74]

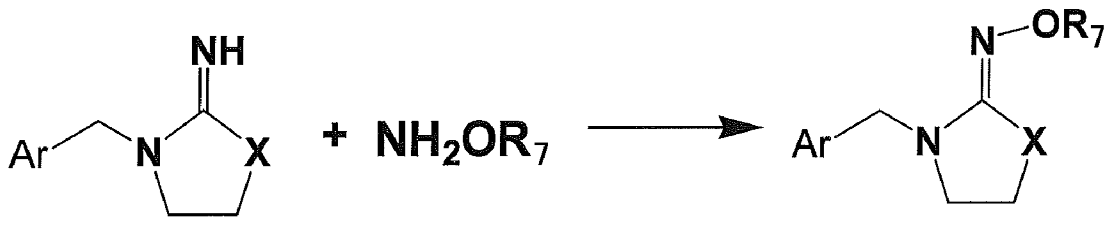

Formula (4) Formula (18) Formula (19)

wherein "Ar", "X", "$R_7$" are defined as described above.

In this production step, 1 mole of a compound represented by Formula (4) is reacted with 1.0 to 3.0 moles of a hydroxyamine derivative represented by Formula (18), thereby producing 1 mole of a compound represented by Compound (19).

In this production step, the amount of a compound represented by Formula (18) to be added is preferably 1.0 to 2.0 moles per 1 mole of a compound represented by Formula (4).

While the base employed in this production step is not limited particularly, those employed preferably include alkaline metal hydrides such as sodium hydride (NaH), acetates such as sodium acetate and potassium acetate, or carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, alcohols such as ethanol and propanol, and aliphatic hydrocarbons such as hexane, heptane, and octane, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction time may be adjusted appropriately in view of temperature, pressure or other conditions, 30 minutes to 24 hours are employed preferably.

(2-7) Method for producing a compound represented by Formula (23) wherein "Ya" in Formula (A) represents "$OZ_\beta$" and "$Z_\beta$" represents "$COR_8$"

A compound represented by Formula (23) shown below can be produced by reacting a compound represented by Formula (20) shown below with a compound represented by Formula (21) shown below or a compound represented by Formula (22) shown below in the presence of a base (see Reaction Schemes (11) and (12)). Herein, a compound represented by Formula (20) is a compound represented by Formula (19) wherein "$R_7$" represents a hydrogen atom.

Reaction Scheme (11)

[C. 75]

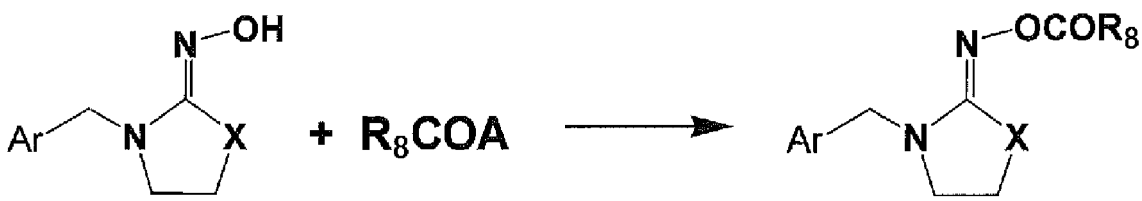

Formula (20) Formula (21) Formula (20)

Reaction Scheme (12)

[C. 76]

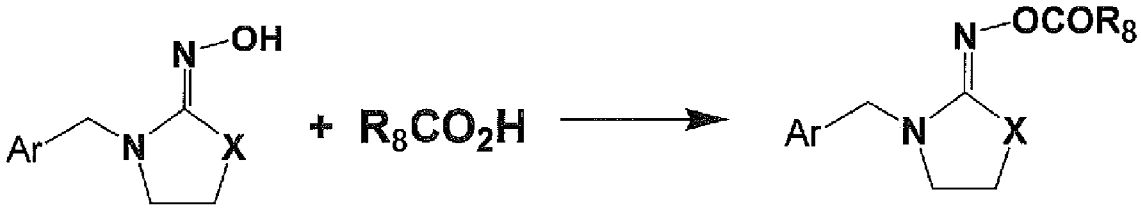

Formula (20) Formula (22) Formula (23)

wherein "Ar", "X", "$R_8$" are defined as described above; and, "A" represents a halogen atom such as chlorine, bromine, and iodine.

In this production step, 1 mole of a compound represented by Formula (20) is reacted with 1 mole to 2 moles of an acid halide compound represented by Formula (21) or 1 mole to 2 moles of an acid represented by Formula (22), thereby producing 1 mole of a compound represented by Formula (23).

In this production step, the amount of a compound represented by Formula (21) or a compound represented by Formula (22) to be added is preferably 1.0 to 1.2 moles per 1 mole of a compound represented by Formula (20).

While the base employed in this production step is not limited particularly, those employed preferably include alkaline metal hydrides such as sodium hydride (NaH), carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, alcohols such as ethanol and propanol, ketones such as acetone and methyl ethyl ketone, aliphatic hydrocarbons such as hexane, heptane, and octane, halogenated hydrocarbons such as dichloromethane, chloroform, chlorobenzene, and dichlorobenzene, and water, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction can be conducted under reduced pressure, at atmospheric temperature, or under pressure, it is conducted preferably at atmospheric temperature. While the reaction time may be adjusted appropriately in view of temperature, pressure, or other conditions, 30 minutes to 24 hours are employed preferably.

(2-8) Method for producing a compound represented by Formula (26) wherein "Ya" in Formula (A) represents "$OZ_\beta$" and "$Z_\beta$" represent "$CONR_9$ $R_{10}$"

A compound represented by Formula (26) shown below can be produced by reacting a compound represented by Formula (20) shown below with a compound represented by Formula (24) shown below or a compound represented by Formula (25) shown below in the presence of a base (see Reaction Schemes (13) and (14)). Herein, a compound represented by Formula (20) is a compound represented by Formula (19) wherein "$R_7$" represents a hydrogen atom.

Reaction Scheme (13)

[C. 77]

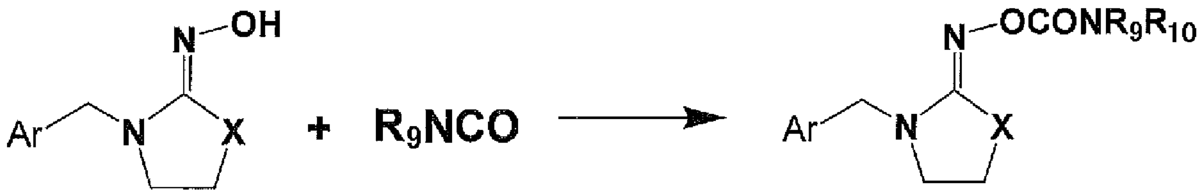

Formula (20) Formula (24) Formula (26)

Reaction Scheme (14)

[C. 78]

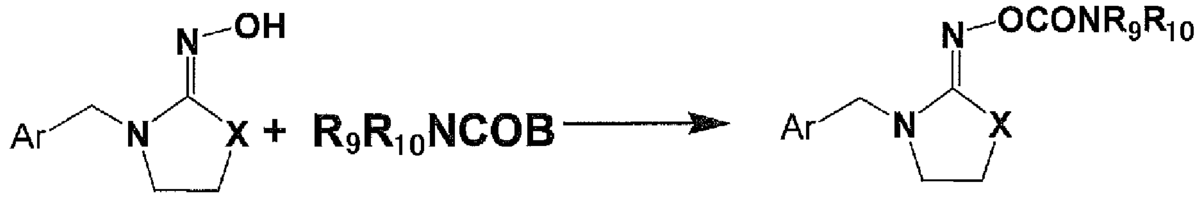

Formula (20) Formula (25) Formula (26)

wherein "Ar", "X", "$R_9$", and "$R_{10}$" are defined as described above; and, "B" represents a halogen atom such as chlorine, bromine, and iodine.

In this production step, 1 mole of a compound represented by Formula (20) is reacted with 1 to 2 moles of an isocyanate compound represented by Formula (24) or a carbamic acid halide represented by Formula (25), thereby producing 1 mole of a compound represented by Formula (26).

In this production step, the amount of a compound represented by Formula (24) or a compound represented by Formula (25) to be added is preferably 1.0 to 1.2 moles per 1 mole of a compound represented by Formula (20).

While the base employed in this production step is not limited particularly, those employed preferably include alkaline metal hydrides such as sodium hydride (NaH), carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, alcohols such as ethanol and propanol, ketones such as acetone and methyl ethyl ketone, aliphatic hydrocarbons such as hexane, heptane, and octane, halogenated hydrocarbons such as dichloromethane, chloroform, chlorobenzene, and dichlorobenzene, and water, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction can be conducted under reduced pressure, at atmospheric temperature, or under pressure, it is conducted preferably at atmospheric temperature. While the reaction time may be adjusted appropriately in view of temperature, pressure, or other conditions, 30 minutes to 24 hours are employed preferably.

(2-9) Method for producing a compound represented by Formula (28) wherein "Ya" in Formula (A) represents "$OZ_\beta$" and "$Z_\beta$" represents "$SO_2$ $R_{11}$"

A compound represented by Formula (28) shown below can be produced by reacting a compound represented

by Formula (20) shown below with a compound represented by Formula (27) shown below in the presence of a base (see Reaction Scheme (15)). Herein, a compound represented by Formula (20) is a compound represented by Formula (19) wherein "$R_1$" represents a hydrogen atom.

Reaction Scheme (15)

[C. 79]

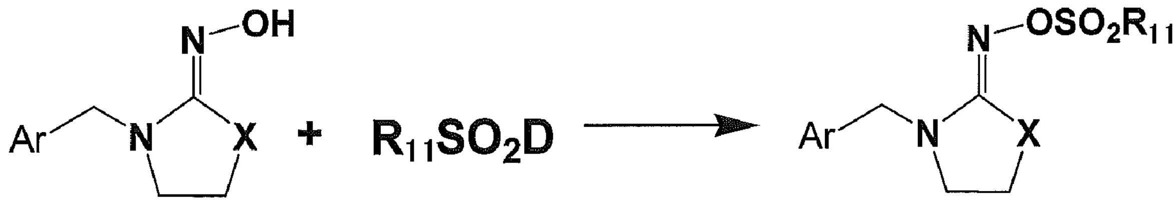

Formula (20) ormula (27) ormula (28)

wherein "Ar", "X", "$R_{11}$" are defined as described above; and, "D" represents a halogen atom such as chlorine, bromine, and iodine.

In this production step, 1 mole of a compound represented by Formula (20) is reacted with 1 to 2 moles of a sulfonyl compound represented by Formula (27), thereby producing 1 mole of a compound represented by Formula (28).

In this production step, the amount of a compound represented by Formula (27) to be added is preferably 1.0 to 1.2 moles per 1 mole of a compound represented by Formula (20).

While the base employed in this production step is not limited particularly, those employed preferably include alkaline metal hydrides such as sodium hydride (NaH), carbonates such as potassium carbonate ($K_2CO_3$) and sodium carbonate ($Na_2CO_3$), alkaline metal hydroxides such as potassium hydroxide (KOH) and sodium hydroxide (NaOH), and tertiary amines such as triethylamine [$(C_2H_5)_3N$].

In this production step, it is preferred to employ a solvent, which may for example be amides such as dimethyl formamide (DMF) and dimethyl acetamide, nitriles such as acetonitrile, sulfoxides such as dimethyl sulfoxide (DMSO), ethers such as diethyl ether and tetrahydrofuran (THF), aromatic hydrocarbons such as benzene, xylene, and toluene, ketones such as acetone, and methyl ethyl ketone, aliphatic hydrocarbons such as hexane, heptane, and octane, halogenated hydrocarbons such as dichloromethane, chloroform, chlorobenzene, and dichlorobenzene, and water, which solvents may be employed alone or in combination of two or more thereof.

The reaction temperature in this production step is preferably 0 to 200°C. A reaction temperature below 0°C leads to a low reaction rate, while that exceeding 200°C leads to a too high reaction rate, which is disadvantageous because it allows a side reaction to be proceeded easily. While the reaction can be conducted under reduced pressure, at atmospheric temperature, or under pressure, it is conducted preferably at atmospheric temperature. While the reaction time may be adjusted appropriately in view of temperature, pressure, or other conditions, 30 minutes to 24 hours are employed preferably.

By employing the production methods according to the invention described above, the oxime ether derivatives represented by Formula (3) according to the invention can be produced using relatively versatile substances as starting materials.

The compounds described in Examples shown below are encompassed more specifically within the definitions described below. Such compounds are especially preferred when they are employed as insecticides.

Thus, an oxime ether derivative represented by Formula (3) shown below:

[C. 80]

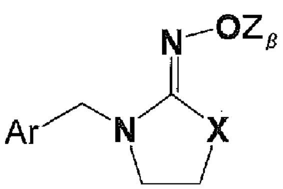

..... Formula (3)

wherein "Ar" represents a 2-chloro-5-pyridyl group;
"X" represents a sulfur atom;
"Zβ" represents "$R_7$", "$COR_8$", "$CONR_9R_{10}$", or "$SO_2R_{11}$";
when "Zβ" represents $R_7$, "$R_7$" represents a hydrogen atom or a C1 to C5 alkyl group, a C6 to C10 aryl group, a (C6 to C10)aryl(C1 to C3)alkyl group; when "Zβ" represents $COR_2$, "$R_2$" represents a C1 to C5 alkyl group or a C6 to C10 aryl group, a (C1 to C4)alkoxy(C1 to C5)alkyl group, a pyridyl group; when "Zβ" represents $CONR_9R_{10}$, each of "$R_9$" and "$R_{10}$" represents a hydrogen atom or a C1 to C5 alkyl group, a C6 to C10 aryl group; and,
when "Zβ" represents $SO_2R_{11}$, "$R_{11}$" represents a C1 to C5 alkyl group.

Among these compounds, a compound represented by Formula (19) wherein "$Z_\beta$" represents "$R_7$" can be produced by reacting a compound represented by Formula (4) shown below with a compound represented by Formula (18) shown below:

[C. 81]

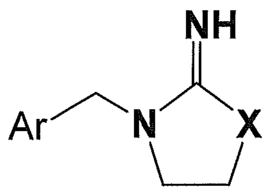

····· Formula (4)

[C. 82]

Formula (18)

$NH_2OR_7$ ·····式(18)

[C. 83]

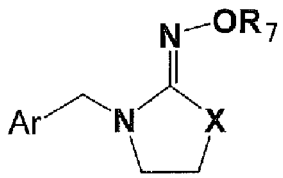

····· Formula (19)

wherein "Ar" represents a 2-chloro-5-pyridyl group;
"X" represents a sulfur atom; and,
"$R_7$" represents a hydrogen atom or a C1 to C5 alkyl group, a C6 to C10 aryl group, a (C6 to C10)aryl(C1 to C3) alkyl group.

Furthermore, a compound represented by Formula (23) wherein "$Z_\beta$" represents "$COR_9$" can be produced by reacting a compound represented by Formula (20) shown below with a compound represented by Formula (21) shown below or a compound represented by Formula (22) shown below:

[C. 84]

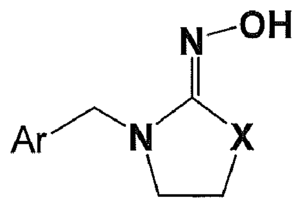

..... Formula (20)

[C. 85] $\mathbf{R_8COA}$ Formula (21)

[C. 86] $\mathbf{R_9CO_2H}$ Formula (22)

[C. 87]

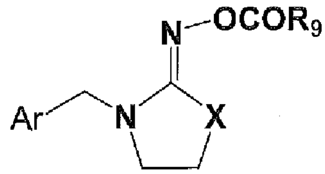

..... Formula (23)

wherein "Ar" represents a 2-chloro-5-pyridyl group;
"X" represents a sulfur atom;
"A" represents a halogen atom such as chlorine, bromine, and iodine; and, "$R_8$" represents a C1 to C5 alkyl group or a C6 to C10 aryl group, a pyridyl group.

Furthermore, a compound represented by Formula (26) wherein "$Z_\beta$" represents "$CONR_9R_{10}$" can be produced by reacting a compound represented by Formula (20) shown below with a compound represented by Formula (24) shown below or a compound represented by Formula (25) shown below:

[C. 88]

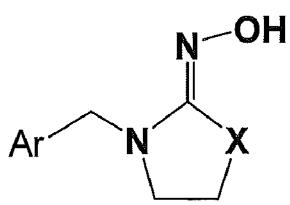

..... Formula (20)

[C. 89] $\mathbf{R_9NCO}$ Formula (24)

[C. 90] $\mathbf{R_9R_{10}NCOB}$ Formula (25)

[C. 91]

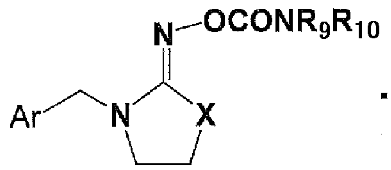

..... Formula (26)

wherein "Ar" represents a 2-chloro-5-pyridyl group;
"X" represents a sulfur atom;
"B" represents a halogen atom such as chlorine, bromine, and iodine; each of "$R_9$" and "$R_{10}$" represents a hydrogen atom or a C1 to C5 alkyl group, a C6 to C10 aryl group; and, provided that in Formula (24) "$R_9$" is other than a hydrogen atom.

Finally, a compound represented by Formula (28) wherein "Ya" represents "$SO_2R_{11}$" can be produced by reacting a compound represented by Formula (20) shown below with a compound represented by Formula (27) shown below:

[C. 92]

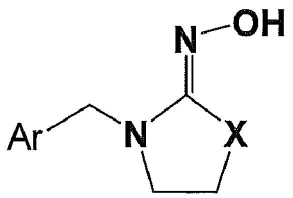

····· Formula (20)

[C. 93] **$R_{11}SO_2D$** Formula (27)

[C. 94]

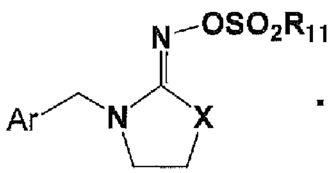

····· Formula (28)

wherein "Ar" represents a 2-chloro-5-pyridyl group;
"X" represents a sulfur atom;
"D" represents a halogen atom such as chlorine, bromine, and iodine; and,
"$R_{11}$" represents a C1 to C5 alkyl group.

3. Insecticides

An imino derivative according to the invention is employed preferably as an insecticide. The followings are the description of agro-horticultural insecticides containing as active ingredients novel imino derivatives represented by Formula (A).

Agricultural pests against which an inventive insecticide can exert its controlling effect are listed below. Lepidoptera pests may for example be oriental corn borer (Ostrinia furnacalis), Oriental armyworm moth (Pseudaletia separata), pink borer (Sesamia inferens), Scarce Bordered Straw (Heliothis sp.), turnip moth (Agrotis segetum), apple leafminer (Phyllonorycter ringoniella), diamondback moth (Plutella xylostella), rice leafroller (Cnaphalocrocis medinalis), yellow stemborer (Scirpophaga incertulas), beet armyworm (Spodoptera exigua), rice stem borer (Chilo suppressalis), common cutworm (Spodoptera litura), soybean pod borer (Leguminivora glycinivorella), peach fruit moth (Carposina niponensis), common cabbage worm (Piers rapae crucivora), summer fruit tortrix (Adoxophyes orana fasciata), and cabbage armyworm (Mamestra brassicae), Hemiptera pests may for example be greenhouse whitefly (Trialeurodes vaporariorum), sweetpotato whitefly (Bemisia tabaci), green rice leafh (Nephotettix cincticeps), brown rice planthopper (Nilaparvata lugens), turnip aphid (Lipaphis erysimi), citrus psyllid (Diaphorina citri), cottony citrus scale (Pulvinaria aurantii), green peach aphid (Myzus persicae), arrowhead scale (Unaspis yanonensis), cotton aphid (Aphis gossypii), tea green leafhopper (Empoasca onukii), whitespotted bug (Eysarcoris ventralis), southern green stink bug (Nezara viridula), Kirkaldy (Triginitylus caelestialium), comstock mealybug (Pseudococcus comstocki), citrus mealybug (Planococcus citri), white peach scale (Pseudaulacaspis pentagona), and california red scale (Aonidiella aurantii). Coleoptera pests may for example be bean beetle (Callosobruchus chinensis),

rice leaf beetle (Oulema oryzae), rice water weevil (Lissorhoptrus oryzophilus), cucurbit leaf beetle (Aulacophora femoralis), striped flea beetle (Phyllotreta striolata), Maize weevil (Sitophilus zeamais), cigarette beetle (Lasioderma serricorne), twenty-eight-spotted ladybird (Epilachna vigintiotopunctata), soybean beetle (Anomala rufocuprea), Brown powder-post beetle (Lyctus brunneus), and Japanese beetle (Popillia japonica), Thysanoptera pests may for example be onion thrips (Thripstabaci), melon thrips (Thrips palmi), and rice thrips,paddy thrips (Stenchaetothrips biformis), Diptera pests may for example be Common house mosquito (Culexpipiens), House Fly(Musca domestica), rice leaf miner (Agromyza oryzae), Melon fly (Dacus(Zeugodacus) cucurbitae), soybean pod gall midge (Asphondylia sp.), onion maggot (Delia antiqua), seed corn maggot (Delia platura), and oriental fruit fly (Dacus(Bactrocera) dorsalis), Dyctyoptera pests may for example be German cockroach(Batella germanica), Orental termite (Coptotermes formosanus), and Japanese subterranean termite (Reticulitermes speratus), Orthoptera pests may for example be Migratory locust (Locusta migratoria), Acari pests may for example be two-spotted spider mite (Tetranychus urticae), kanzawa spider mite (Tetranychus kanzawai), and citrus red mite (Panonychus citri), plant-parasitizing nematodes may for example be southern root-knot nematode (Meloidogyne incognita), soybean cyst nematode (Heterodera glycines), and pine wood nematode (Bursaphelenchus xylophilus), ticks parasiting animals may for example be soft tick (Ornithodoros), cattle tick (Boophilus microplus), bush tick (Haemaphysalis longicornis), brown dog tick (Rhipicephalus sanguineus), hard tick (Haemaphysalis flava), itch mite (Sarcoptidae), red mite (Dermanyssus gallinae), northern fowl mite (Ornithonyssus sylviarum), tropical rat mite (Ornithonyssus bacoti), trombiculid mite (Trombiculidae), fleas may for example be cat flea (Ctenocephalides felis)and dog flea (Ctenocephalides canis), lice may for example be poultry lice (Menopon), body louse (Pediculus humanus), and pubic lice (Phthirus pubis), flies may for example be Blow fly (Lucilia), mosquitoes may for example be asian tiger (Aedes albopictus) and common house mosquito (Culex popiens pallens), robber flies, black flies, sand flies, flukes, thorny-headed worms, tapeworms, nematodes, protozoans, sporozoas, coccidium and the like, and preferred examples are Lepidoptera pests, Hemiptera pests, Thysanoptera pests, animal-parasitizing fleas, animal-parasitizing ticks, with animal-parasitizing ticks being more preferred.

Methods for using the insecticides according to the invention when the insecticides denote agro-horticultural insecticides are not limited particularly, and it may be used as it is or as being diluted to a desired concentration with a diluent such as water. It is also possible to use several types of imino derivatives according to the invention in combination, and a mixture with other pesticides may also be employed as long as the effects of the insecticides are not affected adversely. Other pesticides which may be employed in a mixture are not limited particularly, and may for example be other bactericides, insecticides, acaricides, herbicides, plant growth regulators, and fertilizers.

When using an inventive insecticide as being diluted when the insecticides denote agro-horticultural insecticides, the concentration of the novel imino derivative is preferably within the range of 0.001 to 1.0%. The treatment rate of the inventive insecticide is adjusted in such a manner that 1 ha of an agro-horticultural site such as a field, a paddy field, an orchard, and a greenhouse is treated preferably with 20 to 5000 g, more preferably with 50 to 1000 g of a novel imino derivative. Such concentration and treatment rate are not limited to those specified above, and may appropriately be increased or decreased depending on the dosage form, the timing of use, the method of use, the site of use, the crops to be treated and the like.

When an insecticide according to the invention denotes an agro-horticultural insecticide, then an insecticide according to the invention and a mixture thereof with other pest controlling agents can be applied to genetically recombined crops and the like.

The insecticide according to the invention is used as a formulation in which a novel imino derivative and a carrier are mixed. If necessary, various formulation auxiliaries, such as a surfactant, a spreading agent, a depositing agent, a thickening agent, and a stabilizer, may further be incorporated to formulate a dosage form such as an emulsion, a liquid formulation, a suspension, a wettable powder, a powder, a flowable formulation, a granule, an oil, and an earosol.

In the insecticide according to the invention, when a novel imino derivative is mixed with a carrier, the amount of the carrier is usually within the range of 0.1 to 80% by weight. The carriers for the formulations mentioned above include microparticulate or granulate solid carriers such as kaolin, attapulgite, bentonite, acid clay, pyrophyllite, talc, kieselguhr, calcite, ground walnut powder, urea, ammonium sulfate, and synthetic hydrated silicon oxide, aromatic hydrocarbons such as xylene and naphthalene, alcohols such as isopropanol, ethylene glycol, and cellosolve, ketones such as acetone, cyclohexanone, and isophorone, vegetable oils such as soybean oil and cottonseed oil, liquid carriers such as dimethyl sulfoxide, acetonitrile, and water.

The surfactant employed for emulsification, dispersion, and spreading may for example be an anionic surfactant such as an alkyl sulfate salt, an alkyl(aryl)sulfonate, a dialkylsulfosuccinate, a polyoxiethylene alkylaryl ether phosphate salt, a naphthalenesulfonic acid formaldehyde condensate, and a polycarboxylic acid-type polymer, and a nonionic surfactant such as a polyoxyethylene alkyl ether, a polyoxyethylene alkylaryl ether, a polyoxyethylene polyoxypropylene block copolymer, and a sorbitan fatty acid ester. The formulation auxiliaries may for example be a lignin sulfonate, alginate, a polyvinylalcohol, gum arabic, CMC (carboxymethyl cellulose), PAP (acidic isopropyl phosphate), and xanthane gum.

When an insecticide according to the invention is an animal-parasitizing pest controlling agent, it may preferably

be a liquid formulation, an emulsion, a liquefied drop, a spray, a foam, a tablet, a guranular formulation, a fine particle, a powder, a capsule, a tablet, a chewable, an injection formulation, a suppository, a cream, a shampoo, a rinse, a resin formulation, a fumigant, a bait and the like, with a liquid formulation and a liquefied drop being particularly preferred.

The liquid formulation may further contain ordinary formulation auxiliary agents such as emulsifiers, dispersants, spreading agents, humectants, suspending agents, preservatives, and propellants, as well as ordinary coatings. A surfactant for emulsification, dispersion, and spreading include soaps, polyoxyalkylenealkyl(aryl) ethers, polyoxyethylenealkylallyl ethers, polyoxyethylene fatty acid esters, polyhydric alcohols, alkylarylsulfonates and the like. The dispersants may for example be casein, gelatin, polysaccharides, lignin derivatives, saccharides, and synthetic water-soluble polymers. The spreading agents and the humectants may for example be glycerin and polyethylene glycol. The suspending agents may for example be casein gelatin, hydroxypropyl cellulose, and gum arabic, while the stabilizers may for example be phenolic antioxidants (BHT, BHA and the like), amine-based antioxidants (diphenylamine and the like), and organic sulfur-based antioxidants. The preservatives may for example be methyl paraoxybenzoate, ethyl paraoxybenzoate, propyl paraoxybenzoate, and butyl paraoxybenzoate. The abovementioned carriers, surfactants, dispersants, and auxiliary agents may be employed alone or in combination as necessary. It is also possible to add fragrances and synergistic agents. The amount of the active ingredient in the pest controlling agent of the invention, when employed in a liquid formulation, is usually appropriately 1 to 75% by weight.

Carriers employed for formulating creams may for example be non-volatile hydrocarbons (liquid paraffin and the like), lanolin hydrogenated oils, higher fatty acids, fatty acid esters, animal oils, silicone oils, and water, and those which may further be added, alone or in combination as necessary, include emulsifiers, humectants, antioxidants, fragrances, borax, and ultraviolet absorbing agents. The emulsifiers may for example be fatty sorbitans, polyoxyethylene alkyl ethers, and fatty polyoxyethylenes. The amount of the active ingredient in the pest controlling agent of the invention, when employed in a cream, is usually appropriately 0.5 to 70% by weight.

In the cases of a capsule, pill, or tablet, the active ingredient in a composition of the invention is divided into aliquots appropriately, each of which is mixed with a diluted solution or a carrier such as a starch, lactose, and talc, further together with a diluted solution or a disintegrant and/or a binder such as magnesium stearate, and then may be compacted into tablets as necessary.

A carrier for formulating an injection formulation should be prepared as a sterilized solution, which may contain other substances, for example, a salt or glucose in an amount sufficient to achieve the isotonicity of the solution in relation with blood. Carriers which can be employed may for example be esters such as glycerides, benzyl benzoate, isopropyl myristate and propylene glycol fatty acid derivatives and organic solvents such as N-methylpyrrolidone and glycerol formal. The amount of the active ingredient in the pest controlling agent of the invention, when employed in an injection formulation, is appropriately 0.01 to 10% by weight.

A carrier for formulating a resin formulation may for example be a vinyl chloride-based polymer and a polyurethane. Such a base may be supplemented if necessary with a plasticizer such as phthalates, adipates, and stearates. After an inventive active ingredient is kneaded with the base, molding may be conducted by injection molding, extrusion, press molding and the like. As appropriate, an animal ear tag or collar for repelling pests may be produced via steps such as moldings and cuttings.

A carrier for a bait may for example be a feed or attractant, including meals such as flour and ground maize, starches such as corn starch and potato starch, saccharides such as granulated sugar, maltose, and honey, glycerin, edible flavors such as onion flavor and milk flavor, animal-derived powders such as pupal meal and fish meal, and various pheromones. The amount of the active ingredient in the pest controlling agent of the invention, when employed in a bait, is usually appropriately 0.0001 to 90% by weight.

When an insecticide according to the invention is an animal-parasitizing pest controlling agent, it can serve to control such a pest by being administered into the body of a subject animal orally or via injection, by being administered into the entire or a part of the body surface of a subject animal, or by being coated onto a site of entry, parasitizing, or movement of a pest.

When an insecticide according to the invention is an animal-parasitizing pest controlling agent, it may be employed as it is, and, in some cases, it may be diluted with water, a liquid carrier, a commercially available shampoo, rinse, feed, cage beddings and the like, thereby accomplishing application.

The insecticide capable of being mixed with an insecticide according to the invention may for example be an organic phosphorus-based insecticide (fenitrothion, malathion, acephate, diazinon and the like), a carbamate-based insecticide (benfuracarb, methomyl, carbosulfan and the like), a pyrethroid-based insecticide (allethrin, permethrin, fenvalerate, etofenprox, silafluofen and the like), a nereistoxin-based insecticide (cartap, thiocyclam and the like), a neonicotinoide-based insecticide (imidacloprid, acetamiprid, thiamethoxam, dinotefuran and the like), an IGR agent (diflubenzuron, cyromazine and the like), fipronil, emamectin benzoate, pyridalyl, ethiprole, pymetrozine, flonicamid , chlorfenapyr, pyriproxyfen, indoxacarb, spinosad, avermectin, spinetoram, pyrifluquinazon, chlorantraniliprole, cyantraniliprole, spirotetramat, lepimectin, metaflumizone, pyrafluprole, pyriprole, hydramethylnon, triazamate, sulfoxaflor, organic metal-based compounds, dinitro compounds, organic sulfur compounds, urea-based compounds, triazine-based compounds, hydrazine-based compounds, propylene glycol fatty acid monoester, and a BT agent, a bacteriocide capable of being mixed may for example be a copper bactericide (inorganic copper, organic copper, nonylphenylether copper sulfonate and the like), an inorganic sulfur agent, an organic sulfur bactericide (maneb, manzeb, amobam, propineb, thiuram and the like), an organic phosphorus-based bactericide (fosethyl, tolclofos-methyl and the like), a melanin biosynthesis inhibitor(fthalide, tricyclazole, pyroquilon, carpropamid, diclocymet, fenoxanil), a benzimidazol-based bactericide (thiophanate-methyl, benomyl, diethofencarb, and the like), a dicarboxyimide agent(iprodione, procymidone and the like), an acid amid-based bactericide (mepronil, flutolanil, boscalid, furametpyr, thifluzamide, metalaxyl- and the like), a sterol biosynthesis inhibitor(triflumizole, tebuconazole, ipconazole, metconazole, epoxiconazole and the like), a strobilurin-based bactericide (azoxystrobin, kresoxim-methyl, trifloxystrobin and the like), an anilinopyrimidine-based bactericide (mepanipyrim, cyprodinil and the like), an antibacterial agent(oxolinic acid, tecloftalam and the like), an antibiotic bactericide (kasugamycin, polyoxins, streptomycin and the like), probenazole, ferimzone, TPN, fludioxonil, iminoctadine acetate, cyazofamid, cyflufenamid, morpholine-based bactericides (fenpropimorph, dimethomorph and the like), organic tin-based bactericides (triphenyltin hydroxide, triphenyltin acetate and the like), as well as fenpiclonil , fluazinam, cymoxanil, triforine, pyrifenox, fenarimol, fenpropidin, pencycuron, tebufloquin, iprovalicarb, and benthiavalicarb-isopropyl, an acaricide capable of being mixed may for example be bifenazate, milbemectin, etoxazole, cyenopyrafen, spirodiclofen, and spiromesifen a herbicide capable of being mixed may for example be a phenoxy acid-based herbicide (2,4-d, clomeprop, and fluazifop), a carbamate-based herbicide (benthiocarb, molinate, pyributicarb and the like), an acid amide-based herbicide (pretilachlor, diflufenican, mefenacet, cafenstrole, asulam and the like), an urea-based herbicide (daimuron, isouron and the like), a sulfonyl urea-based herbicide (imazosulfuron, thifensulfuron-methyl, nicosulfuron, halosulfuron methyl and the like), a triazine-based herbicide (atrazine, simetryn, simazine, triaziflam and the like), a diazine-based herbicide (bentazone, bromacil and the like), a diazole-based herbicide (pyrazolate, oxadiazon and the like), a bipyridylium-based herbicide (paraquat and the like), a dinitroaniline-based herbicide (trifluralin, pendimethalin, oryzalin and the like), an aromatic carboxylic acid-based herbicide (fentrazamide, imazapyr and the like), a pyrimidyloxy benzoate-based herbicide (bispyribac sodium and the like), a fatty acid-based herbicide (tetrapion and the like), an amino acid-based herbicide (glyphosate, glufosinate and the like), a nitrile-based herbicide (chlorthiamid and the like), a cyclogexadione-based herbicide (sethoxydim, clethodim and the like), a phenylphthalimide-based herbicide (chlorphthalim and the like), butamifos, pentoxazone, and benzobicyclon, a plant growth regulator capable of being mixed may for example be uniconazole p, daminozide, paraffin, wax, and benzylaminopurine. The agents capable of being mixed or presented as mixture formulations are not limited to those listed above.

An imino derivative represented by Formula (1), a sulfonylimino derivative represented by Formula (2) and an oxime ether derivative represented by Formula (3), according to the invention, are employed preferably as insecticides. Agro-horticultural insecticides containing these as active ingredients are described below.

Agricultural pests against which an inventive insecticide can exert its controlling effect are listed below. Lepidoptera pests may for example be oriental corn borer (Ostrinia furnacalis), Oriental armyworm moth (Pseudaletia separata), pink borer (Sesamia inferens), Scarce Bordered Straw (Heliothis sp.), turnip moth (Agrotis segetum), apple leafminer (Phyllonorycter ringoniella), diamondback moth (Plutella xylostella), rice leafroller (Cnaphalocrocis medinalis), yellow stemborer (Scirpophaga incertulas), beet armyworm (Spodoptera exigua), rice stem borer (Chilo suppressalis), common cutworm (Spodoptera litura), soybean pod borer (Leguminivora glycinivorella), peach fruit moth (Carposina niponensis), common cabbage worm (Piers rapae crucivora), summer fruit tortrix (Adoxophyes orana fasciata), and cabbage armyworm (Mamestra brassicae), Hemiptera pests may for example be greenhouse whitefly (Trialeurodes vaporariorum), sweetpotato whitefly (Bemisia tabaci), green rice leafh (Nephotettix cincticeps), brown rice planthopper (Nilaparvata lugens), turnip aphid (Lipaphis erysimi), citrus psyllid (Diaphorina citri), cottony citrus scale (Pulvinaria aurantii), green peach aphid (Myzus persicae), arrowhead scale (Unaspis yanonensis), and cotton aphid (Aphis gossypii). Coleoptera pests may for example be bean beetle (Callosobruchus chinensis), rice leaf beetle (Oulema oryzae), rice water weevil (Lissorhoptrus oryzophilus), cucurbit leaf beetle (Aulacophora femoralis), striped flea beetle (Phyllotreta striolata), Maize weevil (Sitophilus zeamais), cigarette beetle (Lasioderma serricorne), twenty-eight-spotted ladybird (Epilachna vigintiotopunctata), soybean beetle (Anomala rufocuprea), Brown powder-post beetle (Lyctus brunneus), and Japanese beetle (Popillia japonica), Thysanoptera pests may for example be onion thrips (Thripstabaci), melon thrips (Thrips palmi), and rice thrips,paddy thrips (Stenchaetothrips biformis), Diptera pests may for example be Common house mosquito (Culexpipiens), House Fly(Musca domestica), rice leaf miner (Agromyza oryzae), Melon fly (Dacus (Zeugodacus) cucurbitae), soybean pod gall midge (Asphondylia sp.), onion maggot (Delia antiqua), seed corn maggot (Delia platura), and oriental fruit fly (Dacus(Bactrocera) dorsalis), Dyctyoptera pests may for example be German cockroach(Batella germanica), Orental termite (Coptotermes formosanus), and Japanese subterranean termite (Reticulitermes speratus), Orthoptera pests may for example be Migratory locust (Locusta migratoria), Acari pests may for example be two-spotted spider mite (Tetranychus urticae), kanzawa spider mite (Tetranychus kanzawai), and citrus red mite (Panonychus citri), ticks parasiting animals may for example be soft tick (Ornithodoros), fleas may for example be cat flea (Ctenocephalides felis)and dog flea (Ctenocephalides canis), lice may for example be poultry lice (Menopon), flukes, tapeworms, nematodes, and Coccidia may also be exemplified.

Methods for using the insecticides according to the invention are not limited particularly, and it may be used as it is or as being diluted to a desired concentration with a diluent such as water. It is also possible to use several types of imino derivatives according to the invention in combination, and a mixture with other pesticides may also be employed as long as the effects of the insecticides are not affected adversely. Other pesticides which may be employed in a mixture are not limited particularly, and may for example be other bactericides, insecticides, acaricides, herbicides, plant growth regulators, and fertilizers.

When using an inventive insecticide as being diluted, the concentration of the novel imino derivative is preferably within the range of 0.001 to 1.0%. The treatment rate of the inventive insecticide is adjusted in such a manner that 1 ha of an agro-horticultural site such as a field, a paddy field, an orchard, and a greenhouse is treated preferably with 20 to 5000 g, more preferably with 50 to 1000 g of a novel imino derivative. Such concentration and treatment rate are not limited to those specified above, and may appropriately be increased or decreased depending on the dosage form, the timing of use, the method of use, the site of use, the crops to be treated and the like.

The insecticide according to the invention is used as a formulation in which a novel imino derivative and a carrier are mixed. If necessary, various formulation auxiliaries, such as a surfactant, a spreading agent, a depositing agent, a thickening agent, and a stabilizer, may further be incorporated to formulate a dosage form such as a wettable powder, a granule, and a flowable formulation.

In the insecticide according to the invention, when a novel imino derivative is mixed with a carrier, the amount of the carrier is usually within the range of 0.1 to 80% by weight. The carriers for the formulations mentioned above include microparticulate or granulate solid carriers such as kaolin, attapulgite, bentonite, acid clay, pyrophyllite, talc, kieselguhr, calcite, ground walnut powder, urea, ammonium sulfate, and synthetic hydrated silicon oxide, aromatic hydrocarbons such as xylene and naphthalene, alcohols such as isopropanol, ethylene glycol, and cellosolve, ketones such as acetone, cyclohexanone, and isophorone, vegetable oils such as soybean oil and cottonseed oil, liquid carriers such as dimethyl sulfoxide, acetonitrile, and water.

The surfactant employed for emulsification, dispersion, and spreading may for example be an anionic surfactant such as an alkyl sulfate salt, an alkyl(aryl)sulfonate, a dialkylsulfosuccinate, a polyoxiethylene alkylaryl ether phosphate salt, a naphthalenesulfonic acid formaldehyde condensate, and a polycarboxylic acid-type polymer, and a nonionic surfactant such as a polyoxyethylene alkyl ether, a polyoxyethylene alkylaryl ether, a polyoxyethylene polyoxypropylene block copolymer, and a sorbitan fatty acid ester. The formulation auxiliaries may for example be a lignin sulfonate, alginate, a polyvinylalcohol, gum arabic, CMC (carboxymethyl cellulose), PAP (acidic isopropyl phosphate), and xanthane gum.

The insecticide capable of being mixed with an insecticide according to the invention may for example be an organic phosphorus-based insecticide (fenitrothion, malathion, acephate, diazinon and the like), a carbamate-based insecticide (benfuracarb, methomyl, carbosulfan and the like), a pyrethroid-based insecticide (allethrin, permethrin, fenvalerate, etofenprox, silafluofen and the like), a nereistoxin-based insecticide (cartap, thiocyclam and the like), a neo-nicotinoide-based insecticide (imidacloprid, acetamiprid, thiamethoxam, dinotefuran and the like), an IGR agent (diflubenzuron, cyromazine and the like), fipronil, emamectin benzoate, pyridalyl, propylene glycol fatty acid monoester, and a BT agent, a bacteriocide capable of being mixed may for example be a copper bactericide (inorganic copper, organic copper, nonylphenylether copper sulfonate and the like), an inorganic sulfur agent, an organic sulfur bactericide (maneb, manzeb, amobam, propineb, thiuram and the like), an organic phosphorus-based bactericide (fosethyl, tolclofos-methyl and the like), a melanin biosynthesis inhibitor(fthalide, tricyclazole, pyroquilon, carpropamid, diclocymet, fenoxanil), a benzimidazol-based bactericide (thiophanate-methyl, benomyl, diethofencarb, and the like), a dicarboxyimide agent(iprodione, procymidone and the like), an acid amid-based bactericide (mepronil, flutolanil, boscalid, furametpyr, thifluzamide, metalaxyl- and the like), a sterol biosynthesis inhibitor(triflumizole, tebuconazole, ipconazole, metconazole, epoxiconazole and the like), a strobilurin-based bactericide (azoxystrobin, kresoxim-methyl, trifloxystrobin and the like), an anilinopyrimidine-based bactericide (mepanipyrim, cyprodinil and the like), an antibacterial agent(oxolinic acid, tecloftalam and the like), an antibiotic bactericide (kasugamycin, polyoxins, streptomycin and the like), probenazole, ferimzone, TPN, fludioxonil, iminoctadine acetate, cyazofamid, and cyflufenamid, an acaricide capable of being mixed may for example be bifenazate, milbemectin, and etoxazole, a herbicide capable of being mixed_may for example be a phenoxy acid-based herbicide (2,4-d, clomeprop, and fluazifop), a carbamate-based herbicide (benthiocarb, molinate, pyributicarb and the like), an acid amide-based herbicide (pretilachlor, diflufenican, mefenacet, cafenstrole, asulam and the like), an urea-based herbicide (daimuron, isouron and the like), a sulfonyl urea-based herbicide (imazosulfuron, thifensulfuron-methyl, nicosulfuron, halosulfuron methyl and the like), a triazine-based herbicide (atrazine, simetryn, simazine, triaziflam and the like), a diazine-based herbicide (bentazone, bromacil and the like), a diazole-based herbicide (pyrazolate, oxadiazon and the like), a bipyridylium-based herbicide (paraquat and the like), a dinitroaniline-based herbicide (trifluralin, pendimethalin, oryzalin and the like), an aromatic carboxylic acid-based herbicide (fentrazamide, imazapyr and the like), a pyrimidyloxy benzoate-based herbicide (bispyribac sodium and the like), a fatty acid-based herbicide (tetrapion and the like), an amino acid-based herbicide (glyphosate, glufosinate and the like), a nitrile-based herbicide (chlorthiamid and the like), a cyclogexadione-based herbicide (sethoxydim, clethodim and the like), a phenylphthalimide-based herbicide (chlorphthalim and the like), butamifos, pentoxazone, and benzobicyclon, a plant growth regulator capable of being mixed may for example be uniconazole p, daminozide, paraffin, wax, and benzylaminopurine. The agents capable of being mixed or presented as mixture formulations are not limited to those listed above.

[Examples]

Production Examples and Formulation Examples, Experimental Examples of the imino derivatives represented by Formula (A) and the insecticides according to the invention are shown, thereby describing the invention specifically. Unless departing from the subject of the invention, the invention is not limited to the below-indicated Production Examples and the like. The compounds employed in the below-indicated Production Examples and the like may be commercially available products as appropriate. Determination of the physical characteristics of intended compounds in respective Examples was conducted under the conditions indicated in "Table 1" shown below. Unless otherwise indicated, $CDCl_3$ was employed as a solvent.

[Table 1]

| | |
|---|---|
| Melting point | Measured using BUECHI Model B-545 |
| IR (Infrared spectroscopy) | Measured by KBr tablet method using HITACHI, Ltd. Model 270-30 |
| $^1$H-NMR (Proton nuclear magnetic resonance spectroscopy) | Measured by JEOL Ltd. device (400 MHz) using internal standard TMS (tetramethylsilane) and solvent DMSO-$d_6$ (tritylated dimethylsulfoxide) |

[1. Production Examples and Formulation Examples, Experimental Examples of imino derivatives]

The intended compounds obtained in Examples of imino derivatives represented by Formula (1) shown above had the chemical structures and the melting points shown in "Table 2" and "Table 3" and the measured values of IR spectra and NMR spectra shown in "Table 4" and "Table 5". Among those, some Examples are detailed below with regard to Production Examples, Formulation Examples, and Experimental Examples.

[Table 2]

| Compound No | Y (-CSN $R_1$ /$R_2$) | Ar | X | Melting Point (°C) |
|---|---|---|---|---|
| 1 | -CSN H / $CH_3$ | 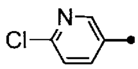 | S | 144-145 |
| 2 | -CSN H / $C_2H_5$ | 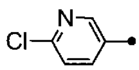 | S | 126-128 |
| 3 | -CSN H / $CH_2CH_2Cl$ | 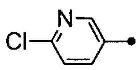 | S | 104-105 |
| 4 | -CSN H / $CH_2CH_2CH_3$ | 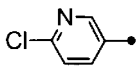 | S | 111-113 |
| 5 | -CSN H / $CH(CH_3)_2$ | 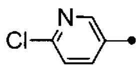 | S | 165-166 |
| 6 | -CSN H / $C(CH_3)_3$ | 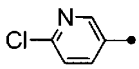 | S | 126-128 |
| 7 | —CSN H / 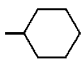 | 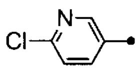 | S | 132-134 |
| 8 | -CSN H / $C_6H_5$ | 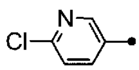 | S | 165-167 |

(continued)

| Compound No | Y (-CSN $R_1$ /$R_2$) | Ar | X | Melting Point (°C) |
|---|---|---|---|---|
| 9 | -CSN H / $CH_2C_6H_5$ | 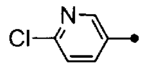 | S | 164-165 |
| 10 | -CSN H / $COCH_3$ | 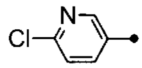 | S | 168-169 |
| 11 | -CSN H / $COC_6H_5$ | 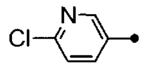 | S | 144-146 |
| 1 2 | -CSN H / $CO_2C_2H_5$ | 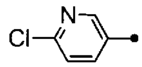 | S | 96 |
| 1 3 | -CSN H / $CH_2CH_2OCH_3$ | 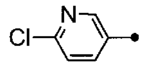 | S | liquid |
| 14 | -CSN H / $CH_2CH_2SCH_3$ | 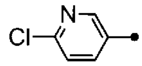 | S | 110-112 |
| 15 | -CSN $CH_3$ /$CH_3$ | 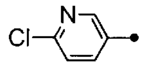 | S | 108-109 |
| 32 | -CSN H / $CH_2C_6H_5$ | 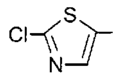 | S | 148-150 |
| 74 | 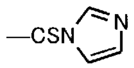 | 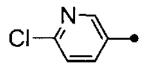 | S | 120-125 |

[Table 3]

| Compound No | Y | Ar | X | Melting Point(°C) |
|---|---|---|---|---|
| 16 | $-CS_2CH_3$ | 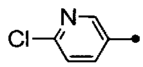 | S | 158-159 |
| 17 | $-CS_2CH_2CH_3$ | 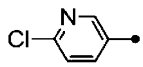 | S | 79-80 |
| 18 | $-CS_2CH_2CH_2CH_3$ | 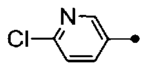 | S | Liquid |
| 19 | $-CS_2C_6H_5$ | 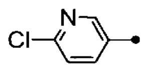 | S | 145-147 |
| 20 | $-CS_2CH_2C_6H_5$ | 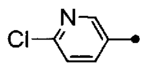 | S | 115-116 |
| 21 | $-CS_2CH_2CH_2OCH_3$ | 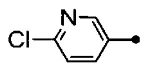 | S | 84-86 |
| 22 | $-CS_2CH_2CO_2CH_3$ | 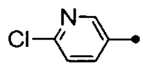 | S | Liquid |
| 23 | $-COSCH_3$ | 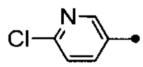 | S | 132-134 |
| 24 | $-COSCH_2CH_2CH_3$ | 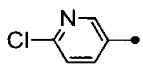 | S | Liquid |
| 25 | $-COSCH(CH_3)_2$ | 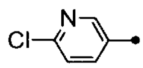 | S | 117 |

(continued)

| Compound No | Y | Ar | X | Melting Point(°C) |
|---|---|---|---|---|
| 26 | $-COSC(CH_3)_3$ | 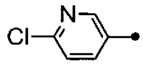 | S | 103-104 |
| 27 | $-CSOC_6H_5$ | 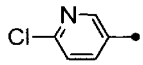 | S | 144-145 |
| 28 | 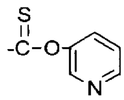 | 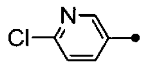 | S | 146-148 |
| 29 | $-CH_2C_6H_5$ | 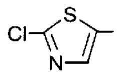 | S | 93-95 |
| 30 | $-CS_2C_2H_5$ | 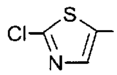 | S | Liquid |
| 31 | $-CS_2CH_2CH_2CH_3$ | 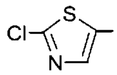 | S | Liquid |
| 75 | $-CSSCH_2CH_2CH_2CH_3$ | 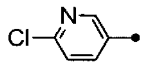 | S | 115-116 |

[Table 4]

| Compoud No | IR (KBr, -, $cm^{-1}$) | NMR ($CDCl_3$, ppm) |
|---|---|---|
| 1 | 1552, 1504, 1444, 1339, 1237 | 3 11 (2H, t), 3.15 (3H, d), 3 53 (2H, t), 4 74 (2H, s), 6.86 (1H, bs), 7 30 (1H, d), 7.59 (1H, dd), 8 30 (1H, d) |
| 2 | 3206, 1550, 1524, 1471,1454 | 1 12 (3H, t), 1.24 (3H, t), 3 11 (2H, t), 3.18 (2H, t), 3 40 (2H, m), 3 55 (2H, t), 3 60-3.66 (2H+2H, overlap), 4 77 (2H, s), 4.83 (2H, s), 7 06 (1H, t), 7 11 (1H, t), 7.30 (1H, d), 7 33 (1H, d), 7.61 (1H+ 1H, overlap), 8.31 (1H, d), 8 33 (1H, d) |
| 3 | 1585, 1541, 1459, 1432, 1306 | 3.12 (2H, t), 3.38 (2H, t), 3 52 (2H, t), 4.21 (2H, t). 4 71 (2H, s), 7.31 (1H, d), 7.69 (1H, dd), 8.31 (1H, d). |
| 4 | 3209, 1549, 1524, 1465, 1455 | 0 88 (3H, dd), 0.97 (3H, dd), 1 52 (2H, m), 1 66 (2H, m), 3.10 (2H, t), 3.16 (2H, t), 3 33 (2H), 3.50 (2H, m), 3.60 (2H+2H, overlap), 4.76 (2H, s), 4.82 (2H, s), 6.73 (1H, bs), 6.79 (1H, bs), 7.31-7.32 (1H+1H, overlap), 7 61 (1H+1H, overlap), 8 30 (1H, d), 8.32 (1H, d) |
| 5 | 3223, 1549, 1524, 1473, 1384, | (6H, d), 1.27 (6H, d), 3 09 (2H, t), 3.15 (2H, t), 3 48 (2H, t), 3.58 (2H, t), 4 17 (1H, septet), 4.54 (1H, septet), 4.76 (2H, s), 4.79 (2H, s), 6.50 (1H, bs), 6 63 (1H, bs), 7 31 (1H, d), 7.32 (1H, d), 7 56 (1H, dd), 7.58 (1H, dd), 8 31 (1H+1H, overlap). |
| 6 | 3335, 1534, 1507, 1457, 1431 1457, 1431 | 1 36 (9H, s), 2.01 (9H, s), 3 06 (2H, t), 3 15 (2H, t), 3.47 (2H, t), 3.59 (2H, t), 4.76 (2H, s), 4 86 (2H, s), 6.79 (1H, bs), 6 88 (1H, bs), 7 31-7.34 (1H+1H, overlap), 7.57 (1H+1H), 8 31 (1H+1H, overlap) |

(continued)

| Compoud No | IR (KBr, -, $cm^{-1}$) | NMR ($CDCl_3$, ppm) |
|---|---|---|
| 7 | 3194, 2927, 1567, 1522, 1460 | 1 12-2.15 (10H+10H, overlap), 3.09 (2H, t), 3.15 (2H, t), 3.48 (2H, t), 3 60 (2H, t), 3 81 (1H, m), 4.24 (1H, m), 4 75 (2H, s), 4 81 (2H, s), 6.59 (1H, d), 6.70 (1H, d), 7.31-7.34 (1H+1H. overlap), 7 57 (1H+1H, overlap), 8.31 (1H, d), 8.32 (1H, d) |
| 8 | 3201, 1542, 1510, 1467, 1453 | 3.18 (2H, overlap), 3.59 (2H, overlap), 4.65 (2H, s), 4.85 (2H, s), 7.09 (1H, bs, overlap), 7.15-7.42 (6H+6H, overlap), 6.70 (1H, d), 731-7.34 (1H+1H, overlap), 7.55-7.75 (1H+1H, overlap), 8 17 (1H, bs), 8.34 (1H, bs) |
| 9 | 3216, 1550, 1528, 1458, 1426 | 3.12 (2H+2H, overlap), 3.15 (2H+2H, overlap), 4.59 (2H, d), 4 66 (2H, s), 4.74 (2H, s), 4.84 (2H, d), 6 89 (1H, bs), 7 06 (1H, bs), 7.15-7 38 (overlap), 7.55 (1H+1H, overlap), 8.19 ( H,d), 8 29 (1H, d) |
| 10 | 1716, 1537, 1464, 1442, 1422 | 2.18 (3H, s), 3.19 (2H, t), 3.71 (2H, t), 4.92 (2H, s), 7.37 (2H, d), 7.85 (1H, bd), 8.44 (1H, bs). 10 59 (1H, bs) |
| 11 | 1700, 1529, 1427, 1408, 1352 | 3.21 (2H, t), 3 67 (2H, t), 4.90 (2H, s), 7 33 (1H, d), 7.43-7.57 (3H, m), 7 83-7.86 (3H, m), 8.37 (1H, d) 9.17 (1H, bs) |
| 12 | 1748, 1541, 1507, 1460, 1424 | 1.27 (3H, t), 3 17 (2H, dd), 3 62 (2H, dd), 4.17 (2H, q), 4.90 (2H, s), 7 31 (1H, d), 7.7 (1H, bd), 8.20 (1H, bs). 8.36 (1H, bs) |
| 13 | 3406, 1557, 1542, 1507, 1458, | 3 11 (2H, t), 3 15 (2H, dd), 3.32 (3H, s), 3.37 (3H, s), 3.51 (2H, t), 3.52-3.65 (2H+(2H+2H), overlap), 3 85 (2H, m), 4 77 (2H, s), 4.80 (2H, s), 7.07 (1H+1H, bs), 7 31-7 33 (1H+1H, overlap), 7.58 (1H+1H, overlap), 8.31 (1H+1H, overlap) |
| 14 | 3217, 1562, 1518, 1445, 1326 | 2 06 (3H, s). 2.14 (3H, s), 2.61 (2H, t), 2.79 (2H, t), 3 11 (2H, dd), 3.16 (2H, dd), 3 51 (2H, t), 3 57 (2H+2H, overlap), 3.86 (2H, dd), 4 77 (2H, s), 4 80 (2H, s), 6 99 (1H, bs), 7.05 (1H, bs), 7.31-7.34 (1H+1H, overlap), 7.58 (1H+1H, overlap), 8.31 (1H+1H, overlap) |
| 15 | 1611, 1581, 1555, 1456, 1430, | 3 00 (3H, s), 3 08 (2H, t), 3.09 (3H, s), 3.51 (2H, t), 4.75 (2H, s), 7 30 (2H, d), 7.63 (1H, d), 8.33 (1H, d) |
| 32 | 1534, 1429, 1359, 1278, 1246 | 3.34 (2H, t), 3.84 (2H, t), 5.00 (2H, s), 6.96 (1H, s), 7.40 (1H, d), 7 71 (1H, dd), 7 85 (1H, s), 8.39 (1H, d), 8.52 (1H, s) |
| 74 | 1216, 1278, 1429, 1534 | 3 34 (2H, t), 3.84 (2H, t), 5 00 (2H, s), 6.96 (1H, s), 7 39 (1H, d), 7.70 (1H, dd), 7.85 (1H, s), 8.39 (1H, s), 8.52 (1H, s) (DMSO-d6) |

[Table 5]

| Compoud No | IR(KBr, -, $cm^{-1}$) | NMR ($CDCl_3$, - ppm) |
|---|---|---|
| 16 | 1523, 1457, 1415, 1390, 1269 | 2.57 (3H, s), 3.17 (2H, dd), 3.59 (2H, dd), 4 84 (2H, s), 7.31 (1H, d), 7.68 (1H, bd), 8.33 (1H, bs) |
| 17 | 1519, 1458, 1415, 1389, 352 | 1.34 (3H, t), 3.17 (2H+2H, overlap), 3.59 (2H, t), 4.85 (2H, s), 7.34 (1H, d), 7.68 (1H, dd), 8.35 (1H, d) |

(continued)

| Compoud No | IR(KBr, -, $cm^{-1}$) | NMR ($CDCl_3$, - ppm) |
|---|---|---|
| 18 | 1523, 1460, 1408, 1289, 1266 | 1.00 (3H, m), 1.72 (2H,m), 3.13 (2H, m), 3.17 (2H, dd), 3.60 (2H, dd), 4.85 (2H, s), 7.34 (1H, d), 7.67 (1H, dd), 8.34 (1H, d) |
| 19 | 1533, 1407, 1389, 1274, 1243 | 3.16 (2H, t), 3.53 (2H, t), 4.44 (2H, s), 7.2-7.4 (5H), 7.5-7.6 (2H), 8.10 (1H, bs) |
| 20 | 1528, 1458, 1403, 1381, 1268 | 3.17 (2H, dd), 3.58 (2H, dd), 4.43 (2H, s), 4 78 (2H, s), 7.22 (1H, m), 7.26-7.36 (5H, overlap), 7.58 (1H, dd), 8 30 (1H, d) |
| 21 | 1588, 1567, 1460, 1392, 1099 | 3.20 (2H, dd), 3.57 (3H, s), 3.38 (2H, m), 3.61-3.64 (4H, overlap), 4.85 (2H, s), 7.33 (1H, d), 7.68 (1H, dd), 8.34 (1H, d) |
| 22 | 1740, 1588, 1488, 1437, 1299 | 3.31 (2H, t), 3.63 (2H, t), 3.76 (2H, s), 4.19 (2H, s), 4.28 2H, s), 7.39 (1H, d), 7 73 (1H, dd), 8 35 (1H, d) |
| 23 | 1613, 1540, 1457, 1396, 1254 | 2.34 (3H, s), 3.18 (2H, dd), 3.58 (2H, dd), 4.78 (2H, s), 7.33 (1H, d), 7.68 (1H, bd), 8 32 (1H, bs) |
| 24 | 1619, 1534, 1462, 1406, 1253 | 1.00 (3H, m), 1 69 (2H, m), 2.86 (2H, m), 3.17 (2H, t), 3.56 (2H, t), 4.77 (2H, s), 7.33 (1H, d), 7.68 (1H, dd), 8.32 (1H, d) |
| 25 | 1608, 1532, 1463, 1397, 1183 | 1.35 (6H, d), 3.16 (2H, t), 3.53-3.58 (2H+1H, overlap), 4.76 (2H, s), 7.33 (1H, d), 7.68 (1H, dd), 8.32 (1H, d) |
| 26 | 2925, 2854, 1615, 1542,1459 | 1.51 (9H, s), 3.16 (2H, t), 3.53 (2H, t), 4.74 (2H, s), 7.33 (1H, d), 7.68 (1H, dd), 8.32 (1H, d) |
| 27 | 1587, 1546, 1463, 1440, 1430 | 3.18 (2H, dd), 3.64 (2H, dd), 4.68 (2H, s), 7.21 (2H, m), 7.20-7.397 68 (4H, overlap), 7.42 (1H, dd), 8.22 (1H, d) |
| 28 | 1656, 1588, 1530, 1458, 1434 | 3.26 (2H, dd), 3.70 (2H, dd), 4.84 (2H, s), 6.15 (1H, t), 6.50 (1H, d), 7.29 (1H, m), 7.37 (1H, d), 7.78 (1H, d), 7.87 (1H, bd), 8.30 (1H, bs) |
| 29 | 1537, 1519, 1460, 1434, 1407 | 3.20 (2H, dd), 3.64 (2H, dd), 4.48 (2H, s), 4.85 (2H, s), 7.25 (1H, m), 7.30 (2H, m), 7 39 (2H, m), 7.45 (1H, s) |
| 30 | 1637, 1617, 1528, 1418, 1047 | 1.37 (3H, t), 3.33 (2H, t), 3.38 (2H, q), 3.62 (2H, t), 4.42 (2H, s), 7.51 (1H, s) |
| 31 | 1637, 1617, 1526. 1419, 1047 | 1.03 (3H, t), 1.75 (2H, tq), 3.33 (2H, t), 3 36 (2H, t), 3.61 (2H, t), 4.42 (2H, s), 7 51 (1H, s) |
| 75 | 1338, 1403, 1528 | 3.17 (2H, t), 3.57 (2H, t), 4.43 (2H, s), 4.78 (2H, s), 7.20-7.36 (6H, m), 7.58 (1H, dd), 8.30 (1H, d) |

<Production Example 1-1>

Synthesis of [3-(6-chloro-3-pyridinylmethyl)]-2-(3H)-thiazolidinylidene]methylthiourea ("Table 2", Compound No. 1)

To a solution of 3-(2-chloro-5-pyridylmethyl)-2-iminoimidazolidine synthesized in accordance with the method described in Journal of Medicinal Chemistry 1999, 42(12), 2227-2234 (227 mg, 1.0 mmol), methyl isothiocyanate (73 mg, 1.0 mmol) in acetonitrile (20 ml), a drop of triethylamine was added and the mixture was heated under reflux for 9 hours. Acetonitrile was distilled away and the treatment of the residue with water resulted in crystallization. The crystal was filtered with suction, and recrystallized from ethyl acetate to obtain the product.
Yield: 240 mg (80%)

<Production Example 1-2>

Synthesis of [3-(6-chloro-3-pyridinylmethyl)]-2-(3H)-thiazolidinylidene]dimethylthiourea ("Table 2", Compound No. 15)

To a suspension of 3-(2-chloro-5-pyridylmethyl)-2-iminoimidazolidine (227 mg, 1.0 mmol), potassium carbonate (165 mg, 1.2 mmol) in acetonitrile (20 ml), a solution of dimethylthiocarbonyl chloride (124 mg, 1 mmol) in acetonitrile

(5 ml) was added dropwise over a period of 15 minutes under cooling with ice. The ice bath was removed, the reaction mixture was stirred for 12 hours, and then potassium iodide (50 mg) was added. The stirring with heating under reflux was continued for further 13 hours. Acetonitrile was distilled away, and the residue was subjected to a column chromatography (column packing: silica gel, eluent: ethyl acetate) to separate a crude product. Purification was accomplished by recrystallization from methanol.
Yield: 126 mg (40%)

<Production Example 1-3>

Synthesis of [3-(6-chloro-3-pyridinylmethyl)]-2-(3H)-thiazolidinylidene]-2-methoxyethylthiourea ("Table 2", Compound No. 13)

3-(2-Chloro-5-pyridylmethyl)-2-iminoimidazolidine (227 mg, 1.0 mmol), bis(1-benzotriazolyl)methanethione (280 mg, 1 mmol), and triethylamine (110 mg, 1.1 mmol) were dissolved in dry dichloromethane (20 ml), and stirred at room temperature for 12 hours. Dichloromethane was distilled away, and the residue was washed with a small amount of cold methanol to obtain a solid. The resultant solid was dried in vacuum to obtain an intermediate (155 mg) which was then dissolved in acetonitrile (20 ml) to form a solution, to which sodium acetate (42 mg, 0.5 mmol) and 2-methoxyethylamine (31 mg, 0.4 mmol) were added, and heating under reflux was conducted for 7 hours. The reaction solution was allowed to cool to room temperature, and the precipitated solid was filtered off. The filtrate was concentrated under reduced pressure, and the residue was subjected to a silica gel column chromatography (eluent: ethyl acetate/hexane (10:1)) to isolate the product.
Yield: 52 mg (38%)

<Production Example 1-4>

Synthesis of 3-(2-chloro-5-pyridinylmethyl)-2-[(methylthio)thiocarbonylimino]-thiazolidine ("Table 3", Compound No. 16)

3-(2-Chloro-5-pyridylmethyl)-2-iminothiaziridine (228 mg, 1.0 mmol), dimethyl trithiocarbonate (150 mg, 1.1 mmol), and triethylamine (112 mg, 1.1 mmol) were dissolved in ethanol (20 ml), and heated under reflux for 12 hours. The solvent was distilled away, and the residue was recrystallized from methanol to obtain the intended product as a yellow crystal.
Yield: 69 mg (85%)

<Production Example 1-5>

Synthesis of 3-(2-chloro-5-pyridinylmethyl)-2-[(phenylthio)thiocarbonylimino]-thiazolidine ("Table 3", Compound No. 19)

3-(2-Chloro-5-pyridylmethyl)-2-iminothiaziridine (1) (228 mg, 1.0 mmol) dissolved in tetrahydrofuran (20 ml) was cooled with ice, and potassium t-butylate (112 mg, 1.0 mmol) was added in portions, and stirring was conducted for 2 hours. To this solution, a solution of phenyl chlorothioformate (189 mg, 1.0 mmol) dissolved in THF (1 ml) was added dropwise under cooling with ice, and stirring was continued for 12 hours. The solvent was distilled away, and the residue was combined with a solution mixture of water and ethyl acetate and stirred. The organic layer was separated, and the organic layer was washed with an aqueous solution of potassium carbonate, water, 1% hydrochloric acid, and finally with water in this order, and then dried. The organic layer was taken out, the solvent was distilled away, and the residue was subjected to a silica gel column chromatography (eluent: ethyl acetate/hexane (1:1)) to separate a crude product, which was washed with ether for purification.
Yield: 260 mg (70%)

<Production Example 1-6>

Synthesis of 3-(2-chloro-5-pyridinylmethyl)-2-[(ethylthio)thiocarbonylimino]-thiazolidine ("Table 3", Compound No. 17)

A solution of ethyl imidazol-1-carbodithioate (303 mg, 1.8 mmol) in acetonitrile (3 ml) was added under cooling with ice dropwise to a solution of 3-(2-chloro-5-pyridylmethyl)-2-iminoimidazolidine (1) (402 mg, 1.8 mmol) and triethylamine (213 mg, 2.1 mmol) in acetonitrile (10 ml). Thereafter, the reaction solution was refluxed at room temperature for 5 hours and then with heating for 8 hours. The solvent was distilled away, and the residue was combined with a mixture solution of water and ethyl acetate, and stirred. The organic layer was separated, and the organic layer was washed with an aqueous solution of potassium carbonate, water, 1% hydrochloric acid, and finally with water in this order, and

then dried. The organic layer was taken out, the solvent was distilled away, and the residue was subjected to a silica gel column chromatography (eluent: ethyl acetate/hexane (1:1)) to separate a crude product, which was washed with a small amount of ether for purification. Yield: 100 mg (30%)

<Formulation Example 1-1>

1. Powder formulation

3 Parts by weight of the imino derivative of Compound No. 1 (Table 2), 40 parts by weight of a clay, 57 parts by weight of a talc were ground respectively and mixed to obtain a powder formulation.

<Formulation Example 1-2>

2. Wettable powder

50 Parts by weight of the imino derivative of Compound No. 4 (Table 2), 5 parts by weight of lignin sulfonate, 3 parts by weight of an alkyl sulfonate, 42 parts by weight of a kieselguhr were ground respectively and mixed to obtain a wettable powder.

<Formulation Example 1-3>

3. Granule formulation

5 Parts by weight of the imino derivative of Compound No. 9 (Table 2), 43 parts by weight of a bentonite, 45 parts by weight of a clay, 7 parts by weight of lignin sulfonate were mixed uniformly, combined with water and kneaded, and then subjected to an extruding granulator to fabricate into granules, which was dried to obtain a granule formulation.

<Formulation Example 1-4>

4. Emulsifiable concentrate

20 Parts by weight of the imino derivative of Compound No. 14 (Table 2), 10 parts by weight of a polyoxyethylene alkylallyl ether, 3 parts by weight of a polyoxyethylene sorbitan monolaurate, 67 parts by weight of a xylene were mixed uniformly while dissolving to obtain an emulsifiable concentrate.

<Formulation Example 1-5>

5. Liquefied drop

20 Parts by weight of the imino derivative of Compound No.27 (Table 3), 66.7 parts by weight of diethylene glycol monoethyl ether, and 13.3 parts by weight of ethanol were mixed uniformly to yield a liquefied drop.

<Experimental Example 1-1>

1. Green peach aphid controlling effect

Onto a two leaf-stage Chinese cabbage seedling, 30 adult insects of green peach aphid were allowed to inhabit, and allowed to stand for several days to accomplish settlement. Thereafter, the overground part was cut off, was immersed in a 100 μg/ml treatment solution. This was placed on a moistened filter paper placed on the bottom of a petri dish of 9 cm in diameter, and the petri dish was covered with the stopper, and allowed to stand in a room whose temperature was constant at 25°C. After 120 hours, the numbers of viable and dead insects were counted.
As a result, Compound Nos. 2, 4, 5, 6, 7, 9, 14, 17, 18, 20, 23, 24, 25, 26, at an active ingredient concentration of 100 μg/ml, exhibited an effect as excellent as a lethality of 50% or higher.

<Experimental Example 1-2>

2. Onion thrips controlling effect

A two leaf-stage Chinese cabbage seedling was immersed in a treatment solution at 100 μg/ml, placed on a moistened filter paper placed on the bottom of a petri dish of 9 cm in diameter. This was allowed to be inhabited with onion thrips larvae, and then the petri dish was covered with the stopper, and then allowed to stand in a room whose temperature was constant at 25°C. After 120 hours, the numbers of viable and dead insects were counted.
As a result, Compound Nos. 4, 5, 13, 17, 18, 20, 24, 25, at an active ingredient concentration of 100 μg/ml, exhibited an effect as excellent as a lethality of 50% or higher.

<Experimental Example 1-3>

3. Diamondback moth controlling effect

A two leaf-stage Chinese cabbage seedling was immersed in a treatment solution at 500 μg/ml, placed on a moistened filter paper placed on the bottom of a petri dish of 9 cm in diameter. This was allowed to be inhabited with diamondback moth 2nd instar larvae, and then the petri dish was covered with the stopper, and then allowed to stand in a room whose temperature was constant at 25°C. After 120 hours, the numbers of viable and dead insects were counted.
As a result, Compound Nos. 2, 4, 6, 7, 9, 14, 17, 18, 20, 24, 25, 26 at an active ingredient concentration of 100 μg/ml, exhibited an effect as excellent as a lethality of 50% or higher.

<Experimental Example 1-4>

4. Bush tick controlling effect

30 μL of an acetone solution of an imino derivative according to the invention prepared at 200 μg/ml or 1 0μg/ml was placed in a 4 mL glass vial. This was placed on a shaker, and a dry film of the compound was formed on the inner wall of the vial. After the vial was dried for 24 hours or longer, it was allowed to be inhabited by 10 juvenile bush ticks and closed with a stopper. The vial was allowed to stand in an entirely dark thermostat chamber at 25°C and 85% humidity. After 1 day of inhabitation, the % dead insect was calculated according to the following equation. The experiment was conducted in duplicate.

$$\%\ \text{Dead insect} = \{\text{Number of dead insects} / (\text{Number of surviving insects} + \text{Number of dead insects})\} \times 100$$

As a result, in the test at 200 μg/ml, Compound Nos. 11, 12, 15, 20, 24, 26, 27, and 28 exhibited an excellent effect as reflected by a % dead insect of 60% or higher.
Also, in the test at 10 μg/ml, Compound Nos. 12, 26, 27, and 28 exhibited an excellent effect as reflected by a % dead insect of 60% or higher.

[2. Production Examples and Formulation Examples, Experimental

The intended compounds obtained in Examples of the sulfonylimino derivatives represented by Formula (2) shown above had the chemical structures and the melting points indicated in "Table 6" and "Table 7"and the measured values of the IR spectra and the NMR spectra indicated in "Table 8" and "Table 9". Among these, some of Examples are described in detail referring to their respective Production Examples, Formulation Examples, and Experimental Examples.
[Table 6]

| Compound No. | $Z_\alpha$ | Ar | X | Melting Point (°C) |
|---|---|---|---|---|
| 33 | $-CH_2CH_3$ | 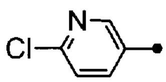 | S | 125-126 |

(continued)

| Compound No. | $Z_\alpha$ | Ar | X | Melting Point (°C) |
|---|---|---|---|---|
| 34 | $-CH_2Cl$ | 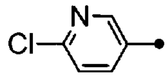 | S | 164-165 |
| 35 | $-CF_3$ | 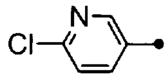 | S | 130-131 |
| 36 | $-CH_2CF_3$ | 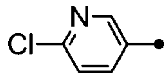 | S | 117-118 |
| 37 | $-CH=CH_2$ | 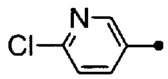 | S | 117-118 |
| 38 | $-CH_2CH_2CH_3$ | 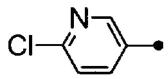 | S | 94 |
| 39 | $-CH(CH_3)_2$ | 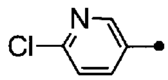 | S | 92 |
| 40 | 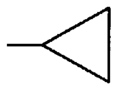 | 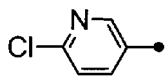 | S | 113-115 |
| 41 | $-CH_2CH_2CH_2Cl$ | 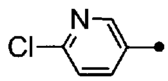 | S | 97 |
| 42 | $-C_6H_5$ | 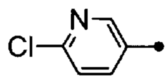 | S | 162-164 |
| 43 | $-CH_2C_6H_5$ | 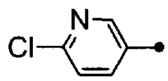 | S | 149 |
| 44 | $-CH=CH-C_6H_5$ | 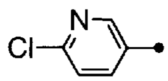 | S | 172-174 |
| 76 | $-CH_3$ | 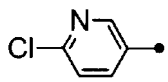 | S | 127 |

[Table 7]

| Compound No. | $Z_\alpha$ | Ar | X | Melting Point (°C) |
|---|---|---|---|---|
| 45 | 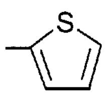 | 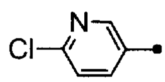 | S | 152-154 |
| 46 | 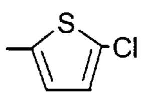 | 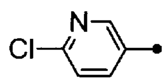 | S | 175-176 |
| 47 | 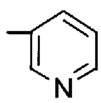 | 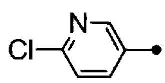 | S | 151-152 |
| 48 | $-NH_2$ | 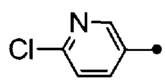 | S | 182-184 |

(continued)

| Compound No. | $Z_\alpha$ | Ar | X | Melting Point (°C) |
|---|---|---|---|---|
| 49 | $-N(CH_3)_2$ | 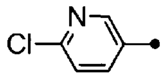 | S | 133-134 |
| 50 | $-NHCO_2CH_2CH_3$ | 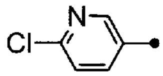 | S | 135-137 |
| 51 | $-NHCO_2C(CH_3)_3$ | 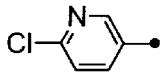 | S | 58-60 |
| 52 | 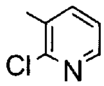 | 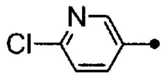 | S | 168-169 |
| 53 | 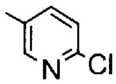 | 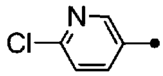 | S | 183 |
| 54 | $CH_2CH_2OMe$ | 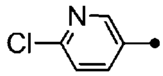 | S | 63-64 |
| 55 | $-CH=CH_2$ | 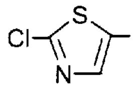 | S | 116 |
| 56 | 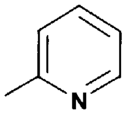 | 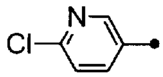 | S | 148-149 |
| 57 | $CH_2CH_2OMe$ | 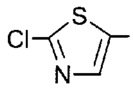 | S | 65 |

[Table 8]

| Compound No | IR(KBr, •, $cm^{-1}$) | NMR ($CDCl^3$, ••• ppm) |
|---|---|---|
| 33 | 1566, 1543, 1465, 1441,1417 | 1.37 (3H, t), 3.10 (2H, q), 3.29 (2H, t), 3.67 (2H, t), 4.71 (2H, s), 7.35 (1H, d), 7.66 (1H, dd), 8.33 (1H, d) |
| 34 | 1567, 1545, 1460, 1440,1415 | 3.29 (2H, t), 3.71 (2H, t), 4.56 (2H, s), 4.73 (2H, s), 7.36 (1H, d), 7.70 (1H, dd), 8.33 (1H, d) |
| 35 | 1556, 1460, 1337, 1216, 1197 | 3.37 (2H, t), 3.82 (2H, t), 4.78 (2H, s), 7.38 (1H, d), 7.66 (1H, dd), 8.32 (1H, d) |
| 36 | 1569, 1543, 1466, 1445,1412 | 3.30 (2H, t), 3.70 (2H, t), 3.88 (2H, m), 4.72 (2H, s), 7.36 (1H, d), 7.65 (1H, dd), 8.31 (1H, d) |
| 37 | 1567, 1540, 1463, 1442,1411 | 3.25 (2H, t), 3.66 (2H, t), 4.71 (2H, s), 5.87 (1H, d), 6.31 (1H, d), 6.66 (1H, dd), 7.34 (1H, d), 7.66 (1H, dd), 8.30 (1H, d) |
| 38 | 1567, 1545, 1467, 1441, 1417 | 1.05 (3H, t), 1.87 (2H, m), 3.06 (2H, m), 3.24 (2H, t), 3.64 (2H, t), 4.69 (2H, s), 7.34 (1H, d), 7.64 (1H, dd), 8.31 (1H, d) |
| 39 | 2924, 1545, 1457, 1295, 1259 | 1.37 (6H, d), 3.18-3.26 (3H, overlap), 3.64 (2H, t), 4.69 (2H, s), 7.35 (1H, d), 7.64 (1H, dd), 8.31 (1H, d) |
| 40 | not yet measured | 0.97 (2H, m), 1.24 (2H, m), 2.56 (1H, m), 3.25 (2H, t), 3.66 (2H, t), 4.72 (2H, s), 7.34 (1H, d), 7.69 (1H, dd), 8.34 (1H, d) |

(continued)

| Compound No | IR(KBr, •, $cm^{-1}$) | NMR ($CDCl^3$, ••• ppm) |
|---|---|---|
| 41 | 2924, 1567, 1547, 1463, 1414 | 2.30 (2H, m), 3.72 (6H, overlap), 3.66 (2H, t), 3.70 (2H. t), 4.70 (2H, s), 7.36 (1H, d), 7.65 (1H, dd), 8.32 (1H, d) |
| 42 | 3412, 2923, 1538, 1461, 1284 | 3.22 (2H, t), 3.63 (2H, t), 4.66 (2H, s), 7.22 (1H, d), 7.47-7.56 (4H, overlap), 7.93 (2H, m), 8.21 (1H, d) |
| 43 | 1550, 1442, 1420, 1277, 1122 | 3.21 (2H, t), 3.75 (2H, t), 4.30 (2H, s), 4.72 (2H, s), 7.31-7.37 (6H, m), 7.63 (1H, bd), 8.33 (1H, bs) |
| 44 | 1617, 1541, 1411, 1300, 1130 | 3.25 (2H, t), 3.65 (2H, t), 4.72 (2H, s), 6.90 (1H, d), 7.31 (1H, d), 7.40 (2H, m), 7.49 (1H, m), 7.54 (1H, d), 7.68 (1H, m), 8.31 (1H, bs) |
| 76 | 1283, 1397, 1465, 1543,1656 | 3.20 (3H, s), 3.27 (2H, t), 3.67 (2H, t), 4.71 (2H, s), 7.34 (1H, d), 7.67 (1H, dd), 8.32 (1H, d) |

[Table 9]

| Compound No | IR (KBr, · $cm^{-1}$) | NMR (CDCl3, ···ppm) |
|---|---|---|
| 45 | 1551, 1458, 1433, 1415, 1403 | 3.25 (2H, d), 3.67 (2H, d), 4.70 (2H, s), 7.05 (1H, m). 7.27 (1H, d), 7.55 (1H, m), 7.60 (1H, m), 7.65 (1H, m), 8.21 (1H, d) |
| 46 | 1566, 1543, 1519, 1461, 1412 | 3.26 (2H, t), 3.68 (2H, t), 4.70 (2H, s), 6.88 (1H, d), 7.31 (1H, d), 7.44 (1H, d), 7.62 (1H, dd), 8.27 (1H, d) |
| 47 | 1538, 1463, 1414, 1395, 1307 | 3.25 (2H, d), 3.66 (2H, d), 4.68 (2H, s), 7.28 (1H, d), 7.43 (1H, m), 7.55 (1H, m), 8.21-8.23 (2H, overlap), 8.78 (1H, m), 9.16 (1H, d) |
| 48 | 3355, 1572, 1550, 1318, 1147 (rev) | 3.23 (2H, t), 3.66 (2H, t), 4.67 (2H, s). 6.10 (2H, bs), 7.35 (1H, d), 7.74 (1H, dd), 8.34 (1H, d) (DMSO-d6) |
| 49 | 3560, 3486, 3408, 1555, 1466 | 2.76 (6H, s), 3.24 (2H, t), 3.65 (2H, t), 4.69 (2H, s), 7.35 (1H, d), 7.65 (1H, dd), 8.33 (1H, d) |
| 50 | 3091, 1731, 1549, 1469, 1329 | 1.28 (3H, t), 3.30 (2H, t), 3.72 (2H, t), 4.19 (2H, q), 4.72 (2H, s), 7.32 (1H, d), 7.43 (1H, s, 7.70 (1H, dd), 8.30 (1H, d) |
| 51 | 3554, 2981, 1737, 1708, 1549 | 1.48, 3.27 (2H, t), 3.71 (2H, t), 4.73 (2H, s), 7.28 (1H, d), 7.38 (1H, s), 7.71 (1H, dd), 8.31 (1H, d) |
| 52 | 1565, 1537, 1464, 1422,1392 | 3.28 (2H, t), 3.69 (2H, t), 4.71 (2H, s), 7.30 (1H, d), 7.42 (1H, dd), 7.64 (1H, dd), 8.26 (1H, d), 8.50 (1H, dd). 8.55 (1H, dd) |
| 53 | 1550, 1464, 1448, 1424 | 3.27 (2H, t), 3.68 (2H, t), 4.68 (2H, s), 7.31 (1H, d), 7.45 (1H, d), 7.57 (1H, dd), 8.14 (1H, dd), 8.24 (1H, d), 8.93 (1H, d) |
| 54 | 1710, 1542, 1461, 1415 | 3.23 (2H, t), 3.35 (3H, s), 3.38 (2H, t), 3.64 (2H, t), 3.82 (2H, t), 4.70 (2H, s), 7.34 (1H, d), 7.68 (1H, dd), 8.33 (1H, d) |
| 55 | 1541, 1420, 1412, 1305, 1293 | 3.27 (2H, d), 3.71 (2H, d), 4.78 (2H, s), 5.90 (1H, d), 6.33 (1H, d), 6.67-6.73 (1H, m), 7.46 (1H, s) |
| 56 | 1539, 1464, 1442, 1412, 1396 | 3.27 (2H, t), 3.69 (2H,t), 4.71 (2H, s), 7.23 (1H, d), 7.48 (1H, m), 7.65 (1H, m), 7.90 (1H, m), 8.08 (1H, m), 8.24 (1H, d), 8.72 (1H, m) |
| 57 | 1541, 1466, 1409, 1248 | 3.26 (2H, t), 3.38 (3H, s), 3.42 (2H, t), 3.70 (2H, t), 3.86 (2H, t), 4 78(2H,S), 7.48(1H,s) |

<Production Example 2-1>

Synthesis of 3-(2-chloro-s-pyridylmethyl)-2-(ethanesulfonylimino)-thiazolidine ("Table 6", Compound No. 33)

To a solution of 3-(2-chloro-5-pyridylmethyl)-2-iminothiazolidine (1) (228 mg, 1.0 mmol) and triethylamine (120 mg, 1.1 mmol)in acetonitrile (20 ml), under cooling with ice, a solution of ethanesulfonyl chloride (128 mg, 1.0 mmol) in acetonitrile (5 ml) was added dropwise. Stirring was conducted for 5 hours, and the solvent was distilled away under reduced pressure. The residue was combined with water and the precipitated crystal was recovered by filtration under reduced pressure. After drying, followed by recrystallization from ethyl acetate, the intended product was obtained. Yield: 385 mg (83%)

<Formulation Example 2-1>

1. Powder formulation

3 Parts by weight of the sulfonylimino derivative of Compound No. 33 (Table 6), 40 parts by weight of a clay, and 57 parts by weight of a talc were ground respectively and mixed to obtain a powder formulation.

<Formulation Example 2-2>

2. Wettable powder

50 Parts by weight of the sulfonylimino derivative of Compound No. 38 (Table 6), 5 parts by weight of lignin sulfonate, 3 parts by weight of an alkyl sulfonate, and 42 parts by weight of a kieselguhr were ground respectively and mixed to obtain a wettable powder.

<Formulation Example 2-3>

3. Granule formulation

5 Parts by weight of the sulfonylimino derivative of Compound No. 48 (Table 7), 43 parts by weight of a bentonite, 45 parts by weight of a clay, and 7 parts by weight of lignin sulfonate were mixed uniformly, combined with water and kneaded, and then subjected to an extruding granulator to fabricate into granules, which were dried to obtain a granule formulation.

<Formulation Example 2-4>

4. Emulsifiable concentrate

20 Parts by weight of the sulfonylimino derivative of Compound No. 50 (Table 7), 10 parts by weight of a polyoxyethylene alkylallyl ether, 3 parts by weight of a polyoxyethylene sorbitan monolaurate, and 67 parts by weight of a xylene were mixed uniformly while dissolving to obtain an emulsifiable concentrate.

<Formulation Example 2-5>

5. Liquefied drop

20 Parts by weight of the imino derivative of Compound No.35 (Table 6), 66.7 parts by weight of diethylene glycol monoethyl ether, and 13.3 parts by weight of ethanol were mixed uniformly to yield a liquefied drop.

<Experimental Example 2-1>

1. Green peach aphid controlling effect

Onto a two leaf-stage Chinese cabbage seedling, 30 adult insects of green peach aphid were allowed to inhabit, and allowed to stand for several days to accomplish settlement. Thereafter, the overground part was cut off, was immersed in a 100 μg/ml treatment solution. This was placed on a moistened filter paper placed on the bottom of a petri dish of 9 cm in diameter, and the petri dish was covered with the stopper, and allowed to stand in a room whose temperature

was constant at 25°C. After 120 hours, the numbers of viable and dead insects were counted.

As a result, Compound Nos. 36, 37, 39, 41, 42, 44, 45, 47, 49, and 55, at an active ingredient concentration of 100 μg/ml, exhibited an effect as excellent as a lethality of 50% or higher.

<Experimental Example 2-2>

2. Onion thrips controlling effect

A two leaf-stage Chinese cabbage seedling was immersed in a treatment solution at 100 μg/ml, placed on a moistened filter paper placed on the bottom of a petri dish of 9 cm in diameter. This was allowed to be inhabited with onion thrips larvae, and then the petri dish was covered with the stopper, and then allowed to stand in a room whose temperature was constant at 25°C. After 120 hours, the numbers of viable and dead insects were counted.

As a result, Compound Nos. 36, 38, 39, 41, 42, 44, 45, 47, 49, and 55, at an active ingredient concentration of 100 μg/ml, exhibited an effect as excellent as a lethality of 50% or higher.

<Experimental Example 2-3>

3. Diamondback moth controlling effect

A two leaf-stage Chinese cabbage seedling was immersed in a treatment solution at 500 μg/ml, placed on a moistened filter paper placed on the bottom of a petri dish of 9 cm in diameter. This was allowed to be inhabited with diamondback moth 2nd instar larvae, and then the petri dish was covered with the stopper, and then allowed to stand in a room whose temperature was constant at 25°C. After 120 hours, the numbers of viable and dead insects were counted.

As a result, Compound Nos. 36, 37, 38, 39, 42, 44, 45, 47, 49, and 55, at an active ingredient concentration of 100 μg/ml, exhibited an effect as excellent as a lethality of 50% or higher.

<Experimental Example 2-4>

4. Bush tick controlling effect

30 μL of an acetone solution of an imino derivative according to the invention prepared at 200 μg/ml was placed in a 4 mL glass vial. This was placed on a shaker, and a dry film of the compound was formed on the inner wall of the vial. After the vial was dried for 24 hours or longer, it was allowed to be inhabited by 10 juvenile bush ticks and closed with a stopper. The vial was allowed to stand in an entirely dark thermostat chamber at 25°C and 85% humidity. After 1 day of inhabitation, the % dead insect was calculated according to the following equation. The experiment was conducted in duplicate.

$$\text{\% Dead insect} = \{\text{Number of dead insects}/(\text{Number of surviving insects} + \text{Number of dead insects})\} \times 100$$

As a result, Compound Nos. 34, 35, 36, 38, 40, 45, 48, 49, and 54 exhibited an excellent effect as reflected by a % dead insect of 60% or higher.

[3. Production Examples and Formulation Examples, Experimental Examples of oxime ether derivatives]

Production Examples and Formulation Examples, Experimental Examples of the oxime ether derivatives and the insecticides according to the invention are shown below thereby describing the invention specifically. The compounds employed in the below-indicated Production Examples and the like may be commercially available products as appropriate. Determination of the physical characteristics of intended compounds in respective Examples was conducted under the conditions indicated in "Table 1" shown above. Unless otherwise indicated, $CDCl_3$ was employed as a solvent.

The intended compounds obtained in Examples of oxime ether derivatives represented by Formula (3) shown above had the chemical structures and the melting points shown in "Table 10" and "Table 11" and the measured values of IR spectra and NMR spectra shown in "Table 12". Among those, some Examples are detailed below with regard to Production Examples, Formulation Examples, and Experimental Examples.

[Table 10]

| Compound No | $Z_\beta$ | Ar | X | Melting Point (°C) |
|---|---|---|---|---|
| 58 | -H | 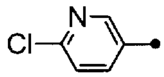 | S | 138-141 |
| 59 | $-CH_3$ | 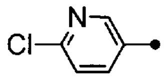 | S | 67-69 |
| 60 | $-CH_2C_6H_5$ | 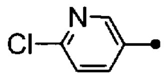 | S | 100-102 |
| 61 | $-COCH_3$ | 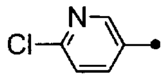 | S | 76-78 |
| 62 | $-COC_2H_5$ | 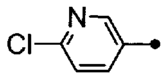 | S | Liquid |
| 63 | $-COC_6H_5$ | 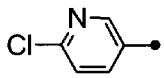 | S | 95-97 |
| 64 | $-COCH_2OCH_2CH_3$ | 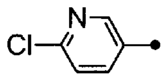 | S | Liquid |
| 65 | $-COCH_2CH_2OCH_3$ | 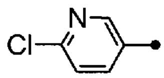 | S | Liquid |
| 66 | 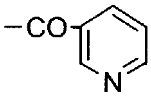 | 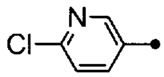 | S | 114-116 |

[Table 11]

| Compound No | $Z_\beta$ | Ar | X | |
|---|---|---|---|---|
| 67 | $-CONHCH_3$ | 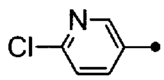 | S | 170-172 |
| 68 | $-CONHCH_2CH_3$ | 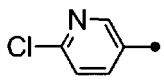 | S | 128-130 |
| 69 | $-CONHCH_2CH_2CH_3$ | 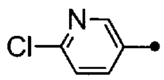 | S | 94-96 |
| 70 | $-CONHC_6H_5$ | 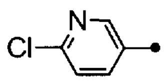 | S | 98-100 |
| 71 | $-CON(CH_3)_2$ | 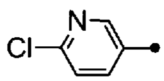 | S | 106-109 |
| 72 | $-SO_2CH_3$ | 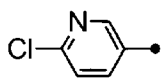 | S | 135-137 |
| 73 | $-SO_2CH_2CH_3$ | 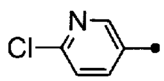 | S | 143-145 |

(continued)

| Compound No | $Z_\beta$ | Ar | X | |
|---|---|---|---|---|
| 77 | 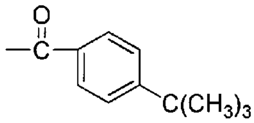 | 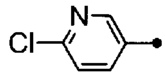 | S | 121-123 |
| 78 | -COCH2Cl | 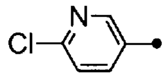 | S | 97-99 |
| 79 | 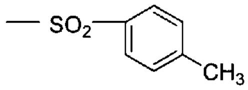 | 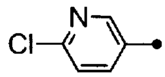 | S | 143-145 |

[Table 12]

| Compound No | IR (KBr, ·, $cm^{-1}$) | NMR ($CDCl_3$, ··· ppm) |
|---|---|---|
| 58 | 3422, 1630, 1457, 1385, 1310 | 3 08 (2H, dd), 3.36 (2H, dd), 4.40 (2H, s), 7.31 (1H, d), 7.70 (1H, bd), 8.33 (1H, bs), 9.10 (1H, bs) |
| 59 | 1600, 1567, 1460, 1440, 1385 | 3.06 (2H, dd), 3.35 (2H, dd), 3.79 (3H, s), 4 40 (2H, s), 7.30 (1H, d), 7 69 (1H, dd), 8.34 (1H, d) |
| 60 | 1595, 1598, 1459, 1383, 1233 | 3.05 (2H, dd), 3 32 (2H, dd), 4.36 (2H, s), 4.98 (2H, s), 7.16 (1H, d), 7.29-7.38 (5H), 7.49 (1H, dd), 8.25 (1H, d) |
| 61 | 1749, 1584, 1461, 1388, 1290 | 2.15 (3H, s), 3.12 (2H, t), 3.48 (2H, t), 4 54 (2H, s), 7.33 (1H, d), 7.80 (1H, bd), 8.33 (1H, bs) |
| 62 | 1756, 1580, 1461, 1388, 1291 | 1.22 (3H, t), 2.43 (2H, q), 3.12 (2H, t), 3 47 (2H, t), 4.55 (2H, s). 7.33 (1H, d), 7.83 (1H, dd), 8.30 (1H, d) |
| 63 | 1732, 1574, 1460, 1389, 1293 | 3.16 (2H, t), 3.52 (2H, t). 4.62 (2H, s), 7.35 (1H, d), 7.47 (2H, m), 7.58 (1H, m), 7.88 (1H, dd), 8 07 (2H, m), 8.30 (1H, d) |
| 64 | 1624, 1586, 1523, 1460, 1445 | 3.22 (2H, dd), 3.66 (2H, dd), 4.98 (2H, s), 7.33 (1H, d), 7.37 (1H, m), 7.69 (1H, dd), 8.48 (1H, m), 8.72 (1H, m), 8.39 (1H, d), 9.47 (1H, d) |
| 65 | 1769, 1578, 1460, 1444, 1413 | 1.27 (3H, t), 3.14 (2H, t), 3.49 (2H, t), 3.64 (2H, q), 4.20 (2H, s), 4.54 (2H, s), 7.33 (1H, d), 7 81 (1H, dd), 8 30 (1H, d) |
| 66 | 1755, 1579, 1461, 1388, 1248 | 2.67 (3H, t), 3.12 (2H, t), 3.48 (2H,), 3 72 (2H, q), 4.54 (2H, s), 7.33 (1H, d), 7.82 (1H, dd), 8.32 (1H, d) |
| 67 | 3330, 1704, 1600, 1503, 1460 | 2.86 (3H, d), 3.14 (2H, t), 3.51 (2H, t), 4 48 (2H, s), 5.91 (1H, bs), 7.35 (1H, d), 7.67 (1H, bd), 8.33 (1H, bs) |
| 68 | 3343, 1711, 1590, 1496, 1477 | 1.15 (3H, t), 3.13 (2H, t), 3.28 (2H, q), 3.51 (2H, t), 4 47 (2H, s), 5 81 (1H, bs), 7.33 (1H, d), 7.68 (1H, dd), 8.32 (1H, d) |
| 69 | 3350, 1710, 1598, 1505, 1464 | 0.91 (3H, t), 1 54 (2H, dt), 3.15 (2H, t), 3.19 (2H, dd), 3.51 (2H, t), 4.47 (2H, s), 5 90 (1H, bs), 7.34 (1H, d), 7 68 (1H, dd), 8 33 (1H, d) |
| 70 | 3366, 1742, 1659, 1584, 1512 | 3.16 (2H, t), 3.56 (2H, t), 4 51 (2H, s), 7.25-7.33 (3H, m), 7 34 (1H, d), 7.41 (2H, m), 7.70 (dd), 7.80 (1H, bs). 8.36 (1H, d) |
| 71 | 1718, 1583, 1458, 1387, 1168 | 2.97 (6H, s), 3.11 (2H, t), 3.45 (2H, t), 4.55 (2H, s), 7.33 (1H, d), 7.80 (1H, bd), 8 32 (1H, bs) |

(continued)

| Compound No | IR (KBr, ·, $cm^{-1}$) | NMR ($CDCl_3$, ··· ppm) |
|---|---|---|
| 72 | 1585, 1561, 1467, 1450, 1417 | 3.10 (3H, s), 3.18 (2H, t), 3.60 (2H, t), 4.49 (22H, s), 7.35 (1H, d),7.70 (1H, dd), 8.34 (1H, d) |
| 73 | 1584, 1562, 1460, 1445, 1417 | 2.46 (3H, s), 3 11 (2H, t), 3.51 (2H, t). 4.34 (2H, s), 7.20 (1H, d), 7.30 (1H, d), 7.48 (dd), 7.78 (2H, d), 8.34 (1H, d) |
| 77 | 1266, 1389, 1441, 1458, 1581, 1741 | 1.33 (9H, s), 3.14 (2H, t), 3.49 (2H, t), 4.60 (2H, s), 7.33 (1H, d), 7.49 (2H, d), 7.87 (1H, dd), 7.97 (2H, d), 8.34 (1H, d) |
| 78 | 1315, 1461, 1573, 1768 | 3.16 (2H, t). 3.53 (2H, t), 4.16 (2H, s), 4.54 (2H, s), 7.33 (1H, d), 7.81 (1H, dd), 8.34 (1H, d) |
| 79 | 1359, 1385, 1445, 1461, 1584 | 2.46 (3H, s), 3.11 (2H, t), 3.51 (2H, t), 4.34 (2H, s), 7.22 (1H, d), 7.31 (2H, d), 7.50 (1H, dd), 7.79 (2H, d), 8.17 (1H, d) |

<Production Example 3-1>

Synthesis of 3-(2-chloro-5-pyridinylmethyl)-2-(hydroxyimino)-thiazolidine ("Table 10", Compound No. 58)

To a suspension of 3-(2-chloro-5-pyridylmethyl)-2-iminothiazolidine (1) (682 mg, 3.0 mmol) and hydroxyamine hydrochloride (480 mg, 6.9 mmol) in ethanol, sodium acetate (560 mg, 6.8 mmol) was added and then heating under reflux was conducted for 4 hours. Ethanol was distilled away under reduced pressure and the resultant residue was crystallized upon washing with water. This crystal was recovered by filtration and recrystallized from ethyl acetate.
Product: 627 mg (yield: 86%)

<Production Example 3-2>

Synthesis of 3-(2-chloro-5-pyridinylmethyl)-2-(acetylimino)-thiazolidine ("Table 10", Compound No. 61)

To a solution of 3-(2-chloro-5-pyridylmethyl)-2-hydroxyiminothiazolidine (2) synthesized in accordance with the method described in Journal of Medicinal Chemistry 1999, 42(12), 2227-2234 (122 mg, 0.5 mmol) and triethylamine (60 mg, 0.6 mol) in 8 ml of acetonitrile, under cooling with ice, a solution of acetyl chloride (39 mg, 0.5 mmol) in 2 ml of acetonitrile was added dropwise. After stirring overnight at room temperature, acetonitrile was distilled away under reduced pressure. The residue was dissolved in ethyl acetate, washed with a saturated aqueous solution of sodium hydrogen carbonate, and then dried over magnesium sulfate. The desiccant was filtered off, and the filtrate was distilled away under reduced pressure. The remaining crude product crystal was recovered by filtration, separated and purified by a silica gel-packed column chromatography (eluent: ethyl acetate) to obtain the intended product. Product: 73 mg (yield: 51%)

<Production Example 3-3>

3-(2-Chloro-5-pyridylmethyl)-2-(dimethylcarbamoyloxyimino)-thiazolidine ("Table 11", Compound No. 71)

3-(2-Chloro-5-pyridylmethyl)-2-hydroxyiminothiazolidine (2) (244 mg, 1.0 mmol) was dissolved in 10 ml of dimethyl formamide, and then under cooling with ice sodium hydride (1 mmol) was added. After stirring for 15 minutes, dimethylcarbamoyl chloride (107 mg, 1 mmol) was added, and the stirring was continued overnight. The reaction mixture was poured into an ice-water (30 ml), and the chloroform extract was washed with a saturated aqueous solution of sodium hydrogen carbonate, and then dried over magnesium sulfate. The desiccant was filtered off, the filtrate was distilled away under reduced pressure, and the remaining semi-solid was crystallized using ether.
Product: 246 mg (yield: 78%)

<Production Example 3-4>

3-(2-Chloro-5-pyridylmethyl)-2-(nicotinoyloxyimino)-thiazolidine ("Table 10", Compound No. 66)

3-(2-Chloro-5-pyridylmethyl)-2-iminothiazolidine (228 mg, 1.0 mmol), nicotinic acid (123 mg, 1 mmol), 1-ethyl-3-(3-dimethylaminopropyl)-carbodiimide hydrochloride (192 mg, 1 mmol), and 4-dimethylaminopyridine (134 mg, 1.1 mmol) were suspended in methylene chloride (20 ml), and stirred for 12 hours at room temperature. The reaction mixture solution was combined with 20 ml of methylene chloride, and insolubles were filtered off. The filtrate was washed with 1% hydrochloric acid and then water, and dried. The solvent was distilled away, and the crude product was purified by recrystallization from methanol. Product: 203 mg (yield: 61%)

<Production Example 3-5>

3-(2-Chloro-5-pyridylmethyl)-2-(methylcarbamoyloxyimino)-thiazolidine ("Table 11", Compound No. 67)

A mixture of 3-(2-chloro-5-pyridylmethyl)-2-hydroxyiminothiazolidine (2) (244 mg, 1.0 mmol), methyl isocyanate (85 mg, 1.5 mmol), and potassium t-butyrate (10 mg) was suspended in 20 ml of acetonitrile, and heated under reflux for 12 hours. The solvent was distilled away under reduced pressure, and the residue was combined with water, and the precipitated crystal was recrystallized from methanol to obtain the intended substance.
Product: 187 mg (yield: 62%)

<Production Example 3-6>

3-(2-Chloro-5-pyridylmethyl)-2-(methanesulfonyloxyimino)-thiazolidine ("Table 11", Compound No. 72)

To a solution of 3-(2-chloro-5-pyridylmethyl)-2-hydroxyiminothiazolidine (122 mg, 0.5 mmol) and triethylamine (60 mg, 0.6 mmol) in 5 ml of acetonitrile, under cooling with ice, methanesulfonyl chloride (60 mg, 0.52 mmol) was added dropwise. The mixture was allowed to reach room temperature slowly, and the stirring was continued for 12 hours. The reaction solution was poured into an ice-water, and the organics were extracted with ethyl acetate. This extract was washed with water and dried, and then the extraction solvent was distilled away. The residue was crystallized from a small amount of methanol, and the crystal was washed with ether. Product: 266 mg (yield: 83%)

<Formulation Example 3-1>

1. Powder formulation

3 Parts by weight of the oxime ether derivative of Compound No.1 (Table 2), 40 parts by weight of a clay, 57 parts by weight of a talc were ground respectively and mixed to obtain a powder formulation.

<Formulation Example 3-2>

2. Wettable powder

50 Parts by weight of the oxime ether derivative of Compound No. 62 (Table 10), 5 parts by weight of lignin sulfonate, 3 parts by weight of an alkyl sulfonate, 42 parts by weight of a kieselguhr were ground respectively and mixed to obtain a wettable powder.

<Formulation Example 3-3>

3. Granule formulation

5 Parts by weight of the oxime ether derivative of Compound No. 66 (Table 10), 43 parts by weight of a bentonite, 45 parts by weight of a clay, 7 parts by weight of lignin sulfonate were mixed uniformly, combined with water and kneaded, and then subjected to an extruding granulator to fabricate into granules, which was dried to obtain a granule formulation.

<Formulation Example 3-4>

4. Emulsifiable concentrate

20 Parts by weight of the oxime ether derivative of Compound No. 71 (Table 11), 10 parts by weight of a polyoxyethylene alkylallyl ether, 3 parts by weight of a polyoxyethylene sorbitan monolaurate, 67 parts by weight of a xylene were mixed uniformly while dissolving to obtain an emulsifiable concentrate.

<Formulation Example 3-5>

5. Liquefied drop

20 Parts by weight of the imino derivative of Compound No.62 (Table 10), 66.7 parts by weight of diethylene glycol monoethyl ether, and 13.3 parts by weight of ethanol were mixed uniformly to yield a liquefied drop.

<Experimental Example 3-1>

1. Green peach aphid controlling effect

Onto a two leaf-stage Chinese cabbage seedling, 30 adult insects of green peach aphid were allowed to inhabit, and allowed to stand for several days to accomplish settlement. Thereafter, the overground part was cut off, was immersed in a 100 μg/ml treatment solution. This was placed on a moistened filter paper placed on the bottom of a petri dish of 9 cm in diameter, and the petri dish was covered with the stopper, and allowed to stand in a room whose temperature was constant at 25°C. After 120 hours, the numbers of viable and dead insects were counted.
As a result, Compound Nos. 59, 62, 63, 64, 65, 67, 68, 69, 70, 71, 72, and 73, at an active ingredient concentration of 100 μg/ml, exhibited an effect as excellent as a lethality of 50% or higher.

<Experimental Example 3-2>

2. Onion thrips controlling effect

A two leaf-stage Chinese cabbage seedling was immersed in a treatment solution at 100 μg/ml, placed on a moistened filter paper placed on the bottom of a petri dish of 9 cm in diameter. This was allowed to be inhabited with onion thrips larvae, and then the petri dish was covered with the stopper, and then allowed to stand in a room whose temperature was constant at 25°C. After 120 hours, the numbers of viable and dead insects were counted.
As a result, Compound Nos. 59, 60, 66, 67, 68, 69, 71, 72, and 73, at an active ingredient concentration of 100 μg/ml, exhibited an effect as excellent as a lethality of 50% or higher.

<Experimental Example 3-3>

3. Diamondback moth controlling effect

A two leaf-stage Chinese cabbage seedling was immersed in a treatment solution at 500 μg/ml, placed on a moistened filter paper placed on the bottom of a petri dish of 9 cm in diameter. This was allowed to be inhabited with diamondback moth 2nd instar larvae, and then the petri dish was covered with the stopper, and then allowed to stand in a room whose temperature was constant at 25°C. After 120 hours, the numbers of viable and dead insects were counted.
As a result, Compound Nos. 59, 60, 62, 63, 64, 66, 68, 69, 70, 72, and 73, at an active ingredient concentration of 100 μg/ml, exhibited an effect as excellent as a lethality of 50% or higher.

<Experimental Example 3-4>

4. Bush tick controlling effect

30 μL of an acetone solution of an imino derivative according to the invention prepared at 200 μg/ml or 10 μg/ml was placed in a 4 mL glass vial. This was placed on a shaker, and a dry film of the compound was formed on the inner wall of the vial. After the vial was dried for 24 hours or longer, it was allowed to be inhabited by 10 juvenile bush ticks and closed with a stopper. The vial was allowed to stand in an entirely dark thermostat chamber at 25°C and 85% humidity. After 1 day of inhabitation, the % dead insect was calculated according to the following equation. The experiment was conducted in duplicate.

$$\% \text{ Dead insect} = \{\text{Number of dead insects}/(\text{Number of surviving insects} + \text{Number of dead insects})\} \times 100$$

As a result, in the test at 200 μg/ml, Compound Nos. 58, 59, 62, 64, 67, 71, and 72 exhibited an excellent effect

as reflected by a % dead insect of 60% or higher.
Also in the test at 10 μg/ml, Compound Nos. 62, 71, and 72 exhibited an excellent effect as reflected by a % dead insect of 60% or higher.

[Industrial Applicability]

An imino derivative according to the invention and an insecticide containing the same as an active ingredient exhibit excellent controlling effects on agro-horticultural and household pests such as hemipterous pests. Accordingly, the invention can be employed widely in the field of agro-horticultural production, livestock, and household controlling situation, and can contribute to such industrial fields greatly.

## Claims

**1.** An imino derivative represented by Formula (A) shown below:

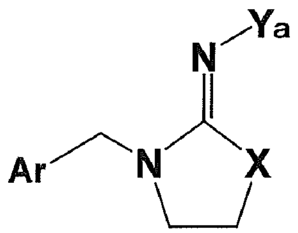

..... Formula (A)

wherein "Ar" represents a heterocyclic group which may have a substituent on the ring;
"X" represents a sulfur atom or $CH_2$, NR; "R" represents a hydrogen atom or an alkyl group;
"Ya" represents "$C(=S)NR_1R_2$", "$C(=S)SR_3$", "$C(=O)SR_4$", "$C(=S)OR_4$", "$SO_2Z_\alpha$", or "$OZ_\beta$";
when "Ya" is "$C(=S)NR_1R_2$", each of "$R_1$ and "$R_2$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a substituted or unsubstituted C6 to C10 arylcarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridylmethyl group, a substituted or unsubstituted pyridyl group, a substituted or unsubstituted imidazolylmethyl group, or a substituted or unsubstituted heterocyclic ring or aromatic ring; provided that "$R_1$" and "$R_2$" do not represent hydrogen atoms simultaneously; "$NR_1R_2$" may form a ring;
when "Ya" is "$C(=S)SR_3$", "$R_3$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C3)alkoxy (C1 to C3)alkyl group, a (C1 to C3)alkoxycarbonyl(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring;
when "Ya" is "$C(=O)SR_4$" or "$C(=S)OR_4$", "$R_4$" represents a C1 to C5 alkyl group, or a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted imidazolylmethyl group, a substituted or unsubstituted pyridyl group, a substituted or unsubstituted heterocyclic ring, aromatic ring;
when "Ya" is "$SO_2Z_\alpha$", "$Z_\alpha$" represents a hydrogen atom or a C1 to C5 alkyl group, a C1 to C5 halogenated

alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C6 to C10)aryl(C2 to C5)alkenyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted thienyl group, or a substituted or unsubstituted heterocyclic ring or aromatic ring; or it represents "$NR_5R_6$"; in "$NR_5R_6$", each of "$R_5$" and "$R_6$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C4 alkoxy group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C1 to C4 alkoxycarbonyl group, a C3 to C7 substituted or unsubstituted cycloalkyl group, a 3-membered ring to 7-membered ring substituted or unsubstituted heterocycloalkyl group, a substituted or unsubstituted pyridyl group, a substituted or unsubstituted heterocyclic ring or aromatic ring group; "$NR_5R_6$" may form a ring;

when "Ya" is "$OZ_\beta$", "$Z_\beta$" represents "$R_7$" "$COR_8$", "$CONR_9R_{10}$" or "$SO_2R_{11}$";

when "$Z_\beta$" represents $R_7$, "$R_7$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3) alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring;

when "$Z_\beta$" represents $COR_8$, "$R_8$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4) alkoxy(C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, a substituted or unsubstituted heterocyclic ring or aromatic ring;

when "$Z_\beta$" represents $CONR_9R_{10}$, each of "$R_9$" and "$R_{10}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a C1 to C3 alkoxycarbonyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted pyridyl group, a substituted or unsubstituted imidazolidylmethyl group, a substituted or unsubstituted heterocyclic ring or aromatic ring; "$NR_9R_{10}$" may form a ring;

when "$Z_\beta$" represents $SO_2R_{11}$, "$R_5$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted imidazolidylmethyl group, a substituted or unsubstituted thienyl group, or a substituted or unsubstituted heterocyclic ring or aromatic ring; and, provided that compounds wherein "Ar" is 2-chloro-5-pyridyl,

"X" is a sulfur atom or an oxygen atom, "Ya" is $SO_2$-methyl, or wherein "Ar" is 2-chloro-5-pyridyl, "Ya" is $SO_2$-substituted phenyl are excluded.

**2.** A method for producing a compound represented by Formula (7) shown below by reacting a compound represented by Formula (4) shown below with a compound represented by Formula (5) shown below or a compound represented by Formula (6) shown below:

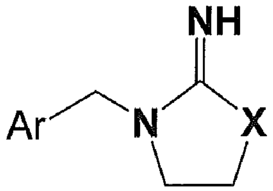

····· Formula (4)

**$R_1$NCS** Formula (5)

**$R_1R_2$NCSA** Formula (6)

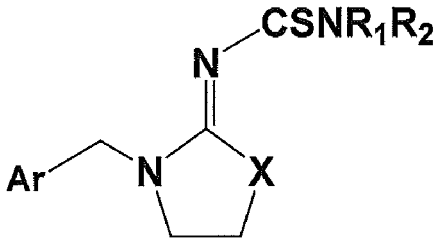

····· Formula (7)

wherein "Ar" represents a heterocyclic group which may have a substituent on the ring;
"X" represents a sulfur atom or $CH_2$, NR; "R" represents a hydrogen atom or an alkyl group;
each of "$R_1$" and "$R_2$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or
unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a substituted or unsubstituted C6 to C10 arylcarbonyl group, a (C1 to C3)alkylsulfonyl(C1 to C3)alkyl group, a (C1 to C3) alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or
unsubstituted heterocycloalkylmethyl group, a cyano(C1 to C3)alkyl group, a substituted or unsubstituted phenoxy(C1 to C3)alkyl group, a substituted or
unsubstituted pyridylmethyl group, a substituted or unsubstituted pyridyl group, a substituted or unsubstituted imidazolylmethyl group, or a substituted or unsubstituted heterocyclic ring or aromatic ring; "$NR_1R_2$" may form a ring; and,
"A" represents a halogen atom such as chlorine, bromine, or iodine.

**3.** A method for producing a compound represented by Formula (7) shown below by reacting a compound represented by Formula (9) shown below, which is produced by reacting a compound represented by Formula (4) shown below with a compound represented by Formula (8) shown below, with a compound represented by Formula (10) shown below:

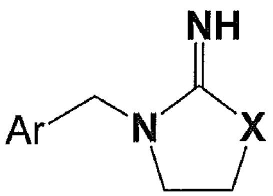

····· Formula (4)

**BtCSBt** Formula (8)

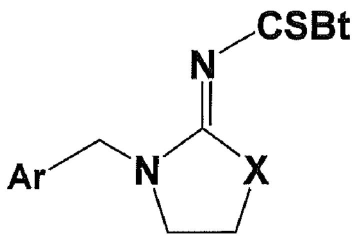

..... Formula (9)

$R_1NH_2$ Formula (10)

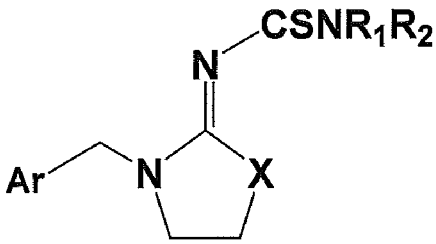

..... Formula (7)

wherein "Ar" represents a heterocyclic group which may have a substituent on the ring;
"X" represents a sulfur atom or $CH_2$, NR; "R" represents a hydrogen atom or an alkyl group;
"Bt" represents 1-(1,2,3-benzotriazole);
"$R_1$" represents a C1 to C5 alkyl group, a C1 to C5 halogenated alkyl group, a C2 to C5 alkenyl group, a C2 to C5 halogenated alkenyl group, a C2 to C5 alkynyl group, a C1 to C5 alkoxy group, a substituted or unsubstituted C6 to C10 aryl group, a substituted or unsubstituted (C6 to C10)aryl(C1 to C3)alkyl group, a (C1 to C4)alkoxy (C1 to C5)alkyl group, a C1 to C3 alkoxycarbonyl group, a C1 to C3 alkylcarbonyl group, a substituted or unsubstituted C6 to C10 arylcarbonyl group, a (C1 to C3)alkylsulfonyl(C1 to C3)alkyl group, a (C1 to C3)alkylthio (C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridylmethyl group, a substituted or unsubstituted pyridyl group, a substituted or unsubstituted imidazolylmethyl group, or a substituted or unsubstituted heterocyclic ring or aromatic ring; and, "$R_2$" represents a hydrogen atom.

**4.** A method for producing a compound represented by Formula (14) shown below by reacting a compound represented by Formula (4) shown below with a compound represented by Formula (11) shown below, a compound represented by Formula (12) shown below, or a compound represented by Formula (13) shown below:

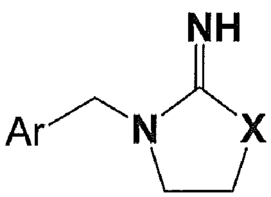

..... Formula (4)

$(R_3S)_2CS$ Formula (11)

$ClCS_2R_3$ Formula (12)

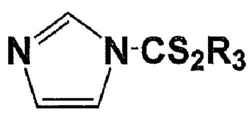

..... Formula (13)

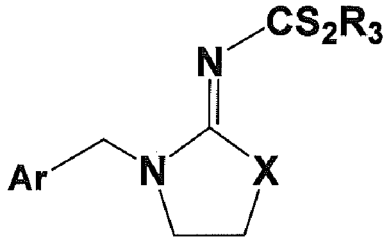

..... Formula (14)

wherein "Ar" represents a heterocyclic group which may have a substituent on the ring;
"X" represents a sulfur atom or $CH_2$, NR; "R" represents a hydrogen atom or an alkyl group; and,
"$R_3$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C3)alkoxy(C1 to C3)alkyl group, a (C1 to C3)alkoxycarbonyl(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or
unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

**5.** A method for producing a compound represented by Formula (16) shown below by reacting a compound represented by Formula (4) shown below with a compound represented by Formula (15) shown below:

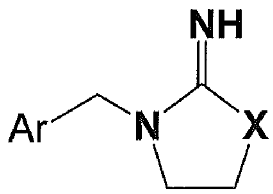

..... Formula (4)

**$BC(=P)QR_4$** Formula (15)

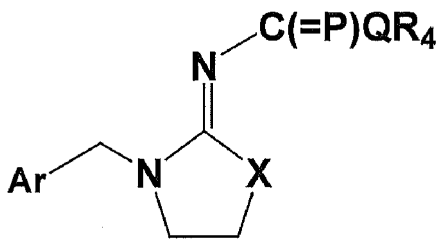

..... Formula (16)

wherein "Ar" represents a heterocyclic group which may have a substituent on the ring;
"X" represents a sulfur atom or $CH_2$, NR; "R" represents a hydrogen atom or an alkyl group;
"B" represents a halogen atom such as chlorine, bromine, or iodine;
each of "P", "Q" represents a sulfur atom or an oxygen atom; provided that "P" and "Q" are different from each other; and,
"$R_4$" represents a C1 to C5 alkyl group, or a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10)aryl(C1 to C3)alkyl group, a (C1 to C3)alkoxy (C1 to C3)alkyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted imidazolylmethyl group, a substituted or unsubstituted pyridyl group, a substituted or unsubstituted heterocyclic ring, aromatic ring.

**6.** A method for producing a compound represented by Formula (2) shown below by reacting a compound represented by Formula (4) shown below with a compound represented by Formula (17) shown below:

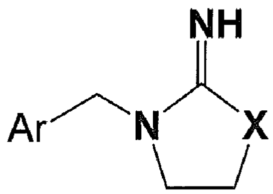

..... Formula (4)

**$Z_\alpha SO_2A$** Formula (17)

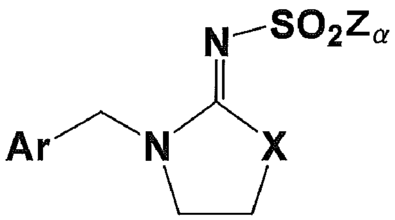

..... Formula (2)

wherein "Ar" represents a heterocyclic group which may have a substituent on the ring;
"X" represents a sulfur atom or $CH_2$, NR; "R" represents a hydrogen atom or an alkyl group;
"$Z_\alpha$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C6 to C10)aryl (C2 to C5)alkenyl group, a (C1 to C4)alkoxy(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5)alkyl group, a substituted or unsubstituted pyridylmethyl group, a substituted or unsubstituted pyridyl group, a substituted or unsubstituted thienyl group, or a substituted or unsubstituted heterocyclic ring or aromatic ring; or it represents "$NR_5R_6$"; in "$NR_5R_6$", each of "$R_5$" and "$R_6$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C4 alkoxy group, a substituted or unsubstituted C6 to C10 aryl group, a substituted or unsubstituted (C6 to C10)aryl(C1 to C3)alkyl group, a (C1 to C4)alkoxy (C1 to C3)alkyl group, a C1 to C4 alkoxycarbonyl group, a C3 to C7 substituted or unsubstituted cycloalkyl group, a 3-membered ring to 7-membered ring substituted or unsubstituted heterocycloalkyl group, a substituted or unsubstituted pyridyl group, a substituted or unsubstituted heterocyclic ring or aromatic ring group; "$NR_5R_6$" may form a ring; and,
"A" represents a halogen atom such as chlorine, bromine, or iodine or "$OSO_2Z_\alpha$".

**7.** A Method for producing a compound represented by Formula (19) shown below by reacting a compound represented by Formula (4) shown below with a compound represented by Formula (18) shown below:

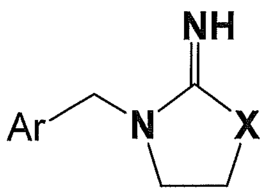

..... Formula (4)

**$NH_2OR_7$** Formula (18)

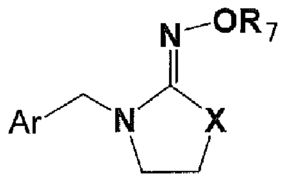

..... Formula (19)

wherein "Ar" represents a heterocyclic group which may have a substituent on the ring;
"X" represents a sulfur atom or $CH_2$, NR; "R" represents a hydrogen atom or an alkyl group; and,
"$R_7$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy (C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring.

**8.** A method for producing a compound represented by Formula (23) shown below by reacting a compound represented by Formula (20) shown below with a compound represented by Formula (21) shown below or a compound represented by Formula (22) shown below:

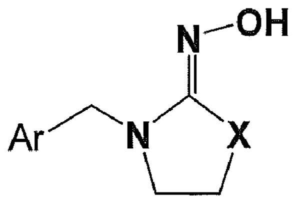

..... Formula (20)

**$R_8COA$** Formula (21)

**$R_8CO_2H$** Formula (22)

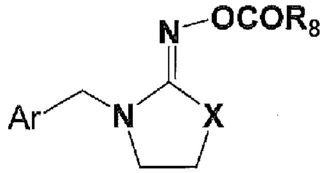

..... Formula (23)

wherein "Ar" represents a heterocyclic group which may have a substituent on the ring;
"X" represents a sulfur atom or $CH_2$, NR; "R" represents a hydrogen atom or an alkyl group;
"A" represents a halogen atom such as chlorine, bromine, and iodine; and,
"$R_8$" represents a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a C1 to C3 alkoxycarbonyl group, a (C1 to C3)alkylsulfonyl(C1 to C3)alkyl group, a (C1 to C3)alkylthio(C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a cyano(C1 to C3)alkyl group, a substituted or unsubstituted phenoxy(C1 to C3)alkyl group, a substituted or unsubstituted pyridyl group, a substituted or unsubstituted pyridylmethyl group, a substituted or unsubstituted imidazolylmethyl group, a substituted or unsubstituted heterocyclic ring or aromatic ring.

**9.** A method for producing a compound represented by Formula (26) shown below by reacting a compound represented

by Formula (20) shown below with a compound represented by Formula (24) shown below or a compound represented by Formula (25) shown below:

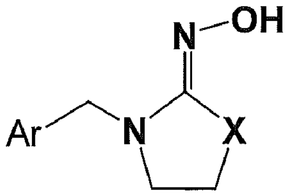

..... Formula (20)

$R_9NCO$ Formula (24)

$R_9R_{10}NCOB$ Formula (25)

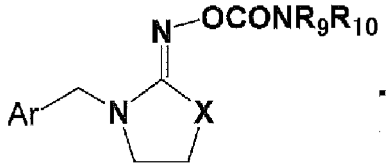

..... Formula (26)

wherein "Ar" represents a heterocyclic group which may have a substituent on the ring;
"X" represents a sulfur atom or $CH_2$, NR; "R" represents a hydrogen atom or an alkyl group;
"B" represents a halogen atom such as chlorine, bromine, and iodine;
each of "$R_9$" and "$R_{10}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a C1 to C3 alkoxy group, a substituted or unsubstituted C6 to C10 aryl group, a substituted or unsubstituted (C6 to C10) aryl(C1 to C3)alkyl group, a (C1 to C3)alkoxy(C1 to C3)alkyl group, a C1 to C3 alkoxycarbonyl group, a C1 to C3 alkoxycarbonylmethyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridylmethyl group, a substituted or unsubstituted pyridyl group, a substituted or unsubstituted imidazolidylmethyl group, a substituted or unsubstituted heterocyclic ring or aromatic ring; provided that in Formula (24) "$R_9$" is other than a hydrogen atom; and, "$NR_9R_{10}$" may form a ring.

**10.** A method for producing a compound represented by Formula (28) shown below by reacting a compound represented by Formula (20) shown below with a compound represented by Formula (27) shown below:

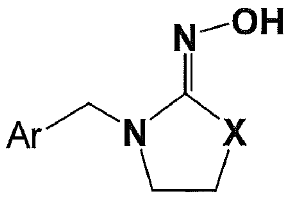

..... Formula (20)

$R_{11}SO_2D$ Formula (27)

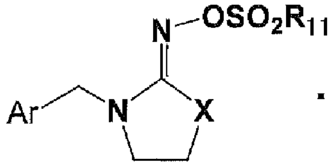

..... Formula (28)

wherein "Ar" represents a heterocyclic group which may have a substituent on the ring; "X" represents a sulfur atom or $CH_2$, NR; "R" represents a hydrogen atom or an alkyl group;

"D" represents a halogen atom such as chlorine, bromine, and iodine; and,

"$R_{11}$" represents a hydrogen atom or a C1 to C5 alkyl group, a C1 to C5 halogenated alkyl group, a C2 to C5 alkenyl group, a C2 to C5 halogenated alkenyl group, a C2 to C5 alkynyl group, a substituted or unsubstituted C6 to C10 aryl group, a substituted or unsubstituted (C6 to C10)aryl(C1 to C3)alkyl group, a (C1 to C4)alkoxy (C1 to C3)alkyl group, a C3 to C7 substituted or unsubstituted cycloalkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted phenoxy(C2 to C5) alkyl group, a substituted or unsubstituted pyridylmethyl group, a substituted or unsubstituted pyridyl group, a substituted or unsubstituted imidazolidylmethyl group, a substituted or unsubstituted thienyl group, or a substituted or unsubstituted heterocyclic ring or aromatic ring.

**11.** An insecticide containing as an active ingredient an imino derivative represented by Formula (A) according to Claim 1.

**12.** An imino derivative represented by Formula (A) shown below:

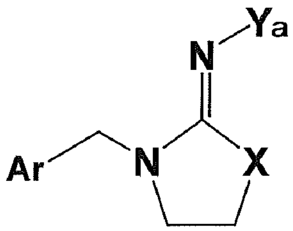

····· Formula (A)

wherein "Ar" represents a 2-chloro-5-pyridyl group or a 2-chloro-5-thiazolyl group;

"X" represents a sulfur atom;

"Ya" represents "$C(=S)NR_1R_2$", "$C(=S)SR_3$", "$C(=O)SR_4$", "$C(=S)OR_4$", or "$SO_2Z_\alpha$";

when "Ya" is "$C(=S)NR_1R_2$", each of "$R_1$" and "$R_2$" represents a hydrogen atom or a C1 to C5 alkyl group, a C1 to C5 halogenated alkyl group, a C6 to C10 aryl group, a (C6 to C10)aryl(C1 to C3)alkyl group, a (C1 to C4) alkoxy(C1 to C5)alkyl group, a C1 to C3 alkoxycarbonyl group, a C1 to C3 alkylcarbonyl group, a C6 to C10 arylcarbonyl group, a (C1 to C3)alkylthio(C1 to C3)alkyl group, a C3 to C7 cycloalkyl group;

when "Ya" is "$C(=S)SR_3$", "$R_3$" represents a C1 to C5 alkyl group, or a C6 to C10 aryl group, a (C6 to C10)aryl (C1 to C3)alkyl group, a (C1 to C3)alkoxy(C1 to C3)alkyl group, a (C1 to C3)alkoxycarbonyl(C1 to C3)alkyl group;

when "Ya" is "$C(=O)SR_4$" or "$C(=S)OR_4$", "$R_4$" represents a C1 to C5 alkyl group, or a C6 to C10 aryl group, a pyridyl group;

when "Ya" is "$SO_2Z_a$", "$Z_a$" represents a C2 to C5 alkyl group or a C1 to C5 halogenated alkyl group, a C2 to C5 alkenyl group, a C6 to C10 aryl group, a (C6 to C10)aryl(C1 to C3)alkyl group, a (C6 to C10)aryl(C2 to C5) alkenyl group, a (C1 to C4)alkoxy(C1 to C3)alkyl group, a C3 to C7 cycloalkyl group, a pyridyl group, a chloropyridyl group, a thienyl group, a chlorothienyl group; or it represents "$NR_5R_6$"; in "$NR_5R_6$", each of "$R_5$" and "$R_6$" represents a hydrogen atom or a C1 to C5 alkyl group, a C1 to C4 alkoxycarbonyl group;

when "Ya" is "$OZ_\beta$" "$Z_\beta$" represents "$R_7$" , "$COR_8$", "$CONR_9R_{10}$" " or "$SO_2R_{11}$";

when "$Z_\beta$" is $R_7$, "$R_7$" represents a hydrogen atom or a C1 to C5 alkyl group, a C6 to C10 aryl group, a (C6 to C10)aryl(C1 to C3)alkyl group;

when "$Z_\beta$" is $COR_8$, "$R_8$" represents a C1 to C5 alkyl group or a C6 to C10 aryl group, a (C1 to C4)alkoxy(C1 to C5)alkyl group, a pyridyl group;

when "$Z_\beta$" is $CONR_9R_{10}$, each of "$R_9$" and "$R_{10}$" represents a hydrogen atom or a C1 to C5 alkyl group, a C6 to C10 aryl group; and,

when "$Z_\beta$" is $SO_2R_{11}$, "$R_{11}$" represents a C1 to C5 alkyl group.

**13.** A method for producing a compound represented by Formula (7) shown below by reacting a compound represented by Formula (4) shown below with a compound represented by Formula (5) shown below or a compound represented by Formula (6) shown below:

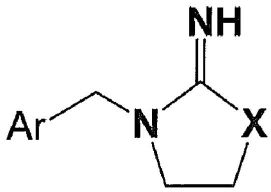

····· Formula (4)

$R_1NCS$ Formula (5)

$R_1R_2NCSA$ Formula (6)

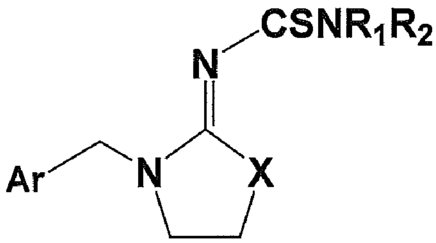

····· Formula (7)

wherein "Ar" represents a 2-chloro-5-pyridyl group or a 2-chloro-5-thiazolyl group;
"X" represents a sulfur atom;
each of "$R_1$" and "$R_2$" represents a hydrogen atom or a C1 to C5 alkyl group, a C1 to C5 halogenated alkyl group, a C6 to C10 aryl group, a (C6 to C10)aryl(C1 to C3)alkyl group, a (C1 to C4)alkoxy(C1 to C5)alkyl group, a C1 to C3 alkoxycarbonyl group, a C1 to C3 alkylcarbonyl group, a C6 to C10 arylcarbonyl group, a (C1 to C3) alkylthio(C1 to C3)alkyl group, a C3 to C7 cycloalkyl group; and,
"A" represents a halogen atom such as chlorine, bromine, or iodine.

**14.** A method for producing a compound represented by Formula (7) shown below by reacting a compound represented by Formula (9) shown below, which is produced by reacting a compound represented by Formula (4) shown below with a compound represented by Formula (8) shown below, with a compound represented by Formula (10) shown below:

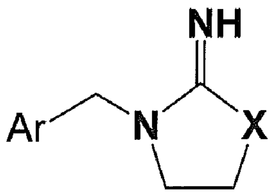

····· Formula (4)

BtCSBt Formula (8)

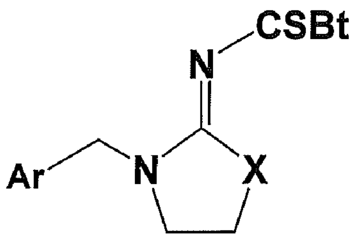

····· Formula (9)

$R_1NH_2$ Formula (10)

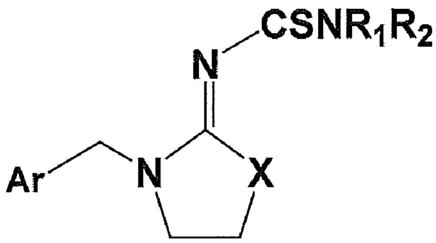

····· Formula (7)

wherein "Ar" represents a 2-chloro-5-pyridyl group or a 2-chloro-5-thiazolyl group;
"X" represents a sulfur atom;
"Bt" represents 1-(1,2,3-benzotriazole);
"$R_1$" represents a C1 to C5 alkyl group, a C1 to C5 halogenated alkyl group, a C6 to C10 aryl group, an aryl(C1 to C3)alkyl group, a (C1 to C4)alkoxy(C1 to C5)alkyl group, a C1 to C3 alkoxycarbonyl group, a (C1 to C3) alkylthio(C1 to C3)alkyl group, a C3 to C7 cycloalkyl group; and, "$R_2$" represents a hydrogen atom.

**15.** A method for producing a compound represented by Formula (14) shown below by reacting a compound represented by Formula (4) shown below with a compound represented by Formula (11) shown below, a compound represented by Formula (12) shown below, or a compound represented by Formula (13) shown below:

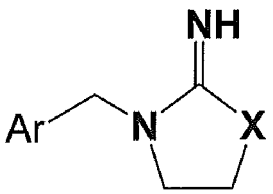

····· Formula (4)

**$(R_3S)2_CS$** Formula (11)

**$ClCS_2R_3$** Formula (12)

Formula (13)

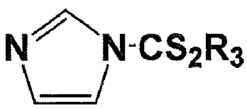

·····式(12)

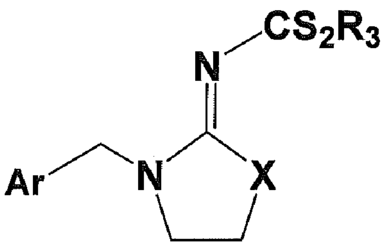

····· Formula (14)

wherein "Ar" represents a 2-chloro-5-pyridyl group or a 2-chloro-5-thiazolyl group;
"X" represents a sulfur atom; and,
"$R_3$" represents a C1 to C5 alkyl group, or a C6 to C10 aryl group, a (C6 to C10)aryl(C1 to C3)alkyl group, a (C1 to C3)alkoxy(C1 to C3)alkyl group, a (C1 to C3)alkoxycarbonyl(C1 to C3)alkyl group.

**16.** A method for producing a compound represented by Formula (16) shown below by reacting a compound represented by Formula (4) shown below with a compound represented by Formula (15) shown below:

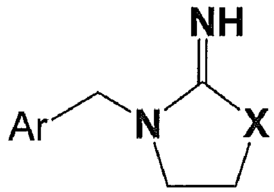

····· Formula (4)

**BC(=P)QR$_4$** Formula (15)

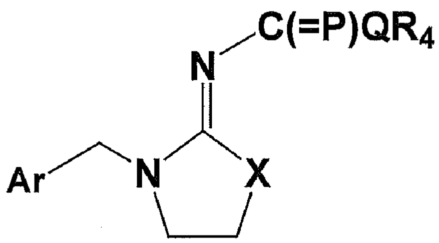

····· Formula (16)

wherein "Ar" represents a 2-chloro-5-pyridyl group or a 2-chloro-5-thiazolyl group;
"X" represents a sulfur atom;
"B" represents a halogen atom such as chlorine, bromine, or iodine;
each of "P", "Q" represents a sulfur atom or an oxygen atom; provided that "P" and "Q" are different from each other; and,
"$R_4$" represents a C1 to C5 alkyl group, or a C6 to C10 aryl group.

**17.** A method for producing a compound represented by Formula (2) shown below by reacting a compound represented by Formula (4) shown below with a compound represented by Formula (17) shown below:

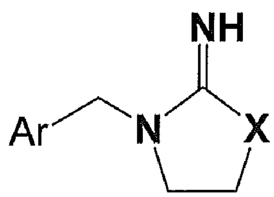

····· Formula (4)

**$Z_\alpha SO_2A$** Formula (17)

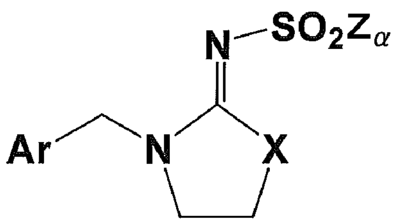

····· Formula (2)

wherein "Ar" represents a 2-chloro-5-pyridyl group or a 2-chloro-5-thiazolyl group;
"X" represents a sulfur atom;
"$Z_\alpha$" represents a C2 to C5 alkyl group or a C1 to C5 halogenated alkyl group, a C2 to C5 alkenyl group, a C6 to C10 aryl group, a (C6 to C10)aryl(C1 to C3)alkyl group, a (C6 to C10)aryl(C2 to C5)alkenyl group, a (C1 to C4)alkoxy(C1 to C3)alkyl group, a C3 to C7 cycloalkyl group, a pyridyl group, a chloropyridyl group, a thienyl group, a chlorothienyl group; or it represents "$NR_5R_6$"; in "$NR_5R_6$", each of "$R_5$" and "$R_6$" represents a hydrogen atom or a C1 to C5 alkyl group, a C1 to C4 alkoxycarbonyl group; and,

"A" represents a halogen atom such as chlorine, bromine, or iodine or **"$OSO_2Z_\alpha$"**.

**18.** A method for producing a compound represented by Formula (19) shown below by reacting a compound represented by Formula (4) shown below with a compound represented by Formula (18) shown below:

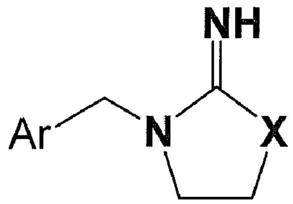

····· Formula (4)

**$NH_2OR_7$** Formula (18)

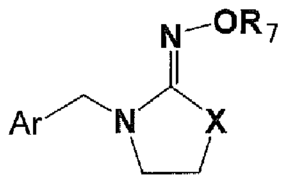

····· Formula (19)

wherein "Ar" represents a 2-chloro-5-pyridyl group;
"X" represents a sulfur atom; and,
"$R_7$" represents a hydrogen atom or a C1 to C5 alkyl group, a C6 to C10 aryl group, a (C6 to C10)aryl(C1 to C3)alkyl group.

**19.** A method for producing a compound represented by Formula (23) shown below by reacting a compound represented by Formula (20) shown below with a compound represented by Formula (21) shown below or a compound represented by Formula (22) shown below:

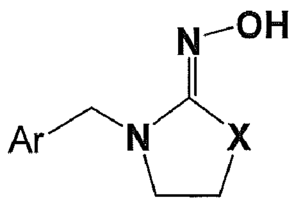

····· Formula (20)

**$R_8COA$** Formula (21)

**$R_8CO_2H$** Formula (22)

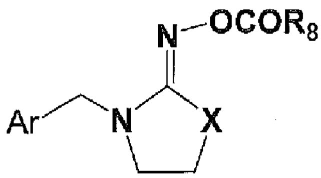

····· Formula (23)

wherein "Ar" represents a 2-chloro-5-pyridyl group;
"X" represents a sulfur atom;
"A" represents a halogen atom such as chlorine, bromine, and iodine; and,
"$R_8$" represents a C1 to C5 alkyl group or a C6 to C10 aryl group, a pyridyl group.

**20.** A method for producing a compound represented by Formula (26) shown below by reacting a compound represented by Formula (20) shown below with a compound represented by Formula (24) shown below or a compound represented by Formula (25):

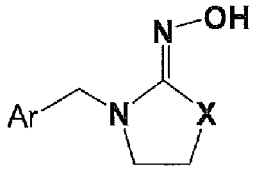 ..... Formula (20)

$R_9NCO$ Formula (24)

$R_9R_{10}NCOB$ Formula (25)

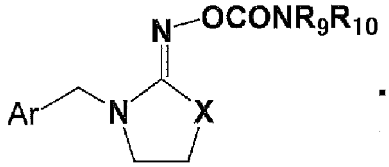 ..... Formula (26)

wherein "Ar" represents a 2-chloro-5-pyridyl group;
"X" represents a sulfur atom;
"B" represents a halogen atom such as chlorine, bromine, and iodine;
each of "$R_9$" and "$R_{10}$" represents a hydrogen atom or a C1 to C5 alkyl group, a C6 to C10 aryl group; and, provided that in Formula (24) "$R_9$" is other than a hydrogen atom.

**21.** A method for producing a compound represented by Formula (28) shown below by reacting a compound represented by Formula (20) shown below with a compound represented by Formula (27) shown below:

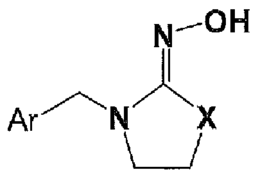 ..... Formula (20)

$R^{11}SO_2D$ Formula (27)

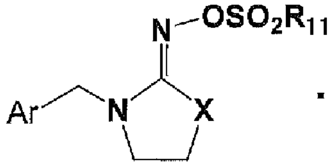 ..... Formula (28)

wherein "Ar" represents a 2-chloro-5-pyridyl group;
"X" represents a sulfur atom;
"D" represents a halogen atom such as chlorine, bromine, and iodine; and,
"$R_{11}$" represents a C1 to C5 alkyl group.

**22.** An insecticide containing as an active ingredient an imino derivative represented by Formula (A) according to Claim 12.

**23.** An imino derivative represented by Formula (A) shown below:

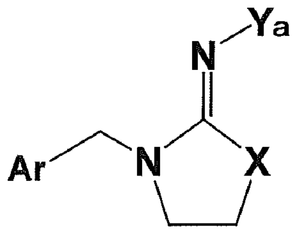

Formula (A)

wherein "Ar" represents a 3-pyridyl group, 5-thiazolyl group, or 3-tetrahydrofuryl group,
"Ya" represents "$C(=S)NR_1R_2$" (wherein each of "$R_1$" and "$R_2$" represents a hydrogen atom or a C1 to C5 alkyl group, a C1 to C5 halogenated alkyl group, a substituted or unsubstituted (C6 to C10)aryl(C1 to C3)alkyl group, a C1 to C3 alkoxycarbonyl group, a C1 to C3 alkylcarbonyl group, a substituted or unsubstituted C6 to C10 arylcarbonyl group, a (C1 to C3)alkylthio(C1 to C3)alkyl group, a C3 to C7 substituted or unsubstituted cycloalkyl group; "$R_1$" and "$R_2$" do not represent hydrogen atoms simultaneously.), or "$C(=S)SR_3$" (wherein "$R_3$" represents a C1 to C5 alkyl group, a substituted or unsubstituted (C6 to C10)aryl(C1 to C3)alkyl group), or "$C(=O)SR_4$", "$C(=S)OR_4$" (wherein "$R_4$" represents a C1 to C5 alkyl group or a substituted or unsubstituted C6 to C10 aryl group, a substituted or unsubstituted pyridyl group), or $SO_2Z_\alpha$ (wherein "$Z_\alpha$" represents a hydrogen atom or a C1 to C5 alkyl group, a C1 to C5 halogenated alkyl group, a C2 to C5 alkenyl group, a substituted or unsubstituted C6 to C10 aryl group, a substituted or unsubstituted (C6 to C10)aryl(C1 to C3)alkyl group, a (C6 to C10)aryl (C2 to C5)alkenyl group, a (C1 to C4)alkoxy(C1 to C3)alkyl group, a C3 to C7 substituted or unsubstituted cycloalkyl group, a substituted or unsubstituted pyridyl group, a substituted or unsubstituted thienyl group , $NR_5R_6$ (wherein each of "$R_5$" and "$R_6$" represents a hydrogen atom or a C1 to C5 alkyl group, a C1 to C4 alkoxycarbonyl group)), or "$OZ_\beta$" (wherein "$Z_\beta$" represents $R_7$ (wherein $R_7$ represents a hydrogen atom or a C1 to C5 alkyl group, a substituted or unsubstituted C6 to C10 aryl group, a substituted or unsubstituted (C6 to C10)aryl(C1 to C3)alkyl group, a (C1 to C4)alkoxy(C1 to C5)alkyl group, a 3-membered ring to 7-membered ring substituted or unsubstituted heterocycloalkyl group, a 3-membered ring to 7-membered ring substituted or unsubstituted heterocycloalkylmethyl group, a substituted or unsubstituted pyridyl group, a substituted or unsubstituted pyridylmethyl group, a substituted or unsubstituted imidazolylmethyl group, or a substituted or unsubstituted heterocyclic ring or aromatic ring, or "$COR_8$" (wherein "$R_8$" represents a C1 to C5 alkyl group, a C1 to C5 halogenated alkyl group, a substituted or unsubstituted C6 to C10 aryl group, a (C1 to C4)alkoxy(C1 to C5)alkyl group, a substituted or unsubstituted pyridyl group, a substituted or unsubstituted heterocyclic ring or aromatic ring), or "$CONR_9R_{10}$" (wherein each of "$R_9$" and "$R_{10}$" represents a hydrogen atom or a C1 to C5 alkyl group, a substituted or unsubstituted C6 to C10 aryl group), or "$SO_2R_{11}$" (wherein "$R_{11}$" represents a hydrogen atom or a C1 to C5 alkyl group, a substituted or unsubstituted C6 to C10 aryl group)).

**24.** An insecticide containing as an active ingredient an imino derivative represented by Formula (A) according to Claim 23.

**INTERNATIONAL SEARCH REPORT**

International application No.
PCT/JP2011/050074

A. CLASSIFICATION OF SUBJECT MATTER
*C07D417/06*(2006.01)i, *A01N43/78*(2006.01)i, *A61P7/02*(2006.01)i, *A61P7/04*(2006.01)i, *C07D417/14*(2006.01)i

According to International Patent Classification (IPC) or to both national classification and IPC

B. FIELDS SEARCHED

Minimum documentation searched (classification system followed by classification symbols)
C07D417/06, A01N43/78, A61P7/02, A61P7/04, C07D417/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | Insecticidally Active New Compounds, Research Disclosure, 1990 p.560-561 (no.31540) | 1-24 |
| X | JP 2002-322175 A (Sinon Corp.), 08 November 2002 (08.11.2002), compounds in examples 1 to 12 (Family: none) | 1-24 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 January, 2011 (24.01.11) | 01 February, 2011 (01.02.11) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2011/050074

C (Continuation). DOCUMENTS CONSIDERED TO BE RELEVANT

| Category* | Citation of document, with indication, where appropriate, of the relevant passages | Relevant to claim No. |
|---|---|---|
| X | JP 11-509530 A (Bayer AG.), 24 August 1999 (24.08.1999), compounds 2, 3 & US 6063798 A & EP 835245 A & WO 1997/001537 A1 & DE 19523658 A & DE 59611287 D & DE 19523658 A1 & AU 6304096 A & BR 9608890 A & HU 9900114 A & TR 9701707 T & ES 2248815 T & CN 1193318 A & MX 9710368 A & AU 705428 B | 1-24 |
| X | JP 4-356481 A (Takeda Chemical Industries, Ltd.), 10 December 1992 (10.12.1992), examples 1 to 3 (Family: none) | 1-24 |
| X | JP 6-316572 A (Nihon Bayer Agrochem Kabushiki Kaisha), 15 November 1994 (15.11.1994), compound of each example (Family: none) | 1-24 |
| A | JP 5-78323 A (Nippon Soda Co., Ltd.), 30 March 1993 (30.03.1993), table 1 & EP 639569 A1 & WO 1992/015564 A1 | 1-24 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5078323 A
- JP 11509530 W
- JP 2002322175 A
- JP 2010001484 A
- JP 2010001485 A
- JP 2010001486 A


### Non-patent literature cited in the description


- *Journal of Medicinal Chemistry,* 1999, vol. 42 (12), 2227-2234